# EUROPÄISCHE PATENTSCHRIFT

(11) **EP 1 105 372 B1**
(45) Veröffentlichungstag und Bekanntmachung des Hinweises auf die Patenterteilung: **11.06.2003**
(21) Anmeldenummer: 99940142.5
(22) Anmeldetag: 04.08.1999
(51) Int. Cl.: C07C 313/28, C07C 275/40, C07C 251/48, C07C 333/04, C07C 261/04, C07F 9/24, A01N 37/28, A01N 37/34, A01N 47/34, A01N 57/30

(54) **METHOXIMINOPHENYLESSIGSÄUREAMIDE**
METHOXIMINOPHENYLACETIC ACID AMIDES
AMIDES D'ACIDE METHOXIMINOPHENYLACETIQUE

(30) Priorität: 17.08.1998 DE 19837065; 05.06.1999 DE 19925780
(43) Veröffentlichungstag der Anmeldung: 13.06.2001
(73) Patentinhaber: Bayer CropScience AG, 40789 Monheim (DE)
(72) Erfinder: MAURER, Fritz, D-40789 Monheim (DE); GAYER, Herbert, D-40789 Monheim (DE); GERDES, Peter, D-52080 Aachen (DE); HEINEMANN, Ulrich, D-42799 Leichlingen (DE); KRÜGER, Bernd-Wieland, D-51467 Bergisch Gladbach (DE); MARKERT, Robert, D-51065 Köln (DE); STENZEL, Klaus, D-40595 Düsseldorf (DE); HÄNSSLER, Gerd, D-51381 Leverkusen (DE); MAULER-MACHNIK, Astrid, D-42799 Leichlingen (DE)
(86) Internationale Anmeldenummer: EP9905643
(87) Internationale Veröffentlichungsnummer: WO00010970

(56) Entgegenhaltungen:
- WO-A-97/00856

## Beschreibung

Die Erfindung betrifft neue Methoximinophenylessigsäureamide, ein Verfahren zu ihrer Herstellung und ihre Verwendung als Fungizide.

Es ist bereits bekannt geworden, daß bestimmte Verbindungen, die den unten beschriebenen konstitutionell ähnlich sind, fungizide Eigenschaften besitzen (vergleiche z.B. EP-A-398 692, EP-A-528 681, WO 97/00 856). Die fungizide Wirkung dieser Verbindungen läßt jedoch, insbesondere bei niedrigen Aufwandmengen, zu wünschen übrig.

Es wurden nun die neuen Methoximinophenylessigsäureamide der allgemeinen Formel (I) gefunden, 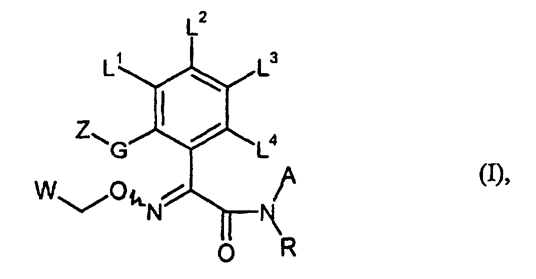 in welcher
- A: für Wasserstoff, Alkoxycarbonyl, Methyl, Ethyl oder Cyclopropyl steht, oder die gleiche Bedeutung wie R hat,
- R: für Alkylthio, Halogenalkylthio, gegebenenfalls substituiertes Arylthio, Alkoxycarbonylthio, -S-Y, oder -S-N(R¹R²) steht, worin
R¹ für Alkyl steht,
R² für Alkyl, Alkoxycarbonyl, oder gemeinsam mit R¹, sowie dem Stickstoffatom, an das sie gebunden, sind für einen gegebenenfalls substituierten heterocyclischen Ring steht,
Y für die gleiche Gruppierung steht, die schon mit dem S-Atom verknüpft ist, oder, wenn A für Methyl steht,
- R: auch für Alkylcarbonyl, Alkoxycarbonyl, Halogenalkoxycarbonyl, Alkoxycarbonylcarbonyl, Alkylaminocarbonyl, Dialkylaminocarbonyl, Alkylsulfonyl oder gegebenenfalls substituiertes Arylsulfonyl steht,
- G: für eine Einfachbindung, für Sauerstoff, Schwefel oder für jeweils gegebenenfalls durch Halogen, Hydroxy, Alkyl, Halogenalkyl oder Cycloalkyl substituiertes Alkandiyl, Alkendiyl, Alkindiyl oder eine der nachstehenden Gruppierungen, in denen jeweils die linke Seite an Z gebunden ist:
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁸)=N-O-, -C(R⁸)=N-O-CH₂-, -N(R⁹)-, -CQ-N(R⁹)-, -N(R⁹)-CQ-, -Q-CQ-N(R⁹)-, -N=C(R⁸)-Q-CH₂-, -CH(R⁸)-O-N=CH-, -C(R⁸)=N-N=CH-, -N(R⁹)-CQ-Q-, -CQ-N(R⁹)-CQ-Q-, -N(R⁹)-CQ-Q-CH₂-, -Q-C(R⁸)=N-O-CH₂-, -N(R⁹)-C(R⁸)=N-O-CH₂-, -O-CH₂-C(R⁸)=N-O-CH₂-, -N=N-C(R⁸)=N-O-CH₂-, -C(=N-O-R¹⁰)-C(R⁸)=N-O-CH₂-, -C(=N-O-R¹⁰)-C(R⁸)-O-N=CH-, -C(=N-O-R¹⁰)-C(R⁸)=N-N=CH-, -C(=N-O-R¹⁰)-C(R⁸)-O-N=C(CH₃)-, -T-Ar¹- oder -T-Ar¹-Q- steht, wobei
Ar¹ für gegebenenfalls substituiertes Arylen, Heteroarylen, Cycloalkylen oder Heterocycloalkylen (d.h. ein zweifach verknüpfter aliphatischer Ring, in dem ein oder mehrere Kohlenstoffatome durch Heteroatome, d.h. von Kohlenstoff verschiedene Atome ersetzt sind) steht,
n für die Zahlen 0, 1 oder 2 steht,
Q für Sauerstoff oder Schwefel steht,
R⁸ für Wasserstoff, Cyano oder jeweils gegebenenfalls substituiertes Alkyl, Alkoxy, Alkylthio, Alkylamino, Dialkylamino oder Cycloalkyl steht,
R⁹ für Wasserstoff, Hydroxy, Cyano oder jeweils gegebenenfalls substituiertes Alkyl, Alkoxy oder Cycloalkyl steht,
R¹⁰ für Wasserstoff, Alkyl oder gegebenenfalls substituiertes Benzyl steht und
T für eine Einfachbindung, für Sauerstoff, Schwefel, -CH₂-O-, -CH₂-S- oder für gegebenenfalls substituiertes Alkandiyl steht,
- L¹, L², L³ und L⁴: gleich oder verschieden sind und unabhängig voneinander jeweils für Wasserstoff, Halogen oder Alkyl stehen,
- W: für Wasserstoff, Fluor, Cyano oder Thiocyanato steht und
- Z: für jeweils gegebenenfalls substituiertes Alkyl, Alkenyl, Alkinyl, Cycloalkyl, Aryl oder Heterocyclyl steht.

In den Definitionen sind die gesättigten oder ungesättigten Kohlenwasserstoffketten, wie Alkyl, auch in Verknüpfung mit Heteroatomen, wie in Alkoxy oder Alkylthio, jeweils geradkettig oder verzweigt.

Halogenalkyl steht für teilweise oder vollständig halogeniertes Alkyl. Bei mehrfach halogeniertem Halogenalkyl können die Halogenatome gleich oder verschieden sein. Bevorzugte Halogenatome sind Fluor und Chlor und insbesondere Fluor. Trägt das Halogenalkyl noch weitere Substituenten, reduziert sich die maximal mögliche Zahl der Halogenatome auf die verbleibenden freien Valenzen.

Halogen steht im allgemeinen für Fluor, Chlor, Brom oder Iod, vorzugsweise für Fluor, Chlor oder Brom, insbesondere für Fluor oder Chlor.

Heterocyclyl steht für gesättigte oder ungesättigte, sowie aromatische, ringförmige Verbindungen, in denen mindestens ein Ringglied ein Heteroatom, d.h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel. Enthält der Ring mehrere Sauerstoffatome, stehen diese nicht benachbart. Gegebenenfalls bilden die ringförmigen Verbindungen mit weiteren carbocyclischen oder heterocyclischen, ankondensierten oder überbrückten Ringen gemeinsam ein polycyclisches Ringsystem. Ein polycyclisches Ringsystem kann über den heterocyclischen Ring oder einen ankondensierten carbocyclischen Ring verknüpft sein. Bevorzugt sind mono- oder bicyclische Ringsysteme, insbesondere mono- oder bicyclische, aromatische Ringsysteme.

Weiterhin wurde gefunden, daß man die neuen Methoximinophenylessigsäureamide der allgemeinen Formel (I) erhält, wenn man Amide der Formel (II) 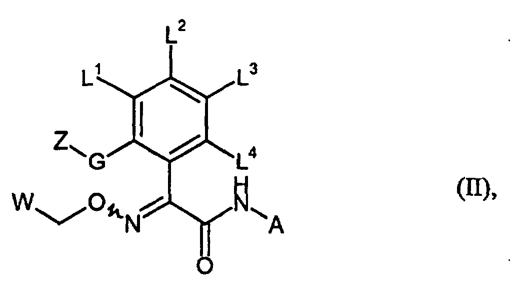 in welcher
- A, G, L¹, L², L³, L⁴, W und Z: die oben angegebenen Bedeutungen haben,
mit einem elektrophilen Reagenz der Formel (III),

R-X (III)

in welcher
- R: die oben angegebene Bedeutung hat, und
- X: für eine Abgangsgruppe steht,
gegebenenfalls in Gegenwart eines Verdünnungsmittels und gegebenenfalls in Gegenwart einer Base umsetzt.

Schließlich wurde gefunden, daß die neuen Methoximinophenylessigsäureamide der allgemeinen Formel (I) eine sehr starke fungizide Wirkung zeigen.

Die erfindungsgemäßen Verbindungen können gegebenenfalls als Mischungen verschiedener möglicher isomerer Formen, insbesondere von Stereoisomeren, wie z.B. E- und Z-, oder optischen Isomeren vorliegen. Es werden sowohl die E- als auch die Z-Isomeren, die einzelnen Enantiomeren, die Racemate, wie auch beliebige Mischungen dieser Isomeren, beansprucht.

Gegenstand der vorliegenden Anmeldung sind vorzugsweise Methoximinophenylessigsäureamide der Formel (I),
in welcher
- A: für Wasserstoff, Methyl, Ethoxycarbonyl, Ethyl oder Cyclopropyl steht, oder die gleiche Bedeutung wie R hat,
- R: für Alkylthio, Halogenalkylthio, Alkoxycarbonylthio, mit jeweils 1 bis 4 Kohlenstoffatomen in den Alkylketten, gegebenenfalls durch Halogen, Cyano, Alkyl, Alkoxy oder Alkoxycarbonyl mit jeweils 1 bis 4 Kohlenstoffatomen, Halogenalkyl oder Halogenalkoxy mit jeweils 1 bis 4 Kohlenstoffatomen und 1 bis 9 gleichen oder verschiedenen Halogenatomen substituiertes Phenylthio, -S-Y, -S-N(R¹R²), worin
R¹ für Alkyl mit 1 bis 4 Kohlenstoffatomen steht,
R² für Alkyl mit 1 bis 4 Kohlenstoffatomen, Alkoxycarbonyl mit 1 bis 4 Kohlenstoffatomen im Alkylteil, oder gemeinsam mit R¹, sowie dem Stickstoffatom, an das sie gebunden, sind für einen gegebenenfalls durch Methyl oder Ethyl substituierten, heterocyclischen Ring mit 5 oder 6 Ringgliedern steht,
Y für die gleiche Gruppierung steht, die schon mit dem Schwefelatom verknüpft ist, oder, wenn A für Methyl steht,
- R: auch für Alkylcarbonyl, Alkoxycarbonyl, Alkoxycarbonylcarbonyl, Halogenalkoxycarbonyl, Alkylaminocarbonyl, Dialkylaminocarbonyl, Alkylsulfonyl mit jeweils 1 bis 4 Kohlenstoffatomen in den Alkylketten oder gegebenenfalls durch Alkyl mit 1 bis 4 Kohlenstoffatomen substituiertes Phenylsulfonyl steht,
- G: für eine Einfachbindung, für Sauerstoff, Schwefel oder für jeweils gegebenenfalls durch Halogen, Hydroxy, C₁-C₄-Alkyl, C₁-C₄-Halogenalkyl oder C₃-C₆-Cycloalkyl substituiertes Alkandiyl, Alkendiyl, Alkindiyl mit jeweils bis zu 4 Kohlenstoffatomen oder eine der nachstehenden Gruppierungen, in denen jeweils die linke Seite an Z gebunden ist:
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁸)=N-O-, -C(R⁸)=N-O-CH₂-, -N(R⁹)-, -CQ-N(R⁹)-, -N(R⁹)-CQ-, -Q-CQ-N(R⁹)-, -N=C(R⁸)-Q-CH₂-, -CH(R⁸)-O-N=CH-, -C(R⁸)=N-N=CH-, -N(R⁹)-CQ-Q-, -CQ-N(R⁹)-CQ-Q-, -N(R⁹)-CQ-Q-CH₂-, -Q-C(R⁸)=N-O-CH₂-, -N(R⁹)-C(R⁸)=N-O-CH₂-, -O-CH₂-C(R⁸)=N-O-CH₂-, -N=N-C(R⁸)=N-O-CH₂-, -C(=N-O-R¹⁰)-C(R⁸)=N-O-CH₂-, -C(=N-O-R¹⁰)-C(R⁸)-O-N=CH-, -C(=N-O-R¹⁰)-C(R⁸)-N-N=CH-, -C(=N-O-R¹⁰)-C(R⁸)-O-N=C(CH₃)-, -T-Ar¹- oder T-Ar¹-Q- steht, wobei
n für die Zahlen 0, 1 oder 2 steht,
Q für Sauerstoff oder Schwefel steht,
R⁸ für Wasserstoff, Cyano, für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₄-Alkyl oder C₁-C₄-Alkoxycarbonyl substituiertes Cycloalkyl mit 3 bis 6 Kohlenstoffatomen steht, und
R⁹ für Wasserstoff, Hydroxy, Cyano oder für gegebenenfalls durch Halogen, Cyano oder C₁-C₄-Alkoxy substituiertes Alkyl mit 1 bis 6 Kohlenstoffatomen oder für gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht,
R¹⁰ für Wasserstoff, C₁-C₄-Alkyl oder gegebenenfalls durch Halogen, Alkyl oder Alkoxy mit jeweils 1 bis 6 Kohlenstoffatomen, Halogenalkyl oder Halogenalkoxy mit jeweils 1 bis 6 Kohlenstoffatomen und 1 bis 13 gleichen oder verschiedenen Halogenatomen substituiertes Benzyl steht,
T für eine Einfachbindung, für Sauerstoff, Schwefel, -CH₂-O-, -CH₂-S- oder für Alkandiyl mit 1 bis 3 Kohlenstoffatomen steht,
Ar¹ für jeweils gegebenenfalls einfach oder mehrfach, gleich oder verschieden substituiertes Phenylen, Naphthylen, Cycloalkylen oder für Heteroarylen oder Heterocycloalkylen mit 3 bis 7 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl; jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, sowie;
Cycloalkyl mit 3 bis 6 Kohlenstoffatomen,
- L¹, L², L³ und L⁴: gleich oder verschieden sind und unabhängig voneinander jeweils für Wasserstoff, Fluor, Chlor, Brom, Methyl, Ethyl oder n- oder i-Propyl, n-, i-, s- oder t-Butyl stehen,
- W: für Wasserstoff, Fluor, Cyano oder Thiocyanato steht und
- Z: für gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 8 Kohlenstoffatomen;
für jeweils gegebenenfalls durch Halogen substituiertes Alkenyl oder Alkinyl mit jeweils bis zu 8 Kohlenstoffatomen;
Tetrahydropyranyl;
für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substituiertes Cycloalkyl mit 3 bis 6 Kohlenstoffatomen;
für jeweils gegebenenfalls einfach oder mehrfach, gleich oder verschieden substituiertes Phenyl, Naphthyl, Tetrahydrobenzofuran oder für Heterocyclyl mit 3 bis 7 Ringgliedem, von denen mindestens eines für Sauerstoff, Schwefel oder Stickstoff und gegebenenfalls ein oder zwei weitere für Stickstoff stehen steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl; Formyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
t-Butyl substituiertes Tetrazol;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen; Phenyl;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl oder Alkylsulfonyloxy, mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel -
oder eine Gruppierung 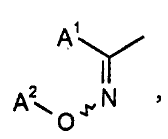 worin
A¹ für Alkyl mit 1 bis 4 Kohlenstoffatomen oder Cycloalkyl mit 1 bis 6 Kohlenstoffatomen steht und
A² für gegebenenfalls durch Cyano, Alkoxy, Alkylthio, Alkylamino, Dialkylamino oder Phenyl substituiertes Alkyl mit 1 bis 4 Kohlenstoffatomen, Alkenyl oder Alkinyl mit jeweils 2 bis 4 Kohlenstoffatomen steht.

Die vorliegende Anmeldung betrifft insbesondere Methoximinophenylessigsäureamide der Formel (I),
in welcher
- A: für Wasserstoff, Methyl, Ethoxycarbonyl, Ethyl oder Cyclopropyl steht, oder die gleiche Bedeutung wie R hat,
- R: für Methylthio, Ethylthio, n- oder i-Propylthio, n-, i-, s- oder t-Butylthio Difluormethylthio, Trifluormethylthio, Difluorchlormethylthio, Dichlorfluormethylthio, Trichlormethylthio, 2,2,2-Trifluorethylthio, 1,1-Difluorethylthio, Methoxycarbonylthio, Ethoxycarbonylthio, gegebenenfalls einfach bis dreifach durch Fluor, Chlor, Brom, Cyano, Methyl, Ethyl, n- oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy, Trifluorethoxy, Methoxycarbonyl oder Ethoxycarbonyl substituiertes Phenylthio, -S-Y, -S-N(R¹R²),
worin
R¹ für Methyl, Ethyl, n- oder i-Propyl, n-, i-, s- oder t-Butyl steht,
R² für Methyl, Ethyl, n- oder i-Propyl, n-, i-, s- oder t-Butyl, Methoxycarbonyl oder Ethoxycarbonyl, oder gemeinsam mit R¹, sowie dem Stickstoffatom, an das sie gebunden, sind für gegebenenfalls durch Methyl oder Ethyl substituiertes Pyrrolidin, Piperidin oder Morpholin steht,
Y für die gleiche Gruppierung steht, die schon mit dem Schwefelatom verknüpft ist, oder, wenn A für Methyl steht,
- R: auch für Acetyl, Propionyl, Methoxycarbonyl, Ethoxycarbonyl, i-Butoxycarbonyl, Methoxycarbonylcarbonyl, 2-Bromethoxycarbonyl, Methylaminocarbonyl, Dimethylaminocarbonyl, Methylsulfonyl, Ethylsulfonyl, Phenylsulfonyl oder Tolylsulfonyl steht,
- G: für Sauerstoff oder für jeweils gegebenenfalls durch Fluor, Chlor oder Brom substituiertes Dimethylen (Ethan-1,2-diyl), Ethen-1,2-diyl oder eine der nachstehenden Gruppierungen, in denen jeweils die linke Seite an Z gebunden ist:
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, - S(O)ₙ-CH₂-, -C(R⁸)=N-O-, -C(R⁸)=N-O-CH₂-, -N(R⁹)-, -CQ-N(R⁹)-, -N(R⁹)-CQ-, -Q-CQ-N(R⁹)-, -N=C(R⁸)-Q-CH₂-, -CH(R⁸)-O-N=CH-, -C(R⁸)=N-N=CH-, -N(R⁹)-CQ-Q-, -CQ-N(R⁹)-CQ-Q-, -N(R⁹)-CQ-Q-CH₂-, -Q-C(R⁸)=N-O-CH₂-, -N(R⁹)-C(R⁸)=N-O-CH₂-, -O-CH₂-C(R⁸)=N-O-CH₂-, -N=N-C(R⁸)=N-O-CH₂-, -C(=N-O-R¹⁰)-C(R⁸)=N-O-CH₂-, -C(=N-O-R¹⁰)-C(R⁸)-O-N=CH-, -C(=N-O-R¹⁰)-C(R⁸)-N-N=CH-, -C(=N-O-R¹⁰)-C(R⁸)-O-N=C(CH₃)-, -T-Ar¹- oder -T-Ar¹-Q- steht, wobei
n für die Zahlen 0, 1 oder 2 steht,
Q für Sauerstoff oder Schwefel steht,
R⁸ für Wasserstoff, Cyano, Methyl, Ethyl oder Cyclopropyl steht und
R⁹ für Wasserstoff, Methyl, Ethyl oder Cyclopropyl steht,
R¹⁰ für Wasserstoff, Methyl, Ethyl, n- oder i-Propyl, n-, i-, s- oder t-Butyl oder für gegebenenfalls durch Fluor, Chlor, Brom, Cyano, Methyl, Ethyl, n- oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy oder Trifluorethoxy substituiertes Benzyl steht,
T für eine Einfachbindung, für Sauerstoff, Schwefel, -CH₂-O-, -CH₂-S- oder für gegebenenfalls substituiertes Alkandiyl steht,
Ar¹ für jeweils gegebenenfalls einfach bis dreifach, gleich oder verschieden substituiertes Phenylen oder Pyridindiyl, für jeweils gegebenenfalls einfach substituiertes Pyrimidindiyl, Pyridazindiyl, Pyrazindiyl, 1,2,3-Triazindiyl, 1,2,4-Triazindiyl oder 1,3,5-Triazindiyl oder für 1,2,4-Thiadiazoldiyl, 1,3,4-Thiadiazoldiyl, 1,2,4-Oxadiazoldiyl, 1,3,4-Oxadiazoldiyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, Cyclopropyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl und
- L¹, L², L³ und L⁴: gleich oder verschieden sind und unabhängig voneinander jeweils für Wasserstoff, Fluor, Chlor, Brom, Methyl oder Ethyl stehen,
- W: für Wasserstoff, Fluor, Cyano oder Thiocyanato steht und
- Z: für Methyl, Ethyl, n- oder i-Propyl, n-, i-, s- oder t-Butyl, Allyl, 2-Buten-1-yl, 2-(1-Methyl)-propyl, 2-Methyl-2-buten-1-yl, Propargyl, Tetrahydropyranyl, t-Butyl substituiertes Tetrazol oder für jeweils gegebenenfalls einfach bis dreifach, gleich oder verschieden substituiertes Phenyl, 1,2,4-Thiadiazolyl, 1,3,4-Thiadiazolyl, 1,2,4-Oxadiazolyl, 1,3,4-Oxadiazolyl, Pyridinyl, Pyrimidyl, Tetrahydrobenzofuranyl, Pyridazinyl, Pyrazinyl, 1,2,3-Triazinyl, 1,2,4-Triazinyl oder 1,3,5-Triazinyl steht, wobei die möglichen Substituenten vorzugsweise aus der nachstehenden Aufzählung ausgewählt sind:
Amino, Fluor, Chlor, Brom, Iod, Cyano, Nitro, Methyl, Ethyl, Formyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl, Phenyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy,
oder eine Gruppierung 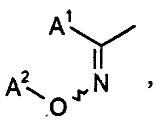 worin
A¹ für Methyl, Ethyl, n- oder i-Propyl, n-, i-, s- oder t-Butyl, Cyclopropyl oder Cyclobutyl steht und
A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht.

Unabhängig von den oben genannten Definitionen steht G auch besonders bevorzugt für
Sauerstoff oder für eine der nachstehenden Gruppierungen, in denen jeweils die linke Seite an Z gebunden ist: -Q-CH₂-, -C(R⁸)=N-O-CH₂-, -N=C(R⁸)-Q-CH₂-, -CH(R⁸)-O-N=CH-, -C(R⁸)=N-N=CH-, -C(=N-O-R¹⁰)-C(R⁸)=N-O-CH₂-, -C(=N-O-R¹⁰)-C(R⁸)-O-N=CH-, -C(=N-O-R¹⁰)-C(R⁸)-N-N=CH- oder -T-Ar¹-Q- steht, wobei R⁸, R⁹, R¹⁰, T, Ar¹ und Q die oben angegebenen bevorzugten und insbesondere bevorzugten Bedeutungen haben.
- L¹, L², L³ und L⁴: stehen unabhängig voneinander besonders bevorzugt für Wasserstoff.
- Q: steht besonders bevorzugt für Sauerstoff.
- W: steht besonders bevorzugt für Wasserstoff.

Eine besonders bevorzugte Gruppe erfindungsgemäßer Verbindungen sind diejenigen Verbindungen der Formel (I),
in welcher
- A: für Wasserstoff, Ethoxycarbonyl, Methyl oder Ethyl, Cyclopropyl steht, oder die gleiche Bedeutung wie R hat,
- R: für Methylthio, Ethylthio, n- oder i-Propylthio, n-, i-, s- oder t-Butylthio Difluormethylthio, Trifluormethylthio, Difluorchlormethylthio, Dichlorfluormethylthio, Trichlormethylthio, 2,2,2-Trifluorethylthio, 1,1-Difluorethylthio, Methoxycarbonylthio, Ethoxycarbonylthio, gegebenenfalls einfach bis dreifach durch Fluor, Chlor, Brom, Cyano, Methyl, Ethyl, n- oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy, Trifluorethoxy, Methoxycarbonyl oder Ethoxycarbonyl substituiertes Phenylthio, -S-Y, -S-N(R¹R²), worin
R¹ für Methyl, Ethyl, n- oder i-Propyl, n-, i-, s- oder t-Butyl steht,
R² für Methyl, Ethyl, n- oder i-Propyl, n-, i-, s- oder t-Butyl, Methoxycarbonyl oder Ethoxycarbonyl, oder gemeinsam mit R¹, sowie dem Stickstoffatom, an das sie gebunden, sind für gegebenenfalls durch Methyl oder Ethyl substituiertes Pyrrolidin, Piperidin oder Morpholin steht,
Y für die gleiche Gruppierung steht, die schon mit dem Schwefelatom verknüpft ist, oder, wenn A für Methyl steht,
- R: auch für Acetyl, Propionyl, Methoxycarbonyl, Ethoxycarbonyl, i-Butoxycarbonyl, Methoxycarbonylcarbonyl, 2-Bromethoxycarbonyl, Methylaminocarbonyl, Dimethylaminocarbonyl, Methylsulfonyl, Ethylsulfonyl, Phenylsulfonyl oder Tolylsulfonyl steht,
- G: für Sauerstoff oder insbesondere -O-CH₂- steht,
- L¹, L², L³ und L⁴: für Wasserstoff stehen,
- W: für Wasserstoff, Fluor, Cyano oder Thiocyanato steht und
- Z: für Methyl, Ethyl, n- oder i-Propyl, n-, i-, s- oder t-Butyl, Allyl, 2-Buten-1-yl, 2-Methyl-2-buten-1-yl, Propargyl, Tetrahydropyranyl oder für jeweils gegebenenfalls einfach bis dreifach, gleich oder verschieden substituiertes Phenyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl, Phenyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy
oder eine Gruppierung 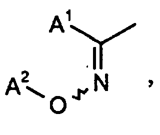 worin
A¹ für Methyl, Ethyl, n- oder i-Propyl, n-, i-, s- oder t-Butyl, Cyclopropyl oder Cyclobutyl steht und
A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht.

Eine ebenfalls besonders bevorzugte Gruppe erfindungsgemäßer Verbindungen sind diejenigen Verbindungen der Formel (I),
in welcher
- A: für Wasserstoff, Ethyl oder Methyl steht, oder die gleiche Bedeutung wie R hat,
- R: für Methylthio, Ethylthio, n- oder i-Propylthio, n-, i-, s- oder t-Butylthio Difluormethylthio, Trifluormethylthio, Difluorchlormethylthio, Dichlorfluormethylthio, Trichlormethylthio, 2,2,2-Trifluorethylthio, Methoxycarbonylthio, Ethoxycarbonylthio, gegebenenfalls einfach bis dreifach durch Fluor, Chlor, Brom, Cyano, Methyl, Ethyl, n- oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy, Trifluorethoxy, Methoxycarbonyl oder Ethoxycarbonyl substituiertes Phenylthio, -S-Y, -S- N(R¹R²), worin
R¹ für Methyl, Ethyl, n- oder i-Propyl, n-, i-, s- oder t-Butyl steht,
R² für Methyl, Ethyl, n- oder i-Propyl, n-, i-, s- oder t-Butyl, Methoxycarbonyl oder Ethoxycarbonyl, oder gemeinsam mit R¹, sowie dem Stickstoffatom, an das sie gebunden, sind für gegebenenfalls durch Methyl oder Ethyl substituiertes Pyrrolidin, Piperidin oder Morpholin steht,
Y für die gleiche Gruppierung steht, die schon mit dem Schwefelatom verknüpft ist, oder, wenn A für Methyl steht,
- R: auch für Acetyl, Propionyl, Methoxycarbonyl, Ethoxycarbonyl, Methoxycarbonylcarbonyl, 2-Bromethoxycarbonyl, Methylaminocarbonyl, Dimethylaminocarbonyl, Methylsulfonyl, Ethylsulfonyl, Phenylsulfonyl oder Tolylsulfonyl steht,
- G: für -C(R⁸)=N-O-CH₂- steht,
- R⁸: für Cyclopropyl oder insbesondere Methyl steht,
- L¹, L², L³ und L⁴: für Wasserstoff stehen,
- W: für Wasserstoff, Fluor, Cyano oder Thiocyanato steht und
- Z: für Methyl, Ethyl, n- oder i-Propyl, n-, i-, s- oder t-Butyl, Allyl, 2-Buten-1-yl, 2-Methyl-2-buten-1-yl, Propargyl oder für jeweils gegebenenfalls einfach bis dreifach, gleich oder verschieden substituiertes Pyridyl, Pyrimidyl oder insbesondere Phenyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy.

Eine weiterhin besonders bevorzugte Gruppe erfindungsgemäßer Verbindungen sind diejenigen Verbindungen der Formel (I),
in welcher
- A: für Wasserstoff, Ethyl oder Methyl steht, oder die gleiche Bedeutung wie R hat,
- R: für Methylthio, Ethylthio, n- oder i-Propylthio, n-, i-, s- oder t-Butylthio Difluormethylthio, Trifluormethylthio, Difluorchlormethylthio, Dichlorfluormethylthio, Trichlormethylthio, 2,2,2-Trifluorethylthio, Methoxycarbonylthio, Ethoxycarbonylthio, gegebenenfalls einfach bis dreifach durch Fluor, Chlor, Brom, Cyano, Methyl, Ethyl, n- oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy, Trifluorethoxy, Methoxycarbonyl oder Ethoxycarbonyl substituiertes Phenylthio, -S-Y, -S- N(R¹R²), worin
R¹ für Methyl, Ethyl, n- oder i-Propyl, n-, i-, s- oder t-Butyl steht,
R² für Methyl, Ethyl, n- oder i-Propyl, n-, i-, s- oder t-Butyl, Methoxycarbonyl oder Ethoxycarbonyl, oder gemeinsam mit R¹, sowie dem Stickstoffatom, an das sie gebunden, sind für gegebenenfalls durch Methyl oder Ethyl substituiertes Pyrrolidin, Piperidin oder Morpholin steht,
Y für die gleiche Gruppierung steht, die schon mit dem Schwefelatom verknüpft ist, oder, wenn A für Methyl steht,
- R: auch für Acetyl, Propionyl, Methoxycarbonyl, Ethoxycarbonyl, Methoxycarbonylcarbonyl, 2-Bromethoxycarbonyl, Methylaminocarbonyl, Dimethylaminocarbonyl, Methylsulfonyl, Ethylsulfonyl, Phenylsulfonyl oder Tolylsulfonyl steht,
- G: für -T-Ar¹-O- steht,
- Ar¹: für 1,2,4-Thiadiazoldiyl, 1,3,4-Thiadiazoldiyl, 1,2,4-Oxadiazoldiyl, 1,3,4-Oxadiazoldiyl oder für jeweils gegebenenfalls einfach oder zweifach, gleich oder verschieden durch Fluor, Chlor, Cyano, Methyl, Cyclopropyl, Methoxy, Methylthio, Trifluormethyl, Difluormethoxy, Trifluormethoxy, Difluorchlormethoxy substituiertes, Pyridindiyl, Pyrimidindiyl oder 1,3,5-Triazindiyl steht,
- T: für eine Einfachbindung, für Sauerstoff, Schwefel, -CH₂-O-, CH₂-S-, Methylen, Ethylen oder Propylen steht und
- L¹, L², L³ und L⁴: für Wasserstoff stehen,
- W: für Wasserstoff, Fluor, Cyano oder Thiocyanato steht und
- Z: für Methyl, Ethyl, n- oder i-Propyl, n-, i-, s- oder t-Butyl, Allyl, 2-Buten-1-yl, 2-(1-Methyl)-propyl, 2-Methyl-2-buten-1-yl, Propargyl oder für jeweils gegebenenfalls einfach bis dreifach, gleich oder verschieden durch Amino, Fluor, Chlor, Brom, Iod, Cyano, Nitro, Methyl, Formyl, Ethyl, n- oder i-Propyl, n-, i-, s- oder t-Butyl, Trifluormethyl, t-Butyl substituiertes Tetrazol, Methoxy, Ethoxy, n- oder i-Propoxy, Difluormethoxy, Difluorchlormethoxy, Trifluorethoxy, Trifluormethoxy, Methoximinomethyl, Ethoximinomethyl, Methoximinoethyl, Ethoximinoethyl, oder jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy, substituiertes Pyridyl, Pyrimidyl, Thienyl, Tetrahydrobenzofuranyl oder insbesondere Phenyl steht.

Noch eine besonders bevorzugte Gruppe erfindungsgemäßer Verbindungen sind diejenigen Verbindungen der Formel (I),
in welcher
- A: für Wasserstoff oder Methyl steht, oder die gleiche Bedeutung wie R hat,
- R: für Methylthio, Ethylthio, n- oder i-Propylthio, n-, i-, s- oder t-Butylthio Difluormethylthio, Trifluormethylthio, Difluorchlormethylthio, Dichlorfluormethylthio, Trichlormethylthio, 2,2,2-Trifluorethylthio, Methoxycarbonylthio, Ethoxycarbonylthio, gegebenenfalls einfach bis dreifach durch Fluor, Chlor, Brom, Cyano, Methyl, Ethyl, n- oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy, Trifluorethoxy, Methoxycarbonyl oder Ethoxycarbonyl substituiertes Phenylthio, -S-Y, -S-N(R¹R²), worin
R¹ für Methyl, Ethyl, n- oder i-Propyl, n-, i-, s- oder t-Butyl steht,
R² für Methyl, Ethyl, n- oder i-Propyl, n-, i-, s- oder t-Butyl, Methoxycarbonyl oder Ethoxycarbonyl, oder gemeinsam mit R¹, sowie dem Stickstoffatom, an das sie gebunden, sind für gegebenenfalls durch Methyl oder Ethyl substituiertes Pyrrolidin, Piperidin oder Morpholin steht,
Y für die gleiche Gruppierung steht, die schon mit dem Schwefelatom verknüpft ist, oder, wenn A für Methyl steht,
- R: auch für Acetyl, Propionyl, Methoxycarbonyl, Ethoxycarbonyl, Methoxycarbonylcarbonyl, 2-Bromethoxycarbonyl, Methylaminocarbonyl, Dimethylaminocarbonyl, Methylsulfonyl, Ethylsulfonyl, Phenylsulfonyl oder Tolylsulfonyl steht,
- G: für -C(=N-O-R¹⁰)-C(R⁸)=N-O-CH₂- steht, worin
R⁸ für Methyl oder Cyclopropyl steht und
R¹⁰ für Wasserstoff, Methyl, Ethyl, n- oder i-Propyl, n-, i-, s- oder t-Butyl oder für gegebenenfalls durch Fluor, Chlor, Brom, Cyano, Methyl, Ethyl, n- oder i-Propyl, n-, i-, s- oder t-Butyl, Methoxy, Ethoxy, n-oder i-Propoxy, Trifluormethyl, Trifluorethyl, Difluormethoxy, Trifluormethoxy, Difluorchlormethoxy oder Trifluorethoxy substituiertes Benzyl steht,
- L¹, L², L³ und L⁴: für Wasserstoff stehen,
- W: für Wasserstoff, Fluor, Cyano oder Thiocyanato steht und
- Z: für Methyl, Ethyl, n- oder i-Propyl, n-, i-, s- oder t-Butyl, Allyl, 2-Buten-1-yl, 2-Methyl-2-buten-1-yl oder für jeweils gegebenenfalls einfach bis dreifach, gleich oder verschieden substituiertes Pyridyl, Pyrimidyl oder insbesondere Phenyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy oder durch t-Butyl substituiertes Tetrazol.

Insbesondere bevorzugt sind Verbindungen der Formel (I),
in denen A für Methyl steht.

Insbesondere bevorzugt sind Verbindungen der Formel (I),
in denen
- G: für Sauerstoff, -O-CH₂, -C(CH₃)=N-O-CH₂- oder gegebenenfalls durch Fluor substituiertes Pyrimidinyloxy steht.

Insbesondere bevorzugt sind Verbindungen der Formel (I),
in denen
- Z: für unsubstituiertes oder einfach oder zweifach substituiertes Phenyl steht.

Insbesondere bevorzugt sind Verbindungen der Formel (I),
in denen
- R: für die oben genannten, über Schwefel gebundenen Substituenten, wie insbesondere Alkyl-, Halogenalkyl-, gegebenenfalls substituiertes Phenyl-, Alkoxycarbonyl- oder Dialkylamino-thio steht.

Insbesondere bevorzugt sind Verbindungen der Formel (I),
in denen
- W: für Wasserstoff steht.

Insbesondere bevorzugt sind Verbindungen der Formel (I),
in denen
- L¹, L², L³ und L⁴: für Wasserstoff stehen.

Die oben aufgeführten allgemeinen oder in den Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Die zur Durchführung des erfindungsgemäßen Verfahrens zur Herstellung der Methoximinophenylessigsäureamide der Formel (I) als Ausgangsstoffe benötigten Amide sind durch die Formel (II) allgemein definiert. In dieser Formel (II) haben A, G, L¹, L², L³, L⁴, W und Z vorzugsweise bzw. insbesondere diejenigen Bedeutungen die bereits weiter oben im Zusammenhang mit der Beschreibung der erfindungsgemäßen Verbindungen der Formel (I) als bevorzugt, bzw. als insbesondere bevorzugt für A, G, L¹, L², L³, L⁴, W und Z genannt wurden.

Die Amide der Formel (I) sind bekannt und können nach bekannten Methoden hergestellt werden (vergleiche z.B. EP-A-398 692, EP-A-528 681, WO 98/21 189).

Die zur Durchführung des erfindungsgemäßen Verfahrens zur Herstellung der Methoximinophenylessigsäureamide der Formel (I) als Ausgangsstoffe weiterhin benötigten elektrophilen Reagenzien sind durch die Formel (III) definiert. X steht für eine Abgangsgruppe, wie beispielsweise Halogen, vorzugsweise Chlor. Steht R für Alkylcarbonyl, kommen als Verbindungen der Formel (III) auch Carbonsäureanhydride in Frage, d.h. R steht für -CO-R', wobei R' für Alkyl, vorzugsweise Methyl oder Ethyl steht. Steht R für Alkylaminocarbonyl, kommen als Reagenzien der Formel (III) auch die entsprechenden Isocyanate in Frage.

Alle Reagenzien der Formel (III) sind bekannte Synthesechemikalien und es ist dem Fachmann durchaus bekannt, welches Reagenz im Einzelfall in Frage kommt.

Als Verdünnungsmittel zur Durchführung des erfindungsgemäßen Verfahrens kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; oder Alkohole, wie tert-Butanol.

Das erfindungsgemäße Verfahren wird gegebenenfalls in Gegenwart eines geeigneten Säureakzeptors durchgeführt. Als solche kommen alle üblichen anorganischen oder organischen Basen in Frage. Hierzu gehören vorzugsweise Erdalkalimetall- oder Alkalimetallhydride, -hydroxide, -amide, -alkoholate -carbonate oder -hydrogencarbonate, wie beispielsweise Natriumhydrid, Kaliumhydrid, Natriumamid, Kaliumtert.-butylat, Natriumhydroxid, Kaliumhydroxid, Natriumcarbonat, Kaliumcarbonat, Kaliumhydrogencarbonat oder Natriumhydrogencarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen von -100°C bis 80°C, vorzugsweise bei Temperaturen von -80°C bis 50°C.

Zur Durchführung des erfindungsgemäßen Verfahrens zur Herstellung der Verbindungen der Formel (I) setzt man pro Mol des Amids der Formel (II) im allgemeinen 1 bis 3 Mol, vorzugsweise 1 bis 2 Mol elektrophiles Reagenz der Formel (III) ein.

Die Reaktionsdurchführung, Aufarbeitung und Isolierung der Reaktionsprodukte erfolgt nach allgemein üblichen Verfahren (vergleiche auch die Herstellungsbeispiele).

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen Erysiphe-Arten, Puccinia- oder Fusarium-Arten, von Krankheiten im Wein-, Obst- und Gemüseanbau, wie beispielsweise gegen Venturia-, Sphaerotheca-, Podosphaera- und Plasmopara-Arten, oder von Reiskrankheiten, wie beispielsweise gegen Pyricularia-Arten, einsetzen.

Die erfindungsgemäßen Wirkstoffe eignen sich auch zur Steigerung des Emteertrages. Sie sind außerdem mindertoxisch und weisen eine gute Pflanzenverträglichkeit auf.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitem oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfemaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
   Dagger G,
   OK-8705,
   OK-8801,
   α-(1,1-Dimethylethyl)-β-(2-phenoxyethyl)-1H-1,2,4-triazol-1-ethanol,
   α-(2,4-Dichlorphenyl)-β-fluor-b-propyl-1H-1,2,4-triazol-1-ethanol,
   α-(2,4-Dichlorphenyl)-β-methoxy-a-methyl-1H-1,2,4-triazol-1-ethanol,
   α-(5-Methyl-1,3-dioxan-5-yl)-β-[[4-(trifluormethyl)-phenyl]-methylen]-1H-1,2,4-triazol-1-ethanol,
   (5RS,6RS)-6-Hydroxy-2,2,7,7-tetramethyl-5-(1H-1,2,4-triazol-1-yl)-3-octanon,
   (E)-a-(Methoxyimino)-N-methyl-2-phenoxy-phenylacetamid,
   {2-Methyl-1-[[[1-(4-methylphenyl)-ethyl]-amino]-carbonyl]-propyl}-carbaminsäure-1-isopropylester,
   1-(2,4-Dichlorphenyl)-2-(1H-1,2,4-triazol-1-yl)-ethanon-O-(phenylmethyl)-oxim,
   1-(2-Methyl-1-naphthalenyl)-1H-pyrrol-2,5-dion,
   1-(3,5-Dichlorphenyl)-3-(2-propenyl)-2,5-pyrrolidindion,
   1-[(Diiodmethyl)-sulfonyl]-4-methyl-benzol,
   1-[[2-(2,4-Dichlorphenyl)-1,3-dioxolan-2-yl]-methyl]-1H-imidazol,
   1-[[2-(4-Chlorphenyl)-3-phenyloxiranyl]-methyl]-1H-1,2,4-triazol,
   1-[1-[2-[(2,4-Dichlorphenyl)-methoxy]-phenyl]-ethenyl]-1H-imidazol,
   1-Methyl-5-nonyl-2-(phenylmethyl)-3-pyrrolidinol,
   2',6'-Dibrom-2-methyl-4'-trifluormethoxy-4'-trifluor-methyl-1,3-thiazol-5-carboxanilid,
   2,2-Dichlor-N-[1-(4-chlorphenyl)-ethyl]-1-ethyl-3-methyl-cyclopropancarboxamid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)-phenyl]-methyl]-benzamid,
   2-(2,3,3-Triiod-2-propenyl)-2H-tetrazol,
   2-[(1-Methylethyl)-sulfonyl]-5-(trichlormethyl)-1,3,4-thiadiazol,
   2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
   2-Aminobutan,
   2-Brom-2-(brommethyl)-pentandinitril,
   2-Chlor-N-(2,3-dihydro-1,1,3-trimethyl-1H-inden-4-yl)-3-pyridincarboxamid,
   2-Chlor-N-(2,6-dimethylphenyl)-N-(isothiocyanatomethyl)-acetamid,
   2-Phenylphenol(OPP),
   3,4-Dichlor-1-[4-(difluormethoxy)-phenyl]-1H-pyrrol-2,5-dion,
   3,5-Dichlor-N-[cyan[(1-methyl-2-propynyl)-oxy]-methyl]-benzamid,
   3-(1,1-Dimethylpropyl-1-oxo-1H-inden-2-carbonitril,
   3-[2-(4-Chlorphenyl)-5-ethoxy-3-isoxazolidinyl]-pyridin,
   4-Chlor-2-cyan-N,N-dimethyl-5-(4-methylphenyl)-1H-imidazol-1-sulfonamid,
   4-Methyl-tetrazolo[1,5-a]quinazolin-5(4H)-on,
   8-(1,1-Dimethylethyl)-N-ethyl-N-propyl-1,4-dioxaspiro[4.5]decan-2-methanamin,
   8-Hydroxychinolinsulfat,
   9H-Xanthen-9-carbonsäure-2-[(phenylamino)-carbonyl]-hydrazid,
   bis-(1-Methylethyl)-3-methyl-4-[(3-methylbenzoyl)-oxy]-2,5-thiophendicarboxylat,
   cis-1-(4-Chlorphenyl)-2-(1H-1,2,4-triazol-1-yl)-cycloheptanol,
   cis-4-[3-[4-(1,1-Dimethylpropyl)-phenyl-2-methylpropyl]-2,6-dimethyl-morpholinhydrochlorid,
   Ethyl-[(4-chlorphenyl)-azo]-cyanoacetat,
   Kaliumhydrogencarbonat,
   Methantetrathiol-Natriumsalz,
   Methyl-1-(2,3-dihydro-2,2-dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,3-Dichlor-4-hydroxyphenyl)-1-methyl-cyclohexancarboxamid.
   N-(2,6-Dimethylphenyl)-2-methoxy-N-(tetrahydro-2-oxo-3-furanyl)-acetamid,
   N-(2,6-Dimethylphenyl)-2-methoxy-N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy-N-(2-oxo-3-oxazolidinyl)-acetamid,
   N-(6-Methoxy)-3-pyridinyl)-cyclopropancarboxamid,
   N-[2,2,2-Trichlor-1-[(chloracetyl)-amino]-ethyl]-benzamid,
   N-[3-Chlor-4,5-bis-(2-propinyloxy)-phenyl]-N'-methoxy-methanimidamid,
   N-Formyl-N-hydroxy-DL-alanin -Natriumsalz,
   O,O-Diethyl-[2-(dipropylamino)-2-oxoethyl]-ethylphosphoramidothioat,
   O-Methyl-S-phenyl-phenylpropylphosphoramidothioat,
   S-Methyl-1,2,3-benzothiadiazol-7-carbothioat,
   spiro[2H]-1-Benzopyran-2,1'(3'H)-isobenzofuran]-3'-on.

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
Granuloseviren,
Halofenozide, HCH, Heptenophos, Hexaflumuron, Hexythiazox, Hydroprene,
Imidacloprid, Isazofos, Isofenphos, Isoxathion, Ivermectin,
Kernpolyederviren,
Lambda-cyhalothrin, Lufenuron,
Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
Naled, Nitenpyram, Nithiazine, Novaluron,
Omethoat, Oxamyl, Oxydemethon M,
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin,
Salithion, Sebufos, Silafluofen, Spinosad, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Thetacypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii,
YI 5302,
Zeta-cypermethrin, Zolaprofos,
(1R-cis)-[5-(Phenylmethyl)-3-furanyl]-methyl-3-[(dihydro-2-oxo-3(2H)-furanyliden)-methyl]-2,2-dimethylcyclopropancarboxylat,
   (3-Phenoxyphenyl)-methyl-2,2,3,3-tetramethylcyclopropanecarboxylat,
   1-[(2-Chlor-5-thiazolyl)methyl]tetrahydro-3,5-dimethyl-N-nitro-1,3,5-triazin-2(1H)-imin,
   2-(2-Chlor-6-fluorphenyl)-4-[4-(1,1-dimethylethyl)phenyl]-4,5-dihydro-oxazol,
   2-(Acetlyoxy)-3-dodecyl-1,4-naphthalindion,
   2-Chlor-N-[[[4-(1-phenylethoxy)-phenyl]-amino]-carbonyl]-benzamid,
   2-Chlor-N-[[[4-(2,2-dichlor-1,1-difluorethoxy)-phenyl]-amino]-carbonyl]-benzamid, 3-Methylphenyl-propylcarbamat,
   4-[4-(4-Ethoxyphenyl)-4-methylpentyl]-1-fluor-2-phenoxy-benzol,
   4-Chlor-2-(1,1-dimethylethyl)-5-[[2-(2,6-dimethyl-4-phenoxyphenoxy)ethyl]thio]-3 (2H)-pyridazinon,
   4-Chlor-2-(2-chlor-2-methylpropyl)-5-[(6-iod-3-pyridinyl)methoxy]-3(2H)-pyridazinon,
   4-Chlor-5-[(6-chlor-3-pyridinyl)methoxy]-2-(3,4-dichlorphenyl)-3(2H)-pyridazinon,
   Bacillus thuringiensis strain EG-2348,
   Benzoesäure [2-benzoyl-1-(1,1-dimethylethyl)-hydrazid,
   Butansäure 2,2-dimethyl-3-(2,4-dichlorphenyl)-2-oxo-1-oxaspiro[4.5]dec-3-en-4-ylester,
   [3-[(6-Chlor-3-pyridinyl)methyl]-2-thiazolidinyliden]-cyanamid,
   Dihydro-2-(nitromethylen)-2H-1,3-thiazine-3(4H)-carboxaldehyd,
   Ethyl-[2-[[1,6-dihydro-6-oxo-1-(phenylmethyl)-4-pyridazinyl]oxy]ethyl]-carbamat,
   N-(3,4,4-Trifluor-1-oxo-3-butenyl)-glycin,
   N-(4-Chlorphenyl)-3-[4-(difluormethoxy)phenyl]-4,5-dihydro-4-phenyl-1H-pyrazol-1-carboxamid,
   N-[(2-Chlor-5-thiazolyl)methyl]-N'-methyl-N"-nitro-guanidin,
   N-Methyl-N'-(1-methyl-2-propenyl)-1,2-hydrazindicarbothioamid,
   N-Methyl-N'-2-propenyl-1,2-hydrazindicarbothioamid,
   O,O-Diethyl-[2-(dipropylamino)-2-oxoethyl]-ethylphosphoramidothioat.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

### Herstellungsbeispiele

### Beispiel 1

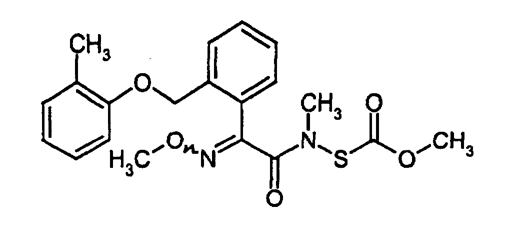

Zu einer Suspension von 0,17 g (4,2 mMol) Natriumhydrid (60 % in Mineralöl) in 15 ml Tetrahydrofuran gibt man bei 0 bis 5°C 1,1 g (3,5 mMol) 2-Methoximino-2[2-(methylphenoxy)-methylphenyl]-N-methyl-acetamid und rührt die Mischung 30 Minuten nach. Unter weiterer Kühlung werden dann bei -70°C 0,493 g (3,9 mMol) Methoxycarbonylsulfensäurechlorid zugetropft. Anschließend rührt man das Reaktionsgemisch über Nacht bei Raumtemperatur nach, filtriert vom anorganischen Material ab und destilliert das Lösungsmittel im Vakuum ab. Der Rückstand wird mit Cyclohexan/Essigester (3:1) an Kieselgel chromatografiert. Man erhält 1,24 g (88 % der Theorie) 2-Methoximino-2[2-(methylphenoxy)-methylphenyl]-N-methyl-N-methoxycarbonylthio-acetamid als farbloses Öl.
HPLC: logP = 4,25

### Beispiel 2

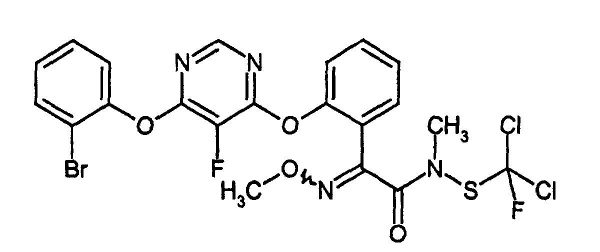

3,15 g (0,00663 Mol) 2-{2-[6-(2-Bromphenoxy)-5-fluorpyrimidin-4-yloxy]-phenyl}-2-methoxyimino-N-methyl-acetamid werden in 25 ml Pyridin gelöst und auf 0°C gekühlt. Hierzu tropft man 1,12 g (0,0066 Mol) Dichlorfluormethansulfensäurechlorid und rührt anschließend 8 Stunden bei 5°C. Das Reaktionsgemisch wird auf 200 ml eisgekühlte 2N Salzsäure gegossen und mit Diethylether extrahiert. Die organische Phase wird über Natriumsulfat getrocknet und das Lösungsmittel im Vakuum abdestilliert. Der Rückstand wird mit n-Hexan:Aceton = 4:1 an Kieselgel chromatographiert. Man erhält 0,3 g (6,07 % der Theorie; Gehalt nach HPLC: 81,6 %;) 2-{2-[6-(2-Brom-phenoxy)-5-fluor-pyrimidin-4-yloxy]-phenyl}-N-(dichlorfluor-methylsulfanyl)-2-methoxyimino-N-methylacetamid.
logP = 5,10
LC/MS: M = 614, 612, 611, 609, 607, 589, 577, 520, 489, 478, 477, 475, 459, 457, 388, 366.
¹H-NMR-Spektrum (DMSOd₆/TMS): δ = 3,35 (3H); 3,74 (3H); 7,29-7,34 (1H); 7,40-7,62 (6H); 7,78-7,81 (1H); 8,17 (1H) ppm.

Analog Beispiel 1 und 2, sowie entsprechend der allgemeinen Beschreibung des erfindungsgemäßen Herstellungsverfahrens zur Herstellung der Verbindungen der Formel (I) wurden auch die folgenden in der Tabelle 1 genannten, erfindungsgemäßen Verbindungen der Formel (Ia) erhalten:

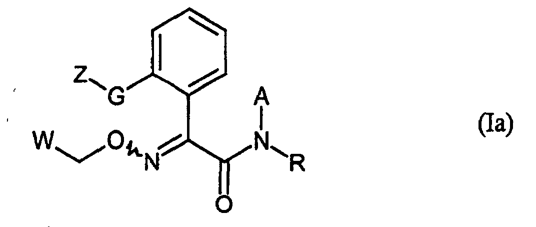

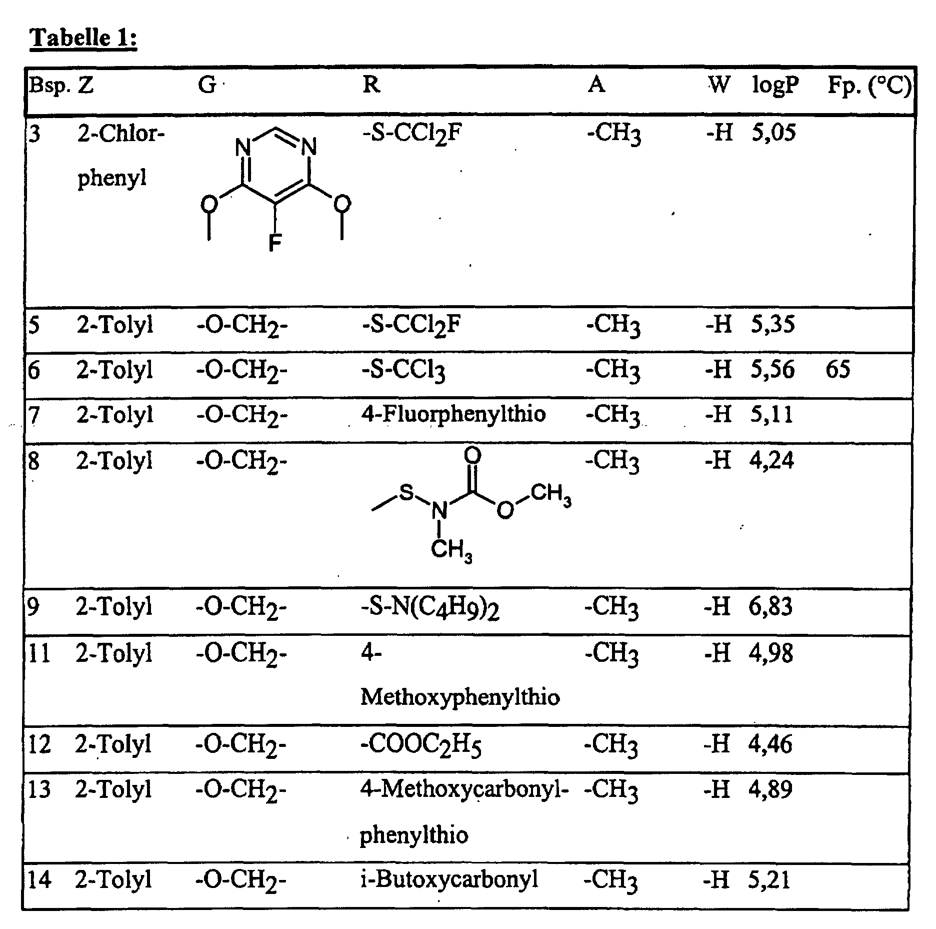

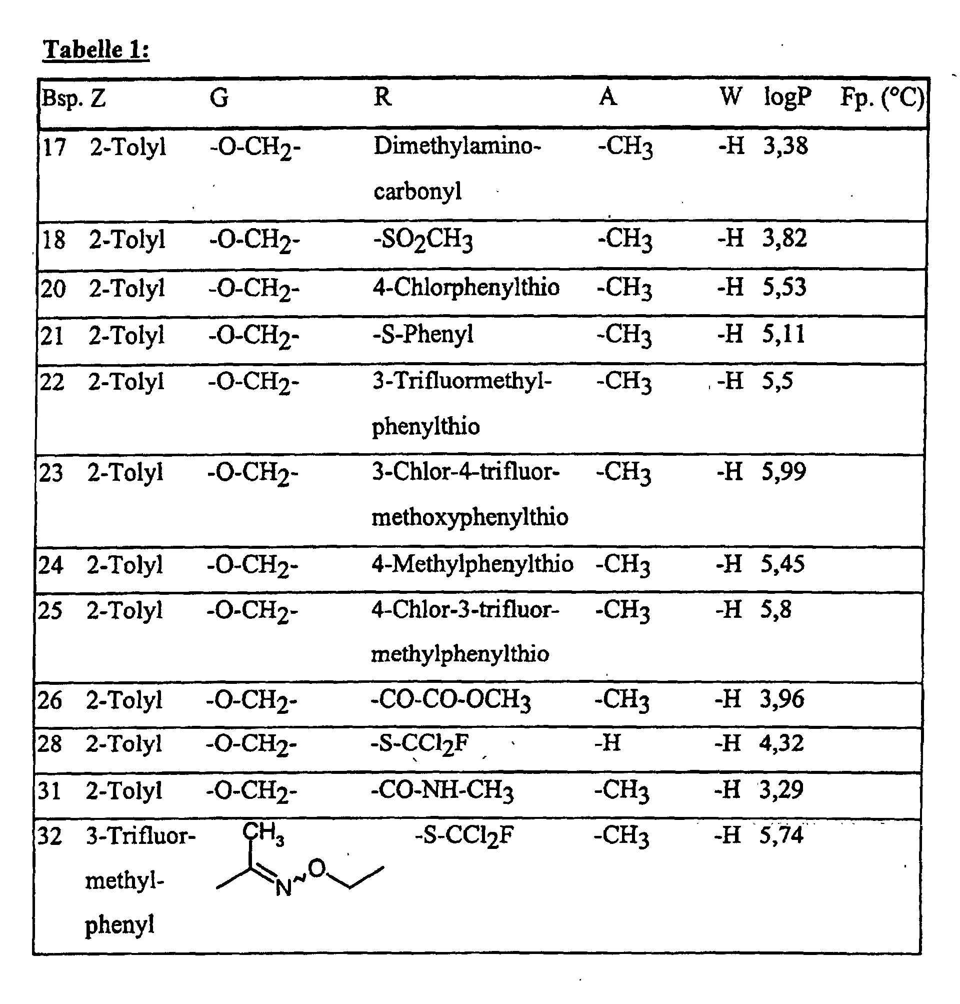

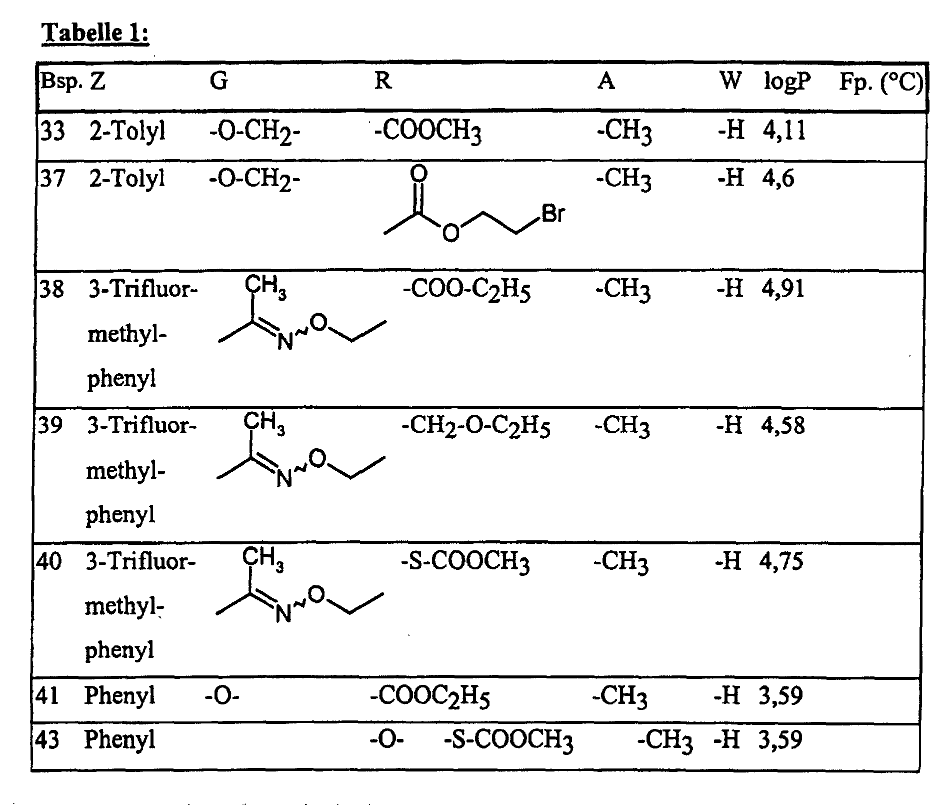

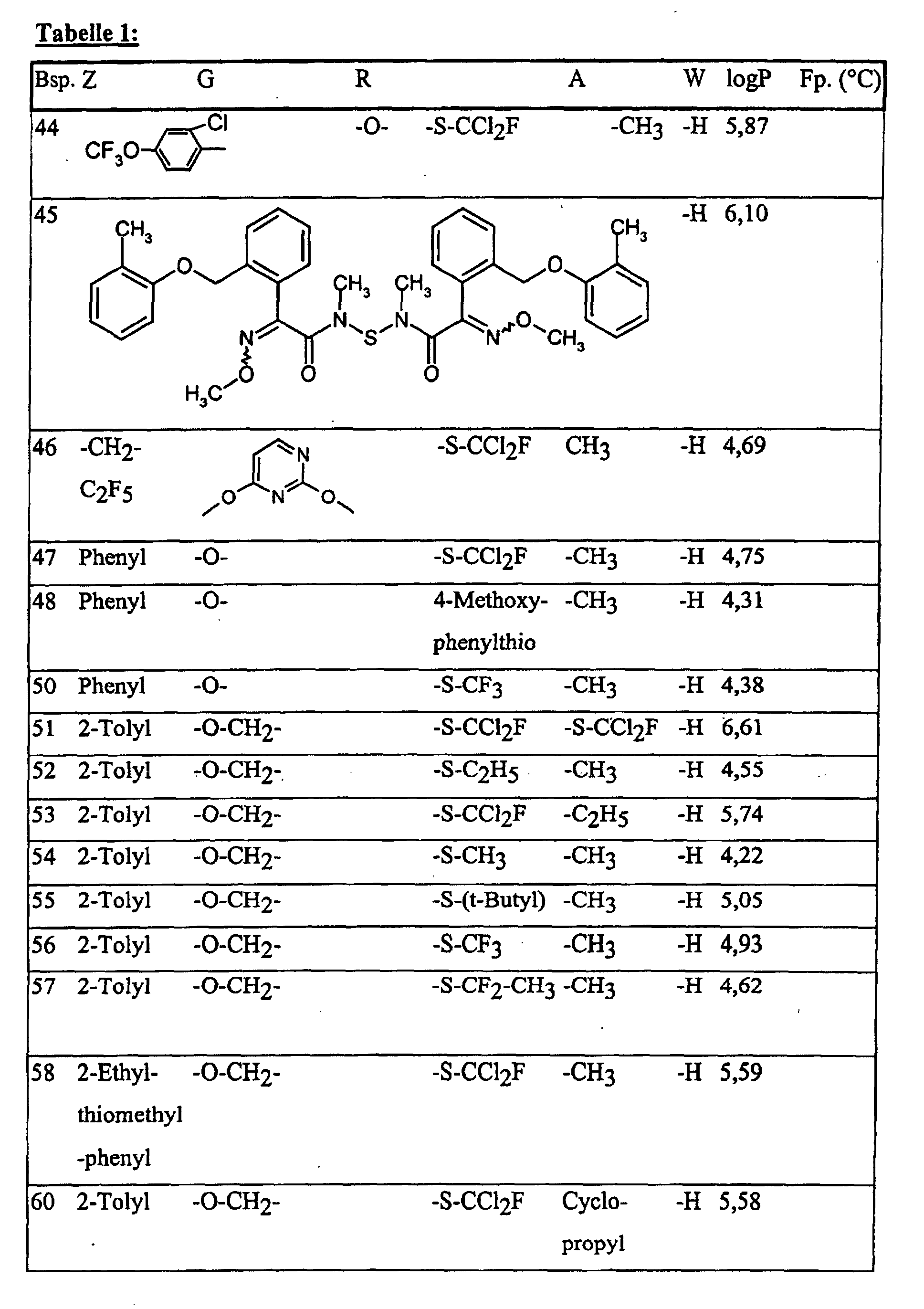

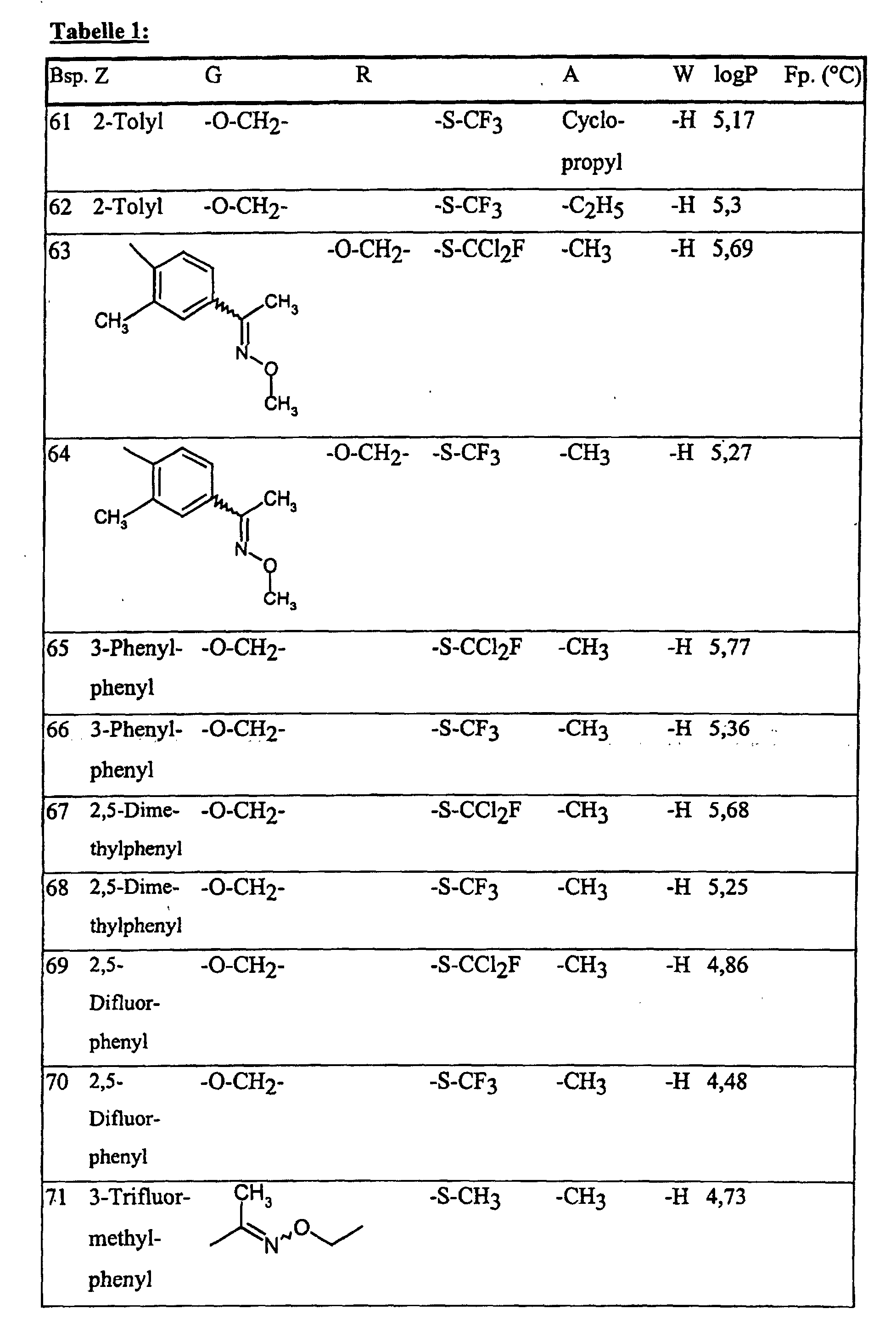

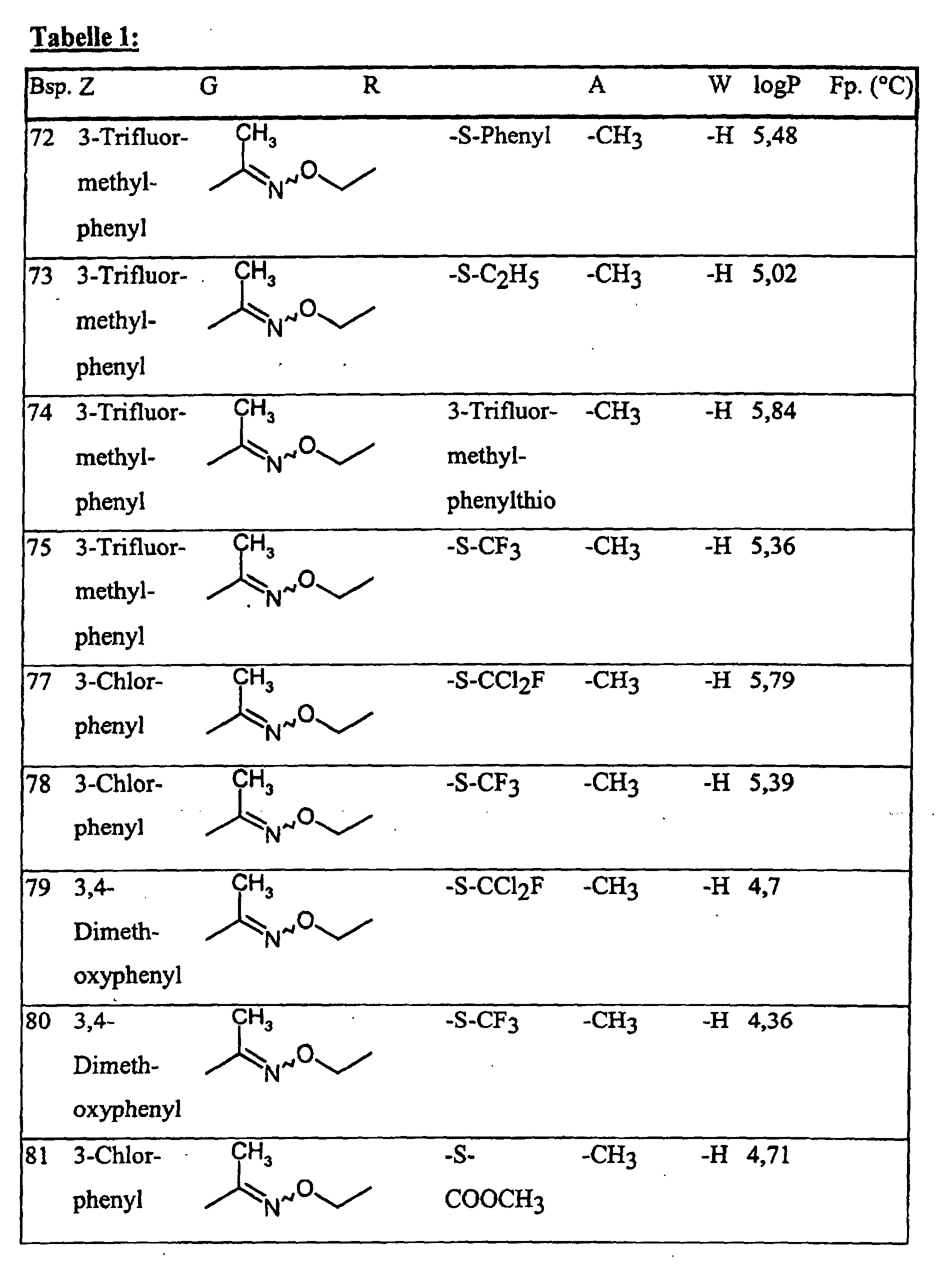

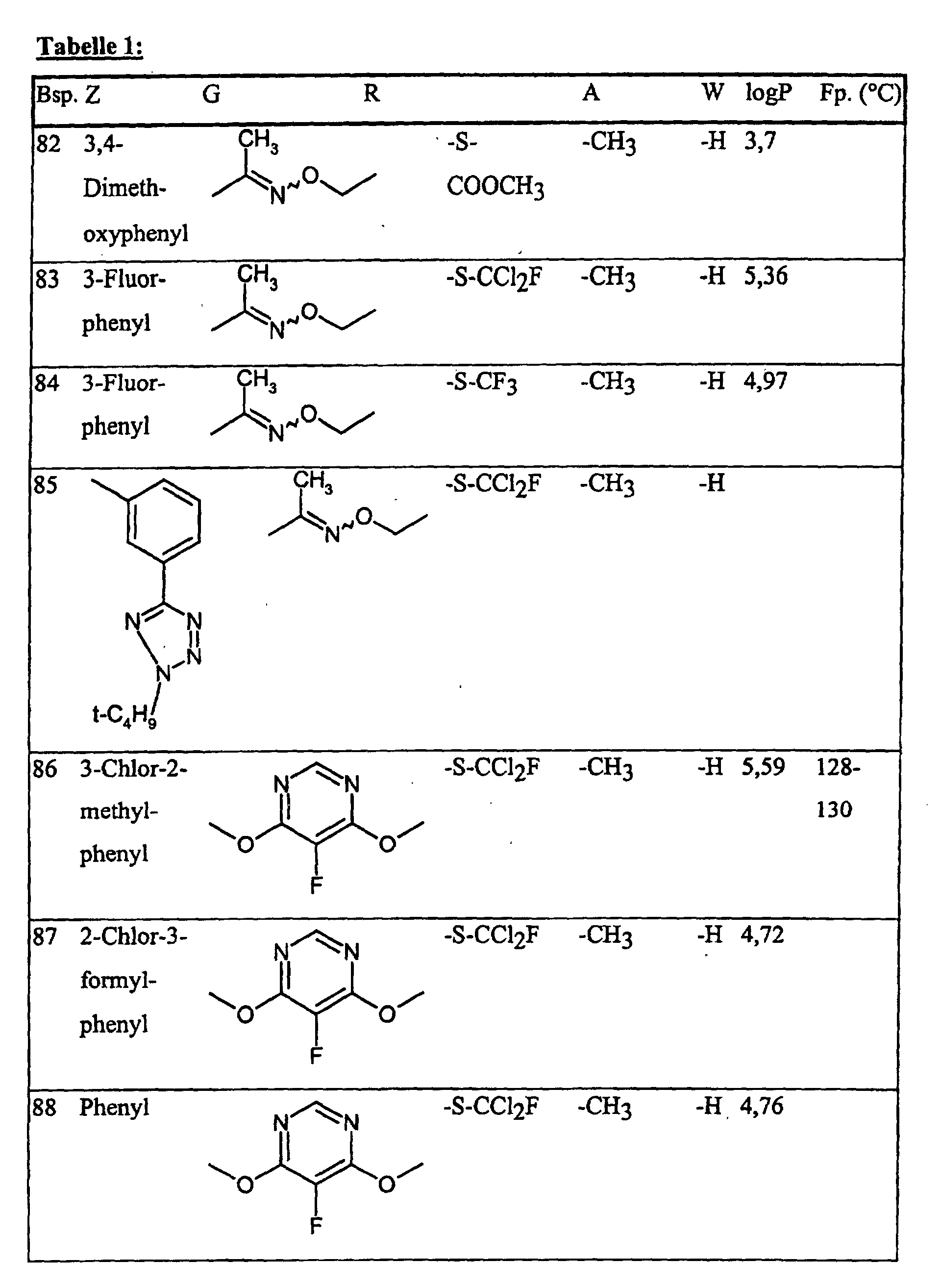

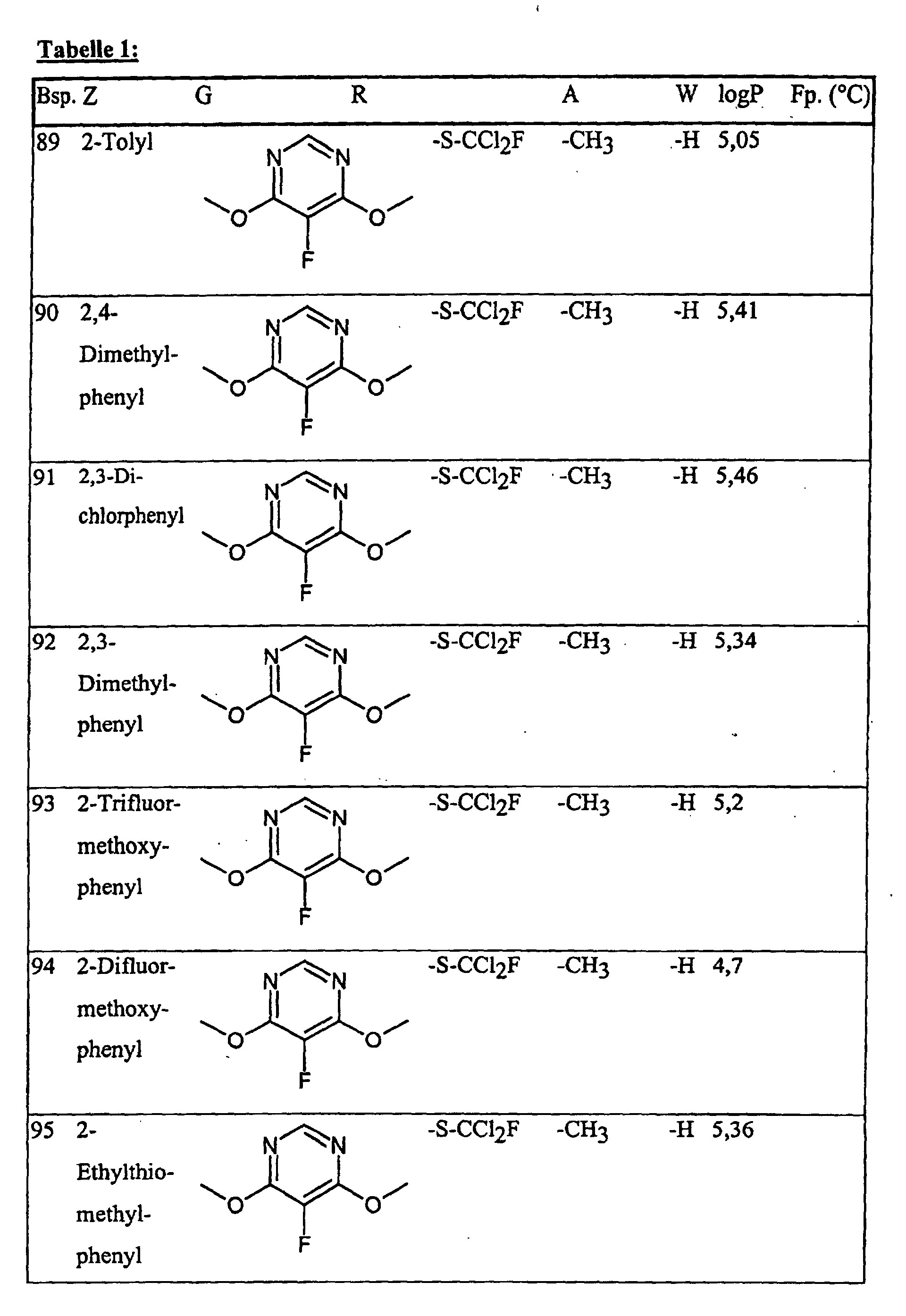

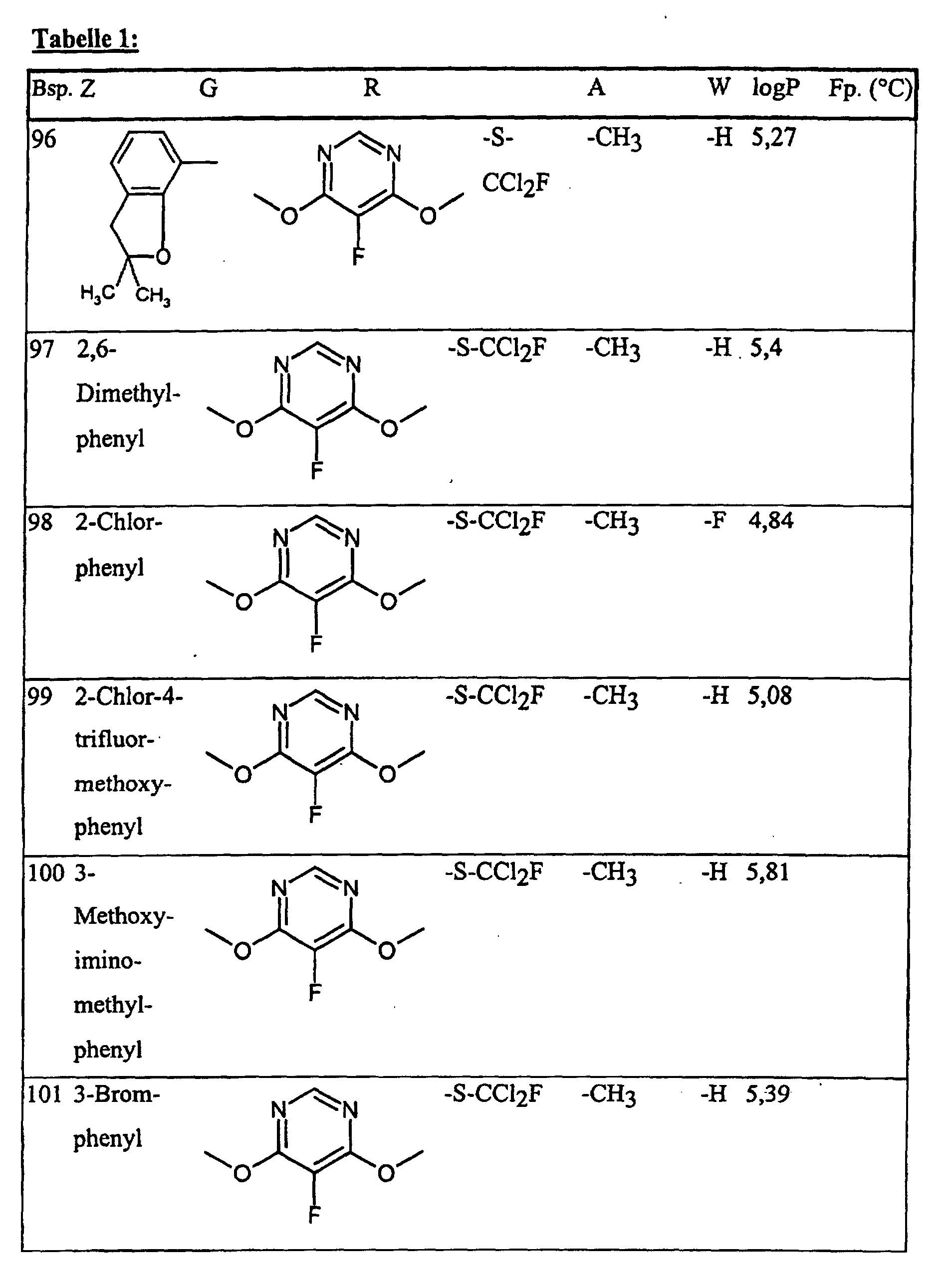

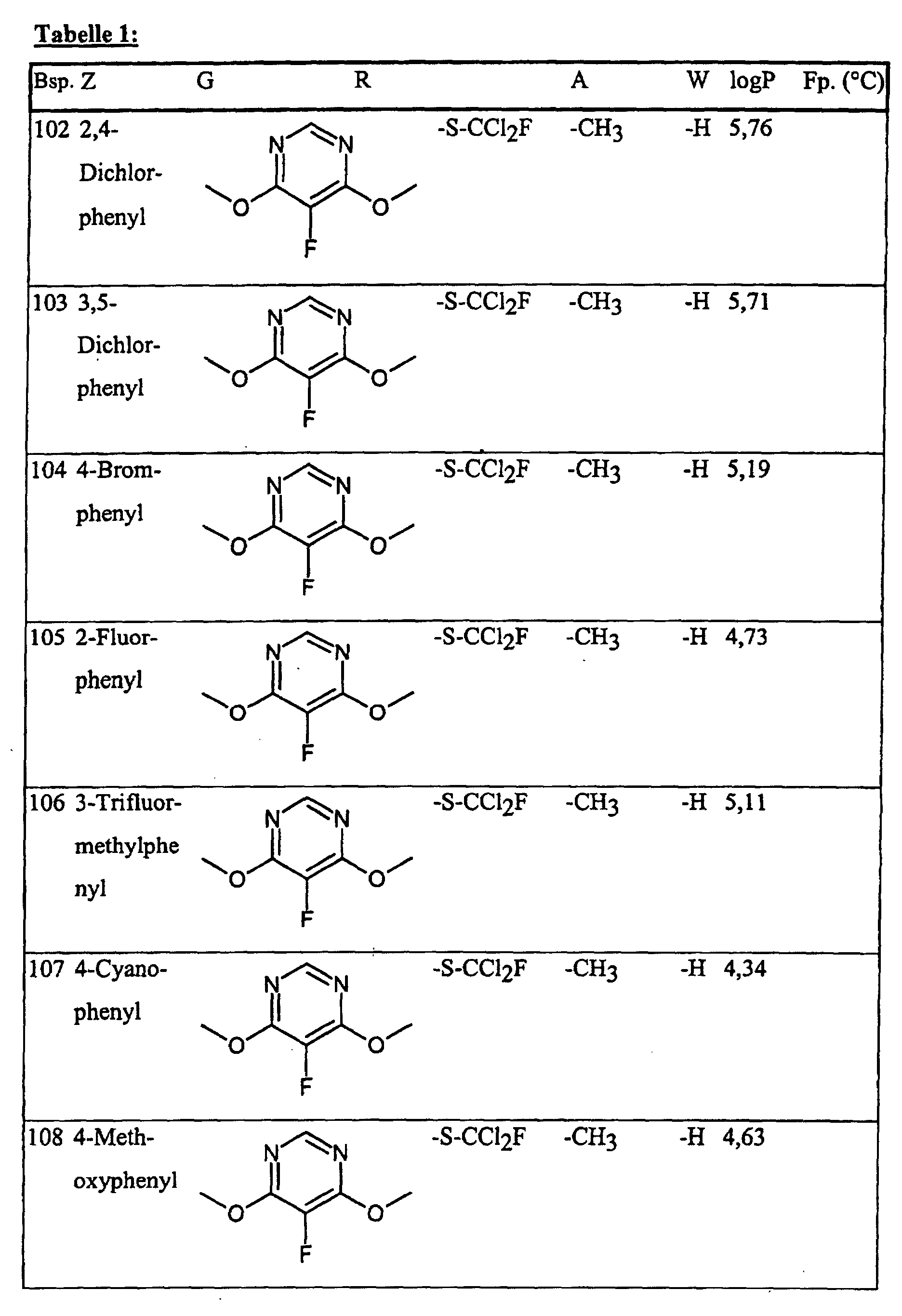

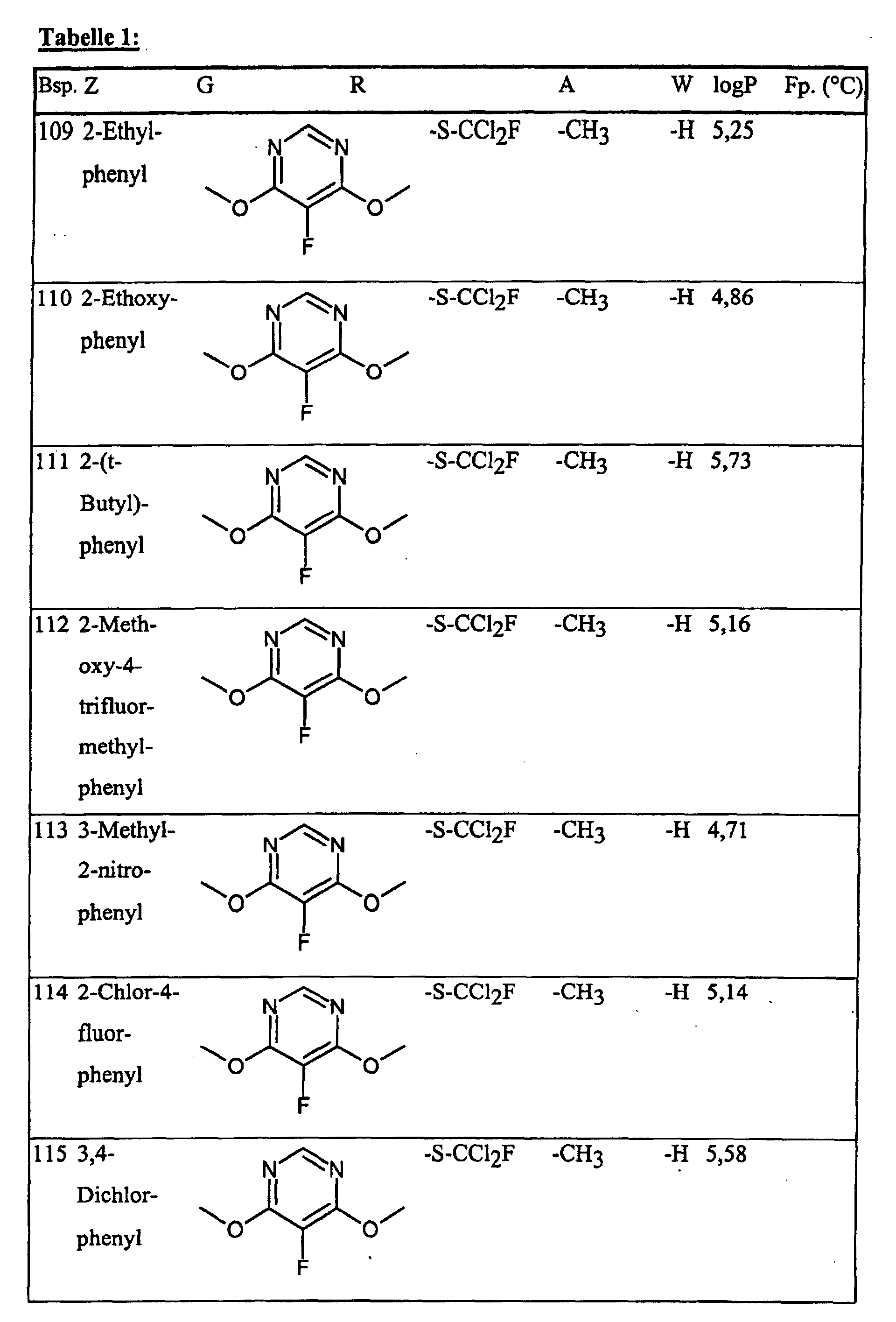

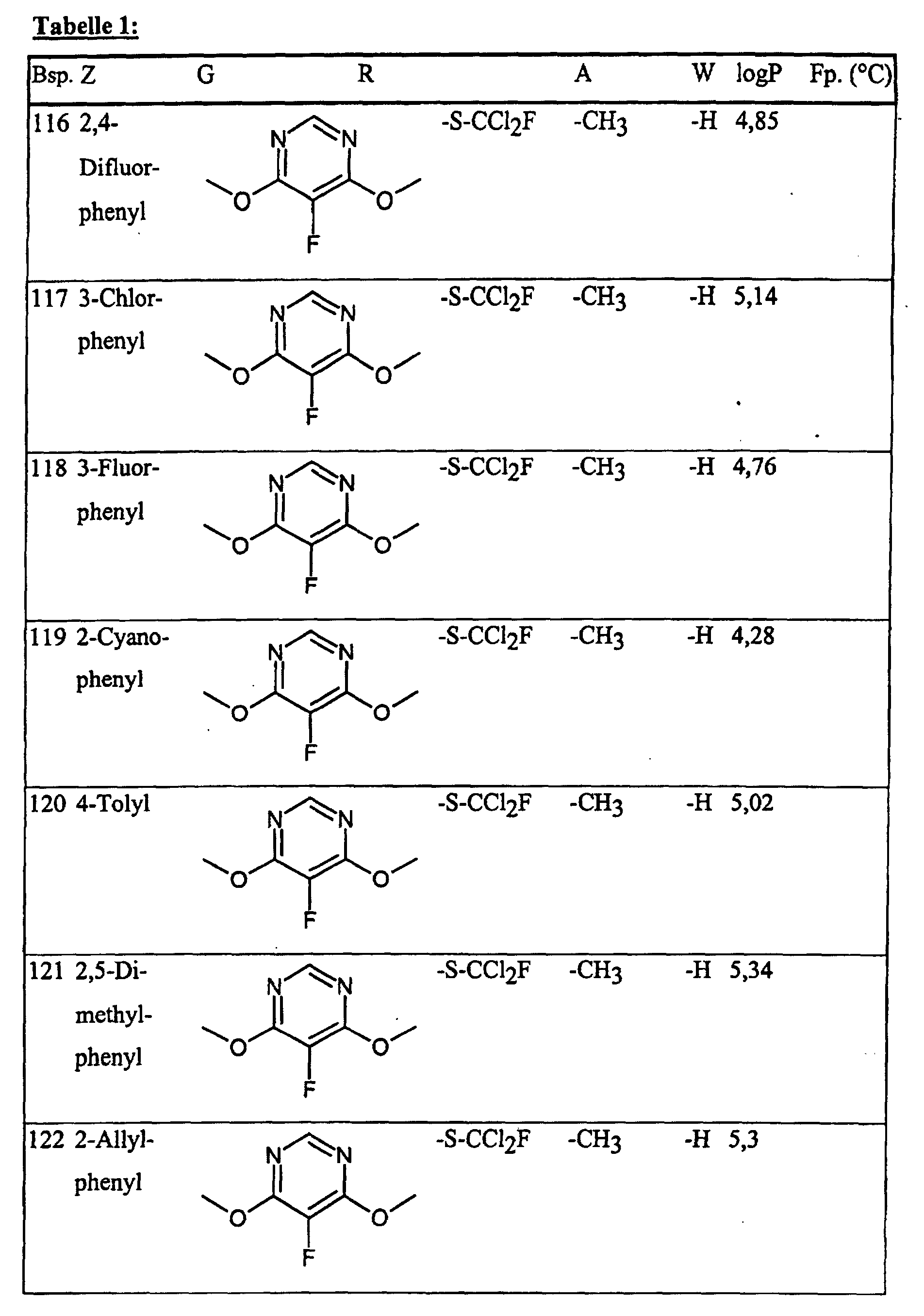

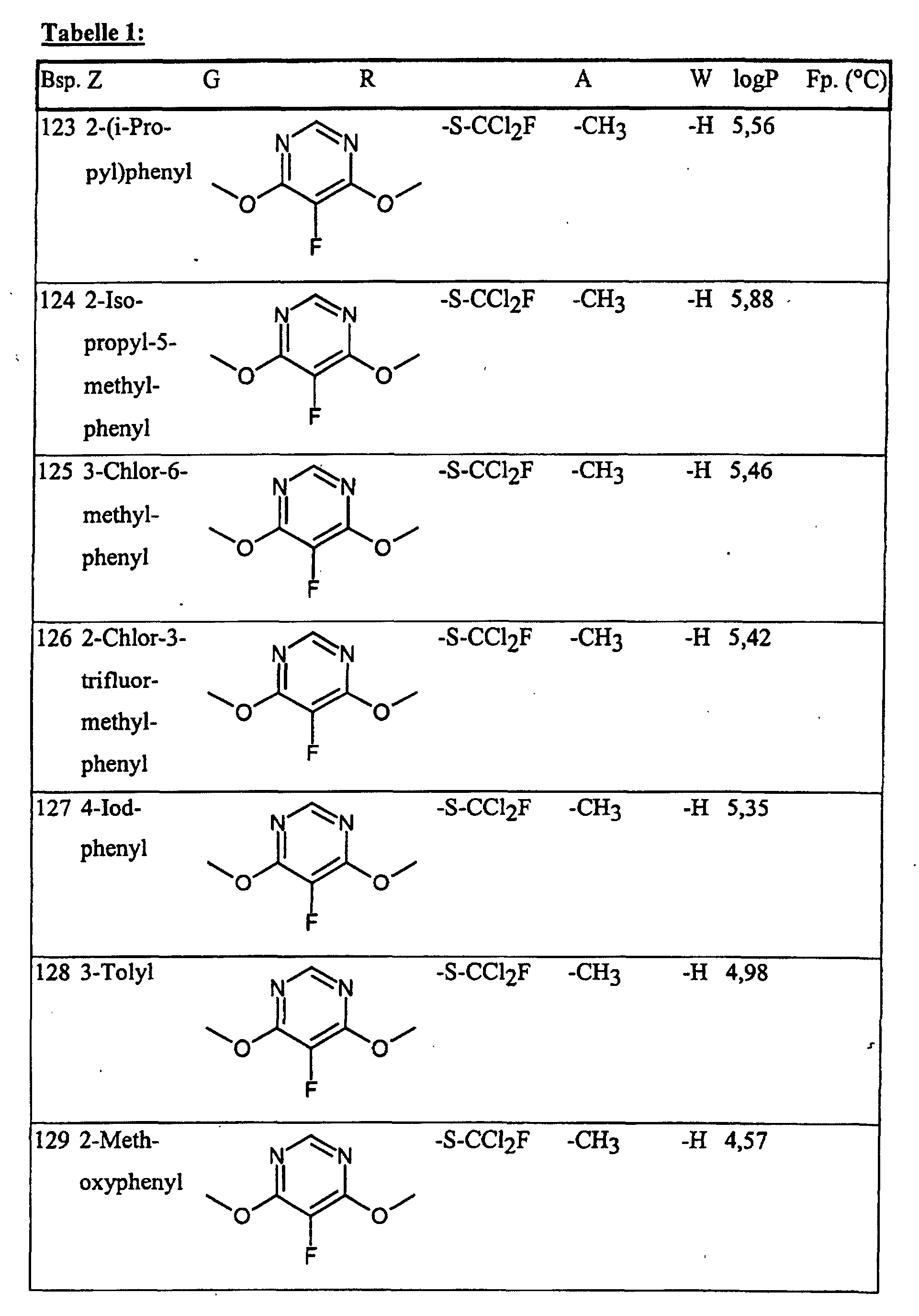

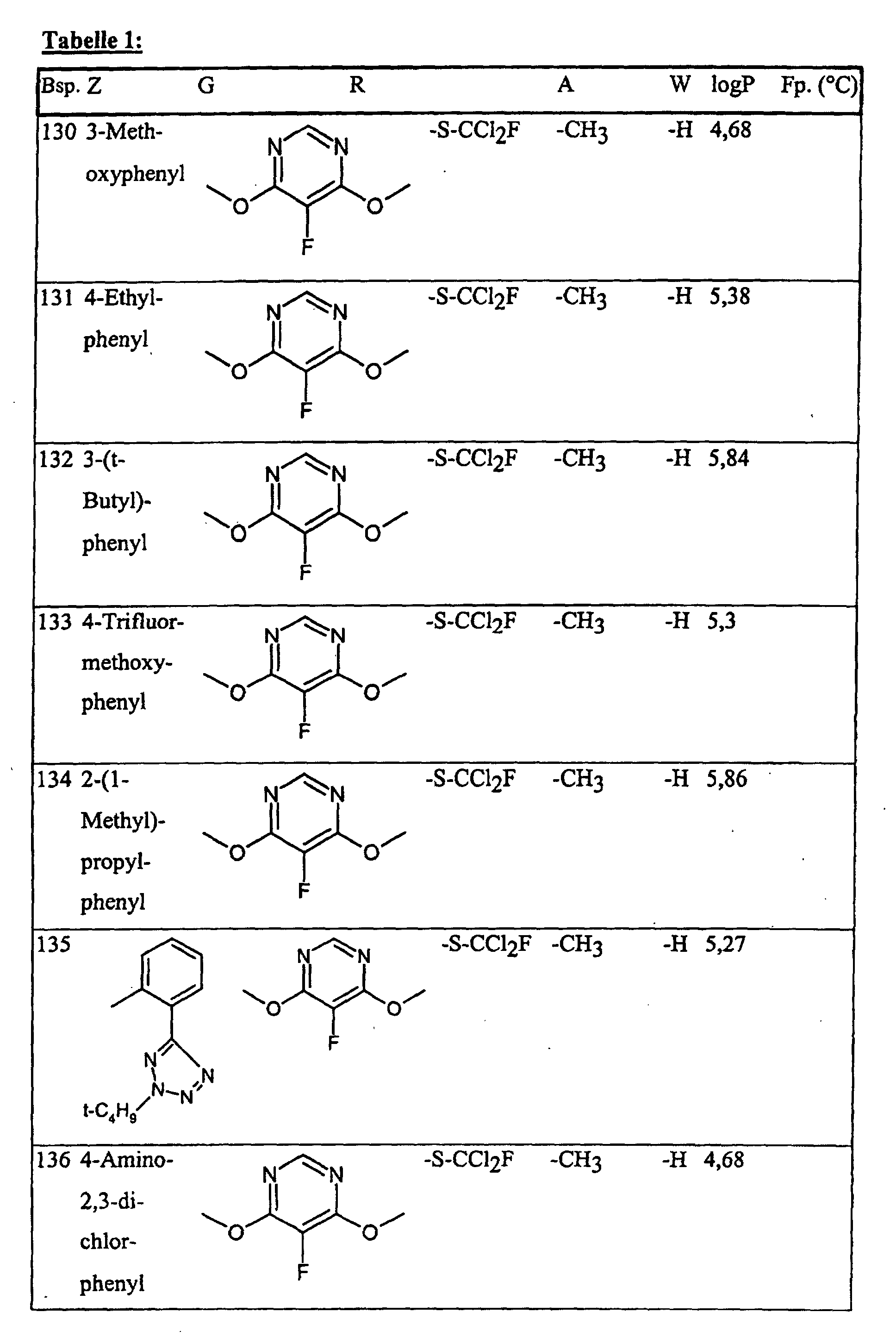

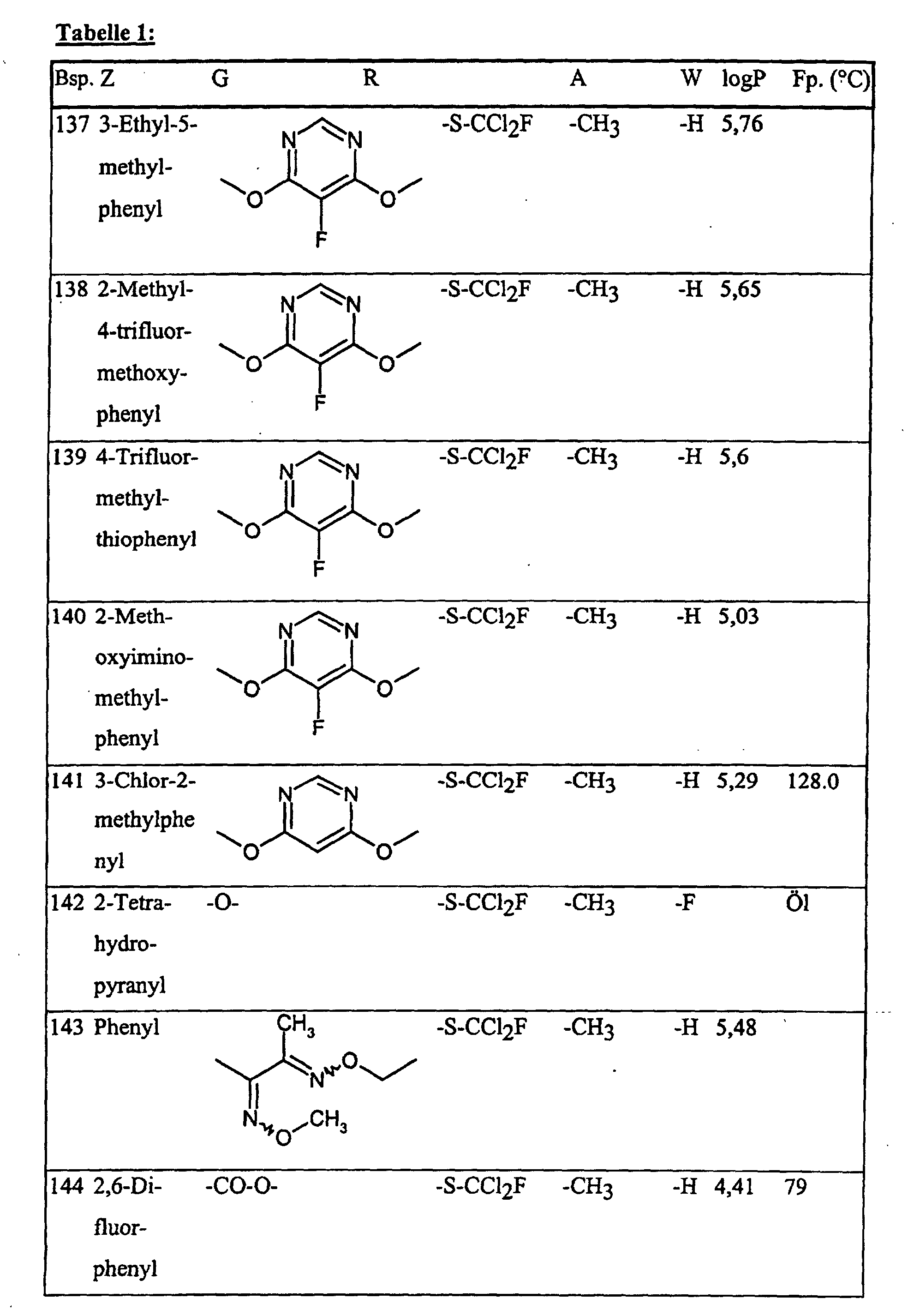

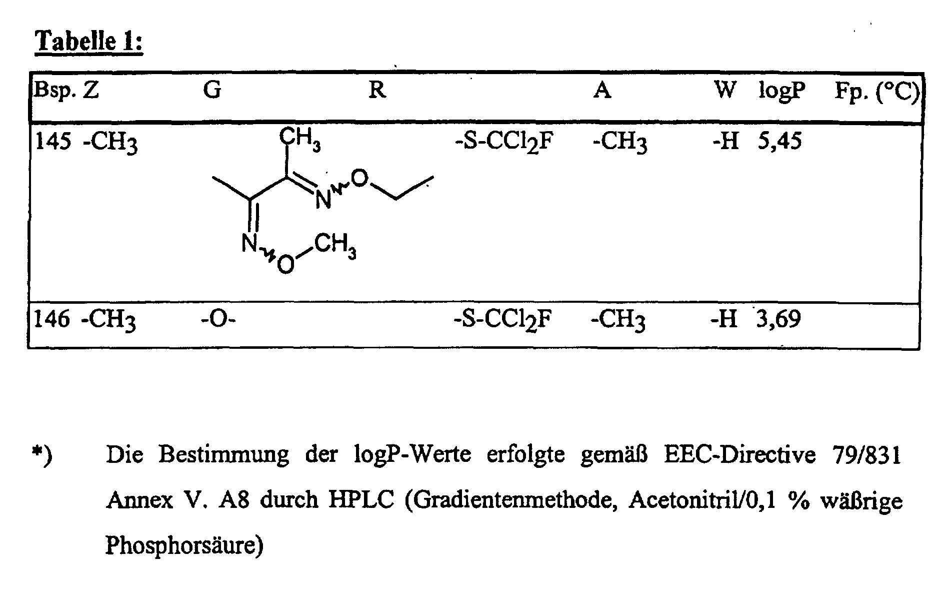

### Anwendungsbeispiele

### Beispiel A

### Plasmopara-Test (Rebe) / protektiv

| | |
|---|---|
| Lösungsmittel | 47 Gewichtsteile Aceton |
| Emulgator | 3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Plasmopara viticola inokuliert und verbleiben dann 1 Tag in einer Inkubationskabine bei ca. 20°C und 100 % relativer Luftfeuchtigkeit. Anschließend werden die Pflanzen 5 Tage im Gewächshaus bei ca. 21°C und ca. 90 % relativer Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (1), (3), (5), (6), (7), (8), (9), (11), (13), (20), (21), (22), (23), (24), (25), (26), (32), (40), (44), (46), (52), (54), (55), (56), (57), (61), (63), (64), (66), (67), (68), (69), (70), (71), (72), (73), (75), (86), (88), (91), (92), (93), (97), (100), (101), (102), (105), (106), (109), (117), (128), (141) und (143) aufgeführten erfindungsgemäßen Stoffe bei einer Aufwandmenge von 100 g/ha einen Wirkungsgrad von 90 % oder mehr.

### Beispiel B

### Podosphaera-Test (Apfel) / protektiv

| | |
|---|---|
| Lösungsmittel | 47 Gewichtsteile Aceton |
| Emulgator | 3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension des Apfelmehltauerregers ***Podosphaera leucotricha*** inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (1), (5), (6), (7), (11), (13), (20), (21), (22), (23), (26), (32), (40), (46), (52), (54), (56), (57), (61), (64), (66), (67), (68), (69), (70), (72), (75), (86), (88), (91), (92), (100), (101), (141) und (143) aufgeführten erfindungsgemäßen Stoffe bei einer Aufwandmenge von 100 g/ha einen Wirkungsgrad von 90 % oder mehr.

### Beispiel C

### Sphaerotheca-Test (Gurke) / protektiv

| | |
|---|---|
| Lösungsmittel | 47 Gewichtsteile Aceton |
| Emulgator | 3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von ***Sphaerotheca fuliginea*** inokuliert. Die Pflanzen werden dann bei ca. 23°C und einer relativen Luftfeuchtigkeit von ca. 70 % im Gewächshaus aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (1), (3), (5), (6), (7), (8), (9), (11), (12), (13), (20), (21), (22), (23), (24), (25), (26), (32), (40), (44), (45), (46), (52), (54), (55), (56), (57), (61), (63), (64), (66), (67), (68), (69), (71), (72), (73), (75), (86), (88), (91), (92), (93), (97), (100), (101), (102), (105), (106), (109), (117), (128), (141) und (143) aufgeführten erfindungsgemäßen Stoffe bei einer Aufwandmenge von 100 g/ha einen Wirkungsgrad von 90 % oder mehr.

### Beispiel D

### Venturia-Test (Apfel) / protektiv

| | |
|---|---|
| Lösungsmittel | 47 Gewichtsteile Aceton |
| Emulgator | 3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers ***Venturia inaequalis*** inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

12 Tage nach der Inokulation erfolgt die Auswertung. Dabei bedeutet 0% ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (1), (3), (5), (6), (7), (8), (9), (11), (12), (13), (14), (15), (20), (21), (22), (23), (24), (25), (26), (32), (40), (44), (52), (54), (55), (56), (57), (63), (64), (66), (71), (72), (73), (75), (86), (88), (91), (92), (93), (97), (100), (101), (102), (105), (106), (109), (117), (128), (141) und (143) aufgeführten erfindungsgemäßen Stoffe bei einer Aufwandmenge von 10 g/ha einen Wirkungsgrad von 90 % oder mehr.

### Beispiel E

### Pyricularia-Test (Reis) / protektiv

| | |
|---|---|
| Lösungsmittel | 25 Gewichtsteile N,N-Dimethylacetamid |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung in der angegebenen Aufwandmenge. Nach dem Antrocknen des Spritzbelages werden die Pflanzen mit einer wäßrigen Sporensuspension von Pyricularia oryzae inokuliert. Anschließend werden die Pflanzen in einem Gewächshaus bei 100 % relativer Luftfeuchtigkeit und 25°C aufgestellt.

4 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (1) und (5) aufgeführten erfindungsgemäßen Stoffe bei einer Aufwandmenge von 125 g/ha einen Wirkungsgrad von 90 % oder mehr.

### Beispiel F

### Erysiphe-Test (Gerste) / protektiv

| | |
|---|---|
| Lösungsmittel | 25 Gew.-Teile N,N-Dimethyacetamid |
| Emulgator | 0,6 Gew.-Teile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0% ein Wirkungsgrad, der demjenigen der Kontrolle entspricht, während ein Wirkungsgrad von 100 % bedeutet, daß kein Befall beobachtet wird.

Bei diesem Test zeigt der in dem Beispiel (5) aufgeführte erfindungsgemäße Stoff bei einer Aufwandmenge von 250 g/ha einen Wirkungsgrad von 90 % oder mehr.

### Beispiel G

### Erysiphe-Test (Weizen) / protektiv

| | |
|---|---|
| Lösungsmittel | 25 Gew.-Teile N,N-Dimethylaceamid |
| Emulgator | 0,6 Gew.-Teile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. tritici bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0% ein Wirkungsgrad, der demjenigen der Kontrolle entspricht, während ein Wirkungsgrad von 100 % bedeutet, daß kein Befall beobachtet wird.

Bei diesem Test zeigt der in dem Beispiel (5) aufgeführte erfindungsgemäße Stoff bei einer Aufwandmenge von 250 g/ha einen Wirkungsgrad von 90 % oder mehr.

### Beispiel H

### Puccinia-Test (Weizen) / protektiv

| | |
|---|---|
| Lösungsmittel | 25 Gewichtsteile N,N-Dimethylacetamid |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Puccinia recondita besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigt der in dem Beispiel (5) aufgeführte erfindungsgemäße Stoff bei einer Aufwandmenge von 250 g/ha einen Wirkungsgrad von 90 % oder mehr.

### Beispiel I

### Fusarium nivale (var. nivale)-Test (Weizen) / protektiv

| | |
|---|---|
| Lösungsmittel | 25 Gewichtsteile N,N-Dimethylacetamid |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Fusarium nivale (var. nivale) besprüht.

Die Pflanzen werden in einem Gewächshaus unter lichtdurchlässigen Inkubationshauben bei einer Temperatur von ca. 15°C und einer relativen Luftfeuchtigkeit von ca. 100 % aufgestellt.

4 Tage nach der Inokulation erfolgt die Auswertung. Dabei bedeutet 0% ein Wirkungsgrad, der demjenigen der Kontrolle entspricht, während ein Wirkungsgrad von 100 % bedeutet, daß kein Befall beobachtet wird.

Bei diesem Test zeigt der in den Beispielen (5), (67), (68), (100), (101), (102), (103), (104), (105), (106), (109), (114), (115), (116), (117), (118), (119), (120), (121), (122), (123), (124), (125), (126), (130), (134), (138) und (139) aufgeführte erfindungsgemäße Stoff bei einer Aufwandmenge von 250 g/ha einen Wirkungsgrad von 90 % oder mehr.

### Beispiel K

### Erysiphe-Test (Gerste) / kurativ

| | |
|---|---|
| Lösungsmittel | 25 Gew.-Teile N,N-Dimethylacetamid |
| Emulgator | 0,6 Gew.-Teile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0% ein Wirkungsgrad, der demjenigen der Kontrolle entspricht, während ein Wirkungsgrad von 100 % bedeutet, daß kein Befall beobachtet wird.

Bei diesem Test zeigt der in den Beispielen (5), (67), (68), (100), (101), (102), (103), (106), (107), (108), (109), (110), (113), (114), (115), (116), (117), (119), (123), (124) und (128) aufgeführte erfindungsgemäße Stoff bei einer Aufwandmenge von 250 g/ha einen Wirkungsgrad von 90 % oder mehr.

## Patentansprüche

1. Verbindungen der Formel (I), 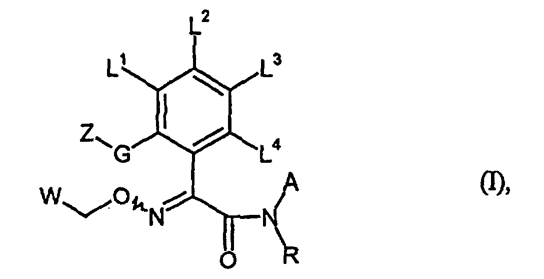 in welcher
A für Wasserstoff, Alkoxycarbonyl, Methyl, Ethyl oder Cyclopropyl steht, oder die gleiche Bedeutung wie R hat,
R für Alkylthio, Halogenalkylthio, gegebenenfalls substituiertes Arylthio, Alkoxycarbonylthio, -S-Y oder -S-N(R¹R²) steht, worin
R¹ für Alkyl steht,
R² für Alkyl, Alkoxycarbonyl, oder gemeinsam mit R¹, sowie dem Stickstoffatom, an das sie gebunden, sind für einen gegebenenfalls substituierten heterocyclischen Ring steht,
Y für die gleiche Gruppierung steht, die schon mit dem S-Atom verknüpft ist,
oder, wenn A für Methyl steht,
R auch für Alkylcarbonyl, Alkoxycarbonyl, Halogenalkoxycarbonyl, Alkoxycarbonylcarbonyl, Alkylaminocarbonyl, Dialkylaminocarbonyl, Alkylsulfonyl oder gegebenenfalls substituiertes Arylsulfonyl steht,
G für eine Einfachbindung, für Sauerstoff, Schwefel oder für jeweils gegebenenfalls durch Halogen, Hydroxy, Alkyl, Halogenalkyl oder Cycloalkyl substituiertes Alkandiyl, Alkendiyl, Alkindiyl oder eine der nachstehenden Gruppierungen, in denen jeweils die linke Seite an Z gebunden ist:
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁸)=N-O-, -C(R⁸)=N-O-CH₂-, -N(R⁹)-, -CQ-N(R⁹)-, -N(R⁹)-CQ-, -Q-CQ-N(R⁹)-, -N=C(R⁸)-Q-CH₂-, -CH(R⁸)-O-N=CH-, -C(R⁸)=N-N=CH-, -N(R⁹)-CQ-Q-, -CQ-N(R⁹)-CQ-Q-, -N(R⁹)-CQ-Q-CH₂-, -Q-C(R⁸)=N-O-CH₂-, -N(R⁹)-C(R⁸)=N-O-CH₂-, -O-CH₂-C(R⁸)=N-O-CH₂-, -N=N-C(R⁸)=N-O-CH₂-, -C(=N-O-R¹⁰)-C(R⁸)=N-O-CH₂-, -C(=N-O-R¹⁰)-C(R⁸)-O-N=CH-, -C(=N-O-R¹⁰)-C(R⁸)=N-N=CH-, -C(=N-O-R¹⁰)-C(R⁸)-O-N=C(CH₃)-, -T-Ar¹- oder -T-Ar¹-Q- steht, wobei
Ar¹ für gegebenenfalls substituiertes Arylen, Heteroarylen, Cycloalkylen oder Heterocycloalkylen (d.h. ein zweifach verknüpfter aliphatischer Ring, in dem ein oder mehrere Kohlenstoffatome durch Heteroatome, d.h. von Kohlenstoff verschiedene Atome ersetzt sind) steht,
n für die Zahlen 0, 1 oder 2 steht,
Q für Sauerstoff oder Schwefel steht,
R⁸ fürWasserstoff, Cyano oder jeweils gegebenenfalls substituiertes Alkyl, Alkoxy, Alkylthio, Alkylamino, Dialkylamino oder Cycloalkyl steht,
R⁹ für Wasserstoff, Hydroxy, Cyano oder jeweils gegebenenfalls substituiertes Alkyl, Alkoxy oder Cycloalkyl steht,
R¹⁰ für Wasserstoff, Alkyl oder gegebenenfalls substituiertes Benzyl steht und
T für eine Einfachbindung, für Sauerstoff, Schwefel, -CH₂-O-, -CH₂-S- oder für gegebenenfalls substituiertes Alkandiyl steht,
L¹, L², L³ und L⁴, gleich oder verschieden sind und unabhängig voneinander jeweils für Wasserstoff, Halogen oder Alkyl stehen,
W für Wasserstoff, Fluor, Cyano oder Thiocyanato steht und
Z für jeweils gegebenenfalls substituiertes Alkyl, Alkenyl, Alkinyl, Cycloalkyl, Aryl oder Heterocyclyl steht.

2. Verbindungen gemäß Anspruch 1,
in welcher
A für Wasserstoff, Methyl, Ethoxycarbonyl, Ethyl oder Cyclopropyl steht, oder die gleiche Bedeutung wie R hat,
R für Alkylthio, Halogenalkylthio, Alkoxycarbonylthio, mit jeweils 1 bis 4 Kohlenstoffatomen in den Alkylketten, gegebenenfalls durch Halogen, Cyano, Alkyl, Alkoxy oder Alkoxycarbonyl mit jeweils 1 bis 4 Kohlenstoffatomen, Halogenalkyl oder Halogenalkoxy mit jeweils 1 bis 4 Kohlenstoffatomen und 1 bis 9 gleichen oder verschiedenen Halogenatomen substituiertes Phenylthio, -S-Y, -S-N(R¹R²), worin
R¹ für Alkyl mit 1 bis 4 Kohlenstoffatomen steht,
R² für Alkyl mit 1 bis 4 Kohlenstoffatomen, Alkoxycarbonyl mit 1 bis 4 Kohlenstoffatomen im Alkylteil, oder gemeinsam mit R¹, sowie dem Stickstoffatom, an das sie gebunden, sind für einen gegebenenfalls durch Methyl oder Ethyl substituierten, heterocyclischen Ring mit 5 oder 6 Ringgliedem steht,
Y für die gleiche Gruppierung steht, die schon mit dem Schwefelatom verknüpft ist,
oder, wenn A für Methyl steht,
R auch für Alkylcarbonyl, Alkoxycarbonyl, Alkoxycarbonylcarbonyl, Halogenalkoxycarbonyl, Alkylaminocarbonyl, Dialkylaminocarbonyl, Alkylsulfonyl mit jeweils 1 bis 4 Kohlenstoffatomen in den Alkylketten oder gegebenenfalls durch Alkyl mit 1 bis 4 Kohlenstoffatomen substituiertes Phenylsulfonyl steht,
G für eine Einfachbindung, für Sauerstoff, Schwefel oder für jeweils gegebenenfalls durch Halogen, Hydroxy, C₁-C₄-Alkyl, C₁-C₄-Halogenalkyl oder C₃-C₆-Cycloalkyl substituiertes Alkandiyl, Alkendiyl, Alkindiyl mit jeweils bis zu 4 Kohlenstoffatomen oder eine der nachstehenden Gruppierungen, in denen jeweils die linke Seite an Z gebunden ist:
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁸)=N-O-, -C(R⁸)=N-O-CH₂-, -N(R⁹)-, -CQ-N(R⁹)-, -N(R⁹)-CQ-, -Q-CQ-N(R⁹)-, -N=C(R⁸)-Q-CH₂-, -CH(R⁸)-O-N=CH-, -C(R⁸)=N-N=CH-, -N(R⁹)-CQ-Q-, -CQ-N(R⁹)-CQ-Q-, -N(R⁹)-CQ-Q-CH₂-, -Q-C(R⁸)=N-O-CH₂-, -N(R⁹)-C(R⁸)=N-O-CH₂-, -O-CH₂-C(R⁸)=N-O-CH₂-, -N=N-C(R⁸)=N-O-CH₂-, -C(=N-O-R¹⁰)-C(R⁸)=N-O-CH₂-, -C(=N-O-R¹⁰)-C(R⁸)-O-N=CH-, -C(=N-O-R¹⁰)-C(R⁸)-N-N=CH-, -C(=N-O-R¹⁰)-C(R⁸)-O-N=C(CH₃)-, -T-Ar¹- oder -T-Ar¹-Q- steht, wobei
n für die Zahlen 0, 1 oder 2 steht,
Q für Sauerstoff oder Schwefel steht,
R⁸ für Wasserstoff, Cyano, für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₄-Alkyl oder C₁-C₄-Alkoxycarbonyl substituiertes Cycloalkyl mit 3 bis 6 Kohlenstoffatomen steht, und
R⁹ für Wasserstoff, Hydroxy, Cyano oder für gegebenenfalls durch Halogen, Cyano oder C₁-C₄-Alkoxy substituiertes Alkyl mit 1 bis 6 Kohlenstoffatomen oder für gegebenenfalls durch Halogen, Cyano, Carboxy, C₁-C₄-Alkyl oder C₁-C₄-Alkoxycarbonyl substituiertes Cycloalkyl mit 3 bis 6 Kohlenstoffatomen steht,
R¹⁰ für Wasserstoff, C₁-C₄-Alkyl oder gegebenenfalls durch Halogen, Alkyl oder Alkoxy mit jeweils 1 bis 6 Kohlenstoffatomen, Halogenalkyl oder Halogenalkoxy mit jeweils 1 bis 6 Kohlenstoffatomen und 1 bis 13 gleichen oder verschiedenen Halogenatomen substituiertes Benzyl steht,
T für eine Einfachbindung, für Sauerstoff, Schwefel, -CH₂-O-, -CH₂-S- oder für Alkandiyl mit 1 bis 3 Kohlenstoffatomen steht,
Ar¹ für jeweils gegebenenfalls einfach oder mehrfach, gleich oder verschieden substituiertes Phenylen, Naphthylen, Cycloalkylen oder für Heteroarylen oder Heterocycloalkylen mit 3 bis 7 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, sowie;
Cycloalkyl mit 3 bis 6 Kohlenstoffatomen und
L¹, L², L³ und L⁴, gleich oder verschieden sind und unabhängig voneinander jeweils für Wasserstoff, Fluor, Chlor, Brom, Methyl, Ethyl oder n-, i-Propyl, n-, i-, s-, oder t-Butyl stehen,
W für Wasserstoff, Fluor, Cyano oder Thiocyanato steht und
Z für gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 8 Kohlenstoffatomen;
für jeweils gegebenenfalls durch Halogen substituiertes Alkenyl oder Alkinyl mit jeweils bis zu 8 Kohlenstoffatomen;
Tetrahydropyranyl;
für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substituiertes Cycloalkyl mit 3 bis 6 Kohlenstoffatomen;
für jeweils gegebenenfalls einfach oder mehrfach, gleich oder verschieden substituiertes Phenyl, Naphthyl, Tetrahydrobenzofuranyl oder für Heterocyclyl mit 3 bis 7 Ringgliedern, von denen mindestens eines für Sauerstoff, Schwefel oder Stickstoff und gegebenenfalls ein oder zwei weitere für Stickstoff stehen steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, Formyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
t-Butyl substituiertes Tetrazol;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl oder Alkylsulfonyloxy, mit jeweils 1 bis 6 Kohlenstoffatomen in den einzelnen Alkylteilen;
Phenyl;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel-
oder eine Gruppierung 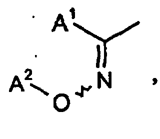 worin
A¹ für Alkyl mit 1 bis 4 Kohlenstoffatomen oder Cycloalkyl mit 1 bis 6 Kohlenstoffatomen steht und
A² für gegebenenfalls durch Cyano, Alkoxy, Alkylthio, Alkylamino, Dialkylamino oder Phenyl substituiertes Alkyl mit 1 bis 4 Kohlenstoffatomen, Alkenyl oder Alkinyl mit jeweils 2 bis 4 Kohlenstoffatomen steht.

3. Verbindungen der Formel (I),
in welcher
A für Wasserstoff, Methyl, Ethoxycarbonyl, Ethyl oder Cyclopropyl steht, oder die gleiche Bedeutung wie R hat,
R für Methylthio, Ethylthio, n- oder i-Propylthio, n-, i-, s- oder t-Butylthio Difluormethylthio, Trifluormethylthio, Difluorchlormethylthio, Dichlorfluormethylthio, Trichlormethylthio, 2,2,2-Trifluorethylthio, 1,1-Difluorethylthio, Methoxycarbonylthio, Ethoxycarbonylthio, gegebenenfalls einfach bis dreifach durch Fluor, Chlor, Brom, Cyano, Methyl, Ethyl, n- oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy, Trifluorethoxy, Methoxycarbonyl oder Ethoxycarbonyl substituiertes Phenylthio, -S-Y, -S-N(R¹R²),
worin
R¹ für Methyl, Ethyl, n- oder i-Propyl, n-, i-, s- oder t-Butyl steht,
R² für Methyl, Ethyl, n- oder i-Propyl, n-, i-, s- oder t-Butyl, Methoxycarbonyl oder Ethoxycarbonyl, oder gemeinsam mit R¹, sowie dem Stickstoffatom, an das sie gebunden, sind für gegebenenfalls durch Methyl oder Ethyl substituiertes Pyrrolidin, Piperidin oder Morpholin steht,
Y für die gleiche Gruppierung steht, die schon mit dem Schwefelatom verknüpft ist,
oder, wenn A für Methyl steht,
R auch für Acetyl, Propionyl, Methoxycarbonyl, Ethoxycarbonyl, i-Butoxycarbonyl, Methoxycarbonylcarbonyl, 2-Bromethoxycarbonyl, Methylaminocarbonyl, Dimethylaminocarbonyl, Methylsulfonyl, Ethylsulfonyl, Phenylsulfonyl oder Tolylsulfonyl steht,
G für Sauerstoff oder für jeweils gegebenenfalls durch Fluor, Chlor oder Brom substituiertes Dimethylen (Ethan-1,2-diyl), Ethen-1,2-diyl oder eine der nachstehenden Gruppierungen, in denen jeweils die linke Seite an Z gebunden ist:
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁸)=N-O-, -C(R⁸)=N-O-CH₂-, -N(R⁹)-, -CQ-N(R⁹)-, -N(R⁹)-CQ-, -Q-CQ-N(R⁹)-, -N=C(R⁸)-Q-CH₂-, -CH(R⁸)-O-N=CH-, -C(R⁸)=N-N=CH-, -N(R⁹)-CQ-Q-, -CQ-N(R⁹)-CQ-Q-, -N(R⁹)-CQ-Q-CH₂-, -Q-C(R⁸)=N-O-CH₂-, -N(R⁹)-C(R⁸)=N-O-CH₂-, -O-CH₂-C(R⁸)=N-O-CH₂-, -N=N-C(R⁸)=N-O-CH₂-, -C(=N-O-R¹⁰)-C(R⁸)=N-O-CH₂-, -C(=N-O-R¹⁰)-C(R⁸)-O-N=CH-, -C(=N-O-R¹⁰)-C(R⁸)-N-N=CH-, -C(=N-O-R¹⁰)-C(R⁸)-O-N=C(CH₃)-, -T-Ar¹- oder -T-Ar¹-Q- steht, wobei
n für die Zahlen 0, 1 oder 2 steht,
Q für Sauerstoff oder Schwefel steht,
R⁸ für Wasserstoff, Cyano, Methyl, Ethyl oder Cyclopropyl steht und
R⁹ für Wasserstoff, Methyl, Ethyl oder Cyclopropyl steht,
R¹⁰ für Wasserstoff, Methyl, Ethyl, n- oder i-Propyl, n-, i-, s- oder t-Butyl oder für gegebenenfalls durch Fluor, Chlor, Brom, Cyano, Methyl, Ethyl, n- oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy oder Trifluorethoxy substituiertes Benzyl steht,
T für eine Einfachbindung, für Sauerstoff, Schwefel, -CH₂-O-, -CH₂-S- oder für gegebenenfalls substituiertes Alkandiyl steht,
Ar¹ für jeweils gegebenenfalls einfach bis dreifach, gleich oder verschieden substituiertes Phenylen oder Pyridindiyl, für jeweils gegebenenfalls einfach substituiertes Pyrimidindiyl, Pyridazindiyl, Pyrazindiyl, 1,2,3-Triazindiyl, 1,2,4-Triazindiyl oder 1,3,5-Triazindiyl oder für 1,2,4-Thiadiazoldiyl, 1,3,4-Thiadiazoldiyl, 1,2,4-Oxadiazoldiyl, 1,3,4-Oxadiazoldiyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, Cyclopropyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl und
L¹, L², L³ und L⁴ gleich oder verschieden sind und unabhängig voneinander jeweils für Wasserstoff, Fluor, Chlor, Brom, Methyl oder Ethyl stehen,
W für Wasserstoff, Fluor, Cyano oder Thiocyanato steht und
Z für Methyl, Ethyl, n- oder i-Propyl, n-, i-, s- oder t-Butyl, Allyl, 2-Buten-1-yl, 2-(1-Methyl)-propyl, 2-Methyl-2-buten-1-yl, Propargyl, Tetrahydropyranyl, t-Butyl substituiertes Tetrazol oder für jeweils gegebenenfalls einfach bis dreifach, gleich oder verschieden substituiertes Phenyl, 1,2,4-Thiadiazolyl, 1,3,4-Thiadiazolyl, 1,2,4-Oxadiazolyl, 1,3,4-Oxadiazolyl, Pyridinyl, Pyrimidyl, Tetrahydrobenzofuranyl, Pyridazinyl, Pyrazinyl, 1,2,3-Triazinyl, 1,2,4-Triazinyl oder 1,3,5-Triazinyl steht, wobei die möglichen Substituenten vorzugsweise aus der nachstehenden Aufzählung ausgewählt sind:
Amino, Fluor, Chlor, Brom, Iod, Cyano, Nitro, Methyl, Ethyl, Formyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlörmethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl, Phenyl; jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy,
oder eine Gruppierung 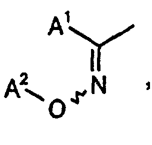 worin
A¹ für Methyl, Ethyl, n- oder i-Propyl, n-, i-, s- oder t-Butyl, Cyclopropyl oder Cyclobutyl steht und
A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht.

4. Verbindungen der Formel (I) gemäß Anspruch 1,
in welcher
A für Wasserstoff, Ethoxycarbonyl, Methyl, Ethyl oder Cyclopropyl steht, oder die gleiche Bedeutung wie R hat,
R für Methylthio, Ethylthio, n- oder i-Propylthio, n-, i-, s- oder t-Butylthio Difluormethylthio, Trifluormethylthio, Difluorchlormethylthio, Dichlorfluormethylthio, Trichlormethylthio, 2,2,2-Trifluorethylthio, 1,1-Difluorethylthio, Methoxycarbonylthio, Ethoxycarbonylthio, gegebenenfalls einfach bis dreifach durch Fluor, Chlor, Brom, Cyano, Methyl, Ethyl, n- oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy, Trifluorethoxy, Methoxycarbonyl oder Ethoxycarbonyl substituiertes Phenylthio, -S-Y, -S-N(R¹R²), worin
R¹ für Methyl, Ethyl, n- oder i-Propyl, n-, i-, s- oder t-Butyl steht,
R² für Methyl, Ethyl, n- oder i-Propyl, n-, i-, s- oder t-Butyl, Methoxycarbonyl oder Ethoxycarbonyl, oder gemeinsam mit R¹, sowie dem Stickstoffatom, an das sie gebunden, sind für gegebenenfalls durch Methyl oder Ethyl substituiertes Pyrrolidin, Piperidin oder Morpholin steht,
Y für die gleiche Gruppierung steht, die schon mit dem Schwefelatom verknüpft ist,
oder, wenn A für Methyl steht,
R auch für Acetyl, Propionyl, Methoxycarbonyl, Ethoxycarbonyl, i-Butoxycarbonyl, Methoxycarbonylcarbonyl, 2-Bromethoxycarbonyl, Methylaminocarbonyl, Dimethylaminocarbonyl, Methylsulfonyl, Ethylsulfonyl, Phenylsulfonyl oder Tolylsulfonyl steht,
G für Sauerstoff oder insbesondere -O-CH₂- steht,
L¹, L², L³ und L⁴ für Wasserstoff stehen,
W für Wasserstoff, Fluor, Cyano oder Thiocyanato steht und
Z für Methyl, Ethyl, n- oder i-Propyl, n-, i-, s- oder t-Butyl, Allyl, 2-Buten-1-yl, 2-Methyl-2-buten-1-yl, Propargyl, Tetrahydropyranyl oder für jeweils gegebenenfalls einfach bis dreifach, gleich oder verschieden substituiertes Phenyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s-oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl, Phenyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy
oder eine Gruppierung 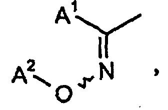 worin
A¹ für Methyl, Ethyl, n- oder i-Propyl, n-, i-, s- oder t-Butyl, Cyclopropyl oder Cyclobutyl steht und
A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht.

5. Verbindungen der Formel (I) gemäß Anspruch 1,
in welcher
A für Wasserstoff, Ethyl oder Methyl steht, oder die gleiche Bedeutung wie R hat,
R für Methylthio, Ethylthio, n- oder i-Propylthio, n-, i-, s- oder t-Butylthio Difluormethylthio, Trifluormethylthio, Difluorchlormethylthio, Dichlorfluormethylthio, Trichlormethylthio, 2,2,2-Trifluorethylthio, Methoxycarbonylthio, Ethoxycarbonylthio, gegebenenfalls einfach bis dreifach durch Fluor, Chlor, Brom, Cyano, Methyl, Ethyl, n-oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy, Trifluorethoxy, Methoxycarbonyl oder Ethoxycarbonyl substituiertes Phenylthio, -S-Y, -S-N(R¹R²), worin
R¹ für Methyl, Ethyl, n- oder i-Propyl, n-, i-, s- oder t-Butyl steht,
R² für Methyl, Ethyl, n- oder i-Propyl, n-, i-, s- oder t-Butyl, Methoxycarbonyl oder Ethoxycarbonyl, oder gemeinsam mit R¹, sowie dem Stickstoffatom, an das sie gebunden, sind für gegebenenfalls durch Methyl oder Ethyl substituiertes Pyrrolidin, Piperidin oder Morpholin steht,
Y für die gleiche Gruppierung steht, die schon mit dem Schwefelatom verknüpft ist,
oder, wenn A für Methyl steht,
R auch für Acetyl, Propionyl, Methoxycarbonyl, Ethoxycarbonyl, Methoxycarbonylcarbonyl, 2-Bromethoxycarbonyl, Methylaminocarbonyl, Dimethylaminocarbonyl, Methylsulfonyl, Ethylsulfonyl, Phenylsulfonyl oder Tolylsulfonyl steht,
G für -C(R⁸)=N-O-CH₂- steht,
R⁸ für Cyclopropyl oder insbesondere Methyl steht,
L¹, L², L³ und L⁴ für Wasserstoff stehen,
W für Wasserstoff, Fluor, Cyano oder Thiocyanato steht und
Z für Methyl, Ethyl, n- oder i-Propyl, n-, i-, s- oder t-Butyl, Allyl, 2-Buten-1-yl, 2-Methyl-2-buten-1-yl, Propargyl oder für jeweils gegebenenfalls einfach bis dreifach, gleich oder verschieden substituiertes Pyridyl, Pyrimidyl oder insbesondere Phenyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s-oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy.

6. Verbindungen der Formel (I) gemäß Anspruch 1,
in welcher
A für Wasserstoff, Methyl oder Ethyl steht, oder die gleiche Bedeutung wie R hat,
R für Methylthio, Ethylthio, n- oder i-Propylthio, n-, i-, s- oder t-Butylthio Difluormethylthio, Trifluormethylthio, Difluorchlormethylthio, Dichlorfluormethylthio, Trichlormethylthio, 2,2,2-Trifluorethylthio, Methoxycarbonylthio, Ethoxycarbonylthio, gegebenenfalls einfach bis dreifach durch Fluor, Chlor, Brom, Cyano, Methyl, Ethyl, n-oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy, Trifluorethoxy, Methoxycarbonyl oder Ethoxycarbonyl substituiertes Phenylthio, -S-Y, -S-N(R¹R²), worin
R¹ für Methyl, Ethyl, n- oder i-Propyl, n-, i-, s- oder t-Butyl steht,
R² für Methyl, Ethyl, n- oder i-Propyl, n-, i-, s- oder t-Butyl, Methoxycarbonyl oder Ethoxycarbonyl, oder gemeinsam mit R¹, sowie dem Stickstoffatom, an das sie gebunden, sind für gegebenenfalls durch Methyl oder Ethyl substituiertes Pyrrolidin, Piperidin oder Morpholin steht,
Y für die gleiche Gruppierung steht, die schon mit dem Schwefelatom verknüpft ist,
oder, wenn A für Methyl steht,
R auch für Acetyl, Propionyl, Methoxycarbonyl, Ethoxycarbonyl, Methoxycarbonylcarbonyl, 2-Bromethoxycarbonyl, Methylaminocarbonyl, Dimethylaminocarbonyl, Methylsulfonyl, Ethylsulfonyl, Phenylsulfonyl oder Tolylsulfonyl steht,
G für -T-Ar¹-O- steht,
Ar¹ für 1,2,4-Thiadiazoldiyl, 1,3,4-Thiadiazoldiyl, 1,2,4-Oxadiazoldiyl, 1,3,4-Oxadiazoldiyl oder für jeweils gegebenenfalls einfach oder zweifach, gleich oder verschieden durch Fluor, Chlor, Cyano, Methyl, Cyclopropyl, Methoxy, Methylthio, Trifluormethyl, Difluormethoxy, Trifluormethoxy, Difluorchlormethoxy substituiertes, Pyridindiyl, Pyrimidindiyl oder 1,3,5-Triazindiyl steht,
T für eine Einfachbindung, für Sauerstoff, Schwefel, -CH₂-O-, CH₂-S-, Methylen, Ethylen oder Propylen steht und
L¹, L², L³ und L⁴ für Wasserstoff stehen,
W für Wasserstoff, Fluor, Cyano oder Thiocyanato steht und
Z für Methyl, Ethyl, n- oder i-Propyl, n-, i-, s- oder t-Butyl, Allyl, 2-Buten-1-yl, 2-(1-Methyl)-propyl, 2-Methyl-2-buten-1-yl, Propargyl oder für jeweils gegebenenfalls einfach bis dreifach, gleich oder verschieden durch Amino, Fluor, Chlor, Brom, Iod, Cyano, Nitro, Methyl, Ethyl, Formyl, n- oder i-Propyl, n-, i-, s- oder t-Butyl, Trifluormethyl, Methoxy, t-Butyl substituiertes Tetrazol, Ethoxy, n-oder i-Propoxy, Difluormethoxy, Difluorchlormethoxy, Trifluorethoxy, Trifluormethoxy, Methoximinomethyl, Ethoximinomethyl, Methoximinoethyl, Ethoximinoethyl, oder jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy, substituiertes Pyridyl, Pyrimidyl, Thienyl, Tetrahydrobenzofuranyl oder insbesondere Phenyl steht.

7. Verbindungen der Formel (I) gemäß Anspruch 1,
in welcher
A für Wasserstoff oder Methyl steht, oder die gleiche Bedeutung wie R hat,
R für Methylthio, Ethylthio, n- oder i-Propylthio, n-, i-, s- oder t-Butylthio Difluormethylthio, Trifluormethylthio, Difluorchlormethylthio, Dichlorfluormethylthio, Trichlormethylthio, 2,2,2-Trifluorethylthio, Methoxycarbonylthio, Ethoxycarbonylthio, gegebenenfalls einfach bis dreifach durch Fluor, Chlor, Brom, Cyano, Methyl, Ethyl, n- oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy, Trifluorethoxy, Methoxycarbonyl oder Ethoxycarbonyl substituiertes Phenylthio, -S-Y, -S-N(R¹R²), worin
R¹ für Methyl, Ethyl, n- oder i-Propyl, n-, i-, s- oder t-Butyl steht,
R² für Methyl, Ethyl, n- oder i-Propyl, n-, i-, s- oder t-Butyl, Methoxycarbonyl oder Ethoxycarbonyl, oder gemeinsam mit R¹, sowie dem Stickstoffatom, an das sie gebunden, sind für gegebenenfalls durch Methyl oder Ethyl substituiertes Pyrrolidin, Piperidin oder Morpholin steht,
Y für die gleiche Gruppierung steht, die schon mit dem Schwefelatom verknüpft ist,
oder, wenn A für Methyl steht,
R auch für Acetyl, Propionyl, Methoxycarbonyl, Ethoxycarbonyl, Methoxycarbonylcarbonyl, 2-Bromethoxycarbonyl, Methylaminocarbonyl, Dimethylaminocarbonyl, Methylsulfonyl, Ethylsulfonyl, Phenylsulfonyl oder Tolylsulfonyl steht,
G für -C(=N-O-R¹⁰)-C(R⁸)=N-O-CH₂- steht, worin
R⁸ für Methyl oder Cyclopropyl steht und
R¹⁰ für Wasserstoff, Methyl, Ethyl, n- oder i-Propyl, n-, i-, s- oder t-Butyl oder für gegebenenfalls durch Fluor, Chlor, Brom, Cyano, Methyl, Ethyl, n- oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy oder Trifluorethoxy substituiertes Benzyl steht,
L¹, L², L³ und L⁴ für Wasserstoff stehen,
W für Wasserstoff, Fluor, Cyano oder Thiocyanato steht und
Z für Methyl, Ethyl, n- oder i-Propyl, n-, i-, s- oder t-Butyl, Allyl, 2-Buten-1-yl, 2-Methyl-2-buten-1-yl oder für jeweils gegebenenfalls einfach bis dreifach, gleich oder verschieden substituiertes Pyridyl, Pyrimidyl oder insbesondere Phenyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy oder durch t-Butyl substituiertes Tetrazol.

8. Verbindungen gemäß Anspruch 1, in welcher
G für Sauerstoff oder für eine der nachstehenden Gruppierungen, in denen jeweils die linke Seite an Z gebunden ist: -Q-CH₂-, -C(R⁸)=N-O-CH₂-, N=C(R⁸)-Q-CH₂-, -CH(R⁸)-O-N=CH-, -C(R⁸)=N-N=CH-, -C(=N-O-R¹⁰)-C(R⁸)=N-O-CH₂-, -C(=N-O-R¹⁰)-C(R⁸)-O-N=CH-, -C(=N-O-R¹⁰)-C(R⁸)-N-N=CH- oder -T-Ar¹-Q- steht, wobei R⁸, R⁹, R¹⁰, T, Ar¹ und Q die in Anspruch 1 angegebenen Bedeutungen haben.

9. Verbindungen der Formel (I) gemäß Anspruch 1, in welcher
A für Methyl steht.

10. Verbindungen der Formel (I) gemäß Anspruch 1, in welcher
G für Sauerstoff, -O-CH₂-, -C(CH₂)=N-O-CH₂- oder gegebenenfalls durch Fluor substituiertes Pyrimidinyloxy steht.

11. Verbindungen der Formel (I) gemäß Anspruch 1, in welcher
Z für unsubstituiertes oder einfach oder zweifach substituiertes Phenyl steht.

12. Mittel enthaltend Streckmittel und/oder Trägerstoffe sowie gegebenenfalls oberflächenaktive Stoffe, **gekennzeichnet durch** einen Gehalt an mindestens einer Verbindung wie in Anspruch 1 definiert.

13. Verfahren zur Bekämpfung von Schädlingen, **dadurch gekennzeichnet, daß** man Verbindungen wie in Anspruch 1 bzw. Mittel wie in Anspruch 12 definiert, auf Schädlinge und/oder ihren Lebensraum einwirken läßt.

14. Verwendung von Verbindungen wie in den Ansprüchen 1 bis 11 bzw. Mittel wie in Anspruch 12 definiert, zur Bekämpfung von Schädlingen.

15. Verfahren zur Herstellung von Mitteln, **dadurch gekennzeichnet, daß** man Verbindungen wie in den Ansprüchen 1 bis 11 definiert, mit Streckmitteln und/oder oberflächenaktiven Mitteln vermischt.

16. Verfahren zur Herstellung von Verbindungen der Formel (I) wie in Anspruch 1 definiert, **dadurch gekennzeichnet, daß** man Amide der Formel (II) 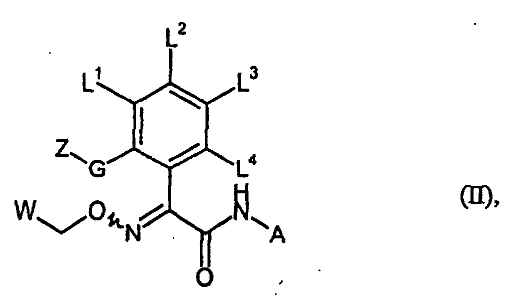 in welcher
A, G, L¹, L², L³, L⁴ und Z die in Anspruch 1 angegebenen Bedeutungen haben,
mit einem elektrophilen Reagenz der Formel (III),
R-X (III)
in welcher
R die in Anspruch 1 angegebene Bedeutung hat, und
X für eine Abgangsgruppe steht,
gegebenenfalls in Gegenwart eines Verdünnungsmittels und gegebenenfalls in Gegenwart einer Base umsetzt.

## Claims

1. Compounds of the formula (I), 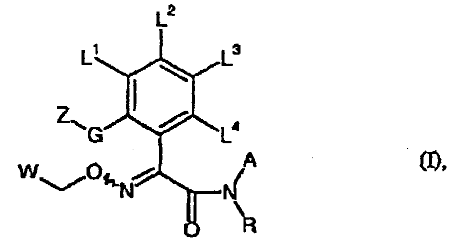 in which
A represents hydrogen, alkoxycarbonyl, methyl, ethyl or cyclopropyl, or has the same meaning as R,
R represents alkylthio, halogenoalkylthio, optionally substituted arylthio, alkoxycarbonylthio, -S-Y or -S-N(R¹R²), in which
R¹ represents alkyl,
R² represents alkyl, alkoxycarbonyl, or together with R¹ and the nitrogen atom to which they are attached represents an optionally substituted heterocyclic ring,
Y represents the same grouping which is already linked to the S atom,
or, if A represents methyl,
R also represents alkylcarbonyl, alkoxycarbonyl, halogenoalkoxycarbonyl, alkoxycarbonylcarbonyl, alkylaminocarbonyl, dialkylaminocarbonyl, alkylsulphonyl or optionally substituted arylsulphonyl,
G represents a single bond, represents oxygen, sulphur or represents in each case optionally halogen-, hydroxyl-, alkyl-, halogenoalkyl- or cycloalkyl-substituted alkanediyl, alkenediyl, alkinediyl or one of the groupings below, in which in each case the left side is attached to Z:
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁸)=N-O-, -C(R⁸)=N-O-CH₂-, -N(R⁹)-, -CQ-N(R⁹)-, -N(R⁹)-CQ-, -Q-CQ-N(R⁹)-, -N=C(R⁸)-Q-CH₂-, -CH(R⁸)-O-N=CH-, -C(R⁸)=N-N=CH-, -N(R⁹)-CQ-Q-, -CQ-N(R⁹)-CQ-Q-, -N(R⁹)-CQ-Q-CH₂-. -Q-C(R⁸)=N-O-CH₂-, -N(R⁹)-C(R⁸)=N-O-CH₂-, -O-CH₂-C(R⁸)=N-O-CH₂-, -N=N-C(R⁸)=N-O-CH₂-, -C(=N-O-R¹⁰)-C(R⁸)=N-O-CH₂-, -C(=N-O-R¹⁰)-C(R⁸)-O-N=CH-, -C(=N-O-R¹⁰)-C(R⁸)=N-N=CH-, -C(=N-O-R¹⁰)-C(R⁸)-O-N=C(CH₃)-, -T-Ar¹- or -T-Ar¹-Q-,
where
Ar¹ represents optionally substituted arylene, heteroarylene, cycloalkylene or heterocycloalkylene (i.e. a doubly attached aliphatic ring in which one or more carbon atoms are replaced by heteroatoms. i.e. atoms different from carbon),
n represents the number 0, 1 or 2,
Q represents oxygen or sulphur,
R⁸ represents hydrogen, cyano or in each case optionally substituted alkyl, alkoxy, alkylthio, alkylamino, dialkylamino or cycloalkyl,
R⁹ represents hydrogen, hydroxyl, cyano or in each case optionally substituted alkyl, alkoxy or cycloalkyl,
R¹⁰ represents hydrogen, alkyl or optionally substituted benzyl and
T represents a single bond, represents oxygen, sulphur, -CH₂-O-, -CH₂-S- or represents optionally substituted alkanediyl,
L¹, L², L³ and L⁴ are identical or different and independently of one another each represent hydrogen, halogen or alkyl,
W represents hydrogen, fluorine, cyano or thiocyanato and
Z represents in each case optionally substituted alkyl, alkenyl, alkinyl, cycloalkyl, aryl or heterocyclyl.

2. Compounds according to Claim 1,
in which
A represents hydrogen, methyl, ethoxycarbonyl, ethyl or cyclopropyl, or has the same meaning as R,
R represents alkylthio, halogenoalkylthio, alkoxycarbonylthio, having in each case 1 to 4 carbon atoms in the alkyl chains, represents phenylthio which is optionally substituted by halogen, cyano, alkyl, alkoxy or alkoxycarbonyl having in each case 1 to 4 carbon atoms, halogenoalkyl or halogenoalkoxy having in each case 1 to 4 carbon atoms and 1 to 9 identical or different halogen atoms, -S-Y, -S-N(R¹R²) where
R¹ represents alkyl having 1 to 4 carbon atoms,
R² represents alkyl having 1 to 4 carbon atoms, alkoxycarbonyl having 1 to 4 carbon atoms in the alkyl moiety, or together with R¹ and the nitrogen atom to which they are attached represents an optionally methyl- or ethyl-substituted heterocyclic ring having 5 or 6 ring members,
Y represents the same grouping which is already linked to the sulphur atom,
or, if A represents methyl,
R also represents alkylcarbonyl, alkoxycarbonyl, alkoxycarbonylcarbonyl, halogenoalkoxycarbonyl, alkylaminocarbonyl, dialkylaminocarbonyl, alkylsulphonyl having in each case 1 to 4 carbon atoms in the alkyl chains or phenylsulphonyl which is optionally substituted by alkyl having 1 to 4 carbon atoms,
G represents a single bond, represents oxygen, sulphur or represents in each case optionally halogen-, hydroxyl-, C₁-C₄-alkyl-, C₁-C₄-halogenoalkyl- or C₃-C₆-cycloalkyl-substituted alkanediyl, alkenediyl, alkinediyl having in each case up to 4 carbon atoms or one of the groupings below in which in each case the left side is attached to Z:
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁸)=N-O-, -C(R⁸)=N-O-CH₂-, -N(R⁹)-, -CQ-N(R⁹)-, -N(R⁹)-CQ-, -Q-CQ-N(R⁹)-, -N=C(R⁸)-Q-CH₂-, -CH(R⁸)-O-N=CH-, -C(R⁸)=N-N=CH-, -N(R⁹)-CQ-Q-, -CQ-N(R⁹)-CQ-Q-, -N(R⁹)-CQ-Q-CH₂-, -Q-C(R⁸)=N-O-CH₂-, -N(R⁹)-C(R⁸)=N-O-CH₂-, -O-CH₂-C(R⁸)=N-O-CH₂-, -N=N-C(R⁸)=N-O-CH₂-, -C(=N-O-R¹⁰)-C(R⁸)=N-O-CH₂-, -C(=N-O-R¹⁰)-C(R⁸)-O-N=CH-, -C(=N-O-R¹⁰)-C(R⁸)-N-N=CH-, -C(=N-O-R¹⁰)-C(R⁸)-O-N=C(CH₃)-, -T-Ar¹- or -T-Ar¹-Q-, where
n represents the number 0,1 or 2,
Q represents oxygen or sulphur,
R⁸ represents hydrogen, cyano, represents in each case optionally halogen-, cyano- or C₁-C₄-alkoxy-substituted alkyl, alkoxy, alkylthio, alkylamino or dialkylamino having in each case 1 to 6 carbon atoms in the alkyl groups or represents in each case optionally halogen-, cyano-, carboxyl-, C₁-C₄-alkyl- or C₁-C₄-alkoxycarbonyl-substituted cycloalkyl having 3 to 6 carbon atoms, and
R⁹ represents hydrogen, hydroxyl, cyano or represents optionally halogen-, cyano- or C₁-C₄-alkoxy-substituted alkyl having 1 to 6 carbon atoms or represents optionally halogen-, cyano-, carboxyl-, C₁-C₄-alkyl- or C₁-C₄-alkoxycarbonyl-substituted cycloalkyl having 3 to 6 carbon atoms,
R¹⁰ represents hydrogen, C₁-C₄-alkyl or benzyl which is optionally substituted by halogen, alkyl or alkoxy having in each case 1 to 6 carbon atoms, halogenoalkyl or halogenoalkoxy having in each case 1 to 6 carbon atoms and 1 to 13 identical or different halogen atoms,
T represents a single bond, represents oxygen, sulphur, -CH₂-O-, -CH₂-S- or represents alkanediyl having 1 to 3 carbon atoms,
Ar¹ represents phenylene, naphthylene, cycloalkylene, each of which is optionally mono- or polysubstituted by identical or different substituents or represents heteroarylene or heterocycloalkylene having 3 to 7 ring members, at least one of which represents oxygen, sulphur or nitrogen, and one or two more of which optionally represent nitrogen, where the possible substituents are preferably selected from the list below:
halogen, cyano, nitro, amino, hydroxyl, formyl, carboxyl, carbamoyl, thiocarbamoyl, formyl;
in each case straight-chain or branched alkyl, alkoxy, alkylthio, alkylsulphinyl or alkylsulphonyl having in each case 1 to 6 carbon atoms;
in each case straight-chain or branched alkenyl or alkenyloxy having in each case 2 to 6 carbon atoms;
in each case straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms;
in each case straight-chain or branched halogenoalkenyl or halogenoalkenyloxy having in each case 2 to 6 carbon atoms and 1 to 11 identical or different halogen atoms;
in each case straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties, and
cycloalkyl having 3 to 6 carbon atoms and
L¹, L², L³ and L⁴ are identical or different and independently of one another each represent hydrogen, fluorine, chlorine, bromine, methyl, ethyl or n-, i-propyl, n-, i-, s-, or t-butyl,
W represents hydrogen, fluorine, cyano or thiocyanato and
Z represents alkyl having 1 to 8 carbon atoms which is optionally mono- or polysubstituted by identical or different substituents from the list consisting of halogen, cyano, hydroxyl, amino, C₁-C₄-alkoxy, C₁-C₄-alkylthio, C₁-C₄-alkylsulphinyl and C₁-C₄-alkylsulphonyl (which may in each case optionally be substituted by halogen);
represents in each case optionally halogen-substituted alkenyl or alkinyl having in each case up to 8 carbon atoms;
tetrahydropyranyl;
represents cycloalkyl having 3 to 6 carbon atoms which is in each case optionally mono- or polysubstituted by identical or different substituents from the group consisting of halogen, cyano, carboxyl, phenyl (which is optionally substituted by halogen, cyano, C₁-C₄-alkyl, C₁-C₄-halogenoalkyl, C₁-C₄-alkoxy or C₁-C₄-halogenoalkoxy), C₁-C₄-alkyl and C₁-C₄-alkoxy-carbonyl;
represents phenyl, naphthyl, tetrahydrobenzofuranyl, each of which is optionally mono- or polysubstituted by identical or different substituents, or represents heterocyclyl having 3 to 7 ring members, at least one of which represents oxygen, sulphur or nitrogen and one or two more of which optionally represent nitrogen, where the possible substituents are preferably selected from the list below:
halogen, cyano, nitro, amino, hydroxyl, formyl, carboxyl, carbamoyl, thiocarbamoyl;
in each case straight-chain or branched alkyl, alkoxy, alkylthio, alkylsulphinyl or alkylsulphonyl having in each case 1 to 6 carbon atoms;
in each case straight-chain or branched alkenyl or alkenyloxy having in each case 2 to 6 carbon atoms;
in each case straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms;
t-butyl-substituted tetrazole;
in each case straight-chain or branched halogenoalkenyl or halogenoalkenyloxy having in each case 2 to 6 carbon atoms and 1 to 11 identical or different halogen atoms;
in each case straight-chain or branched alkylamino, dialkylamino, alkylcarbonyl, alkylcarbonyloxy, alkoxycarbonyl or alkylsulphonyloxy having in each case 1 to 6 carbon atoms in the individual alkyl moieties;
phenyl;
in each case doubly attached alkylene or dioxyalkylene having in each case 1 to 6 carbon atoms and being in each case optionally mono- or polysubstituted by identical or different substituents from the group consisting of halogen, straight-chain or branched alkyl having 1 to 4 carbon atoms and straight-chain or branched halogenoalkyl having 1 to 4 carbon atoms and 1 to 9 identical or different halogen atoms;
cycloalkyl having 3 to 6 carbon atoms;
heterocyclyl or heterocyclyl-methyl having in each case 3 to 7 ring members, 1 to 3 of which are in each case identical or different heteroatoms - in particular nitrogen, oxygen and/or sulphur-
or a grouping 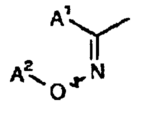 in which
A¹ represents alkyl having 1 to 4 carbon atoms or cycloalkyl having 1 to 6 carbon atoms and
A2 represents optionally cyano-, alkoxy-, alkylthio-, alkylamino-, dialkylamino- or phenyl-substituted alkyl having 1 to 4 carbon atoms, alkenyl or alkinyl having in each case 2 to 4 carbon atoms.

3. Compounds of the formula (I)
in which
A represents hydrogen, methyl, ethoxycarbonyl, ethyl or cyclopropyl, or has the same meaning as R,
R represents methylthio, ethylthio, n- or i-propylthio, n-, i-, s- or t-butylthio, difluoromethylthio, trifluoromethylthio, difluorochloromethylthio, dichlorofluoromethylthio, trichloromethylthio, 2,2,2-trifluoroethylthio, 1,1-difluoroethylthio, methoxycarbonylthio, ethoxycarbonylthio, represents phenylthio which is optionally mono- to trisubstituted by fluorine, chlorine, bromine, cyano, methyl, ethyl, n- or i-propyl, n-, i-, s- or t-butyl, methoxy, ethoxy, n- or i-propoxy, trifluoromethyl, trifluoroethyl, difluoromethoxy, trifluoromethoxy, difluorochloromethoxy, trifluoroethoxy, methoxycarbonyl or ethoxycarbonyl, represents -S-Y, -S-N(R¹R²),
in which
R¹ represents methyl, ethyl, n- or i-propyl, n-, i-, s- or t-butyl,
R² represents methyl, ethyl, n- or i-propyl, n-, i-, s- or t-butyl, methoxycarbonyl or ethoxycarbonyl, or together with R¹ and the nitrogen atom to which they are attached represents optionally methyl- or ethyl-substituted pyrrolidine, piperidine or morpholine,
Y represents the same grouping which is already attached to the sulphur atom,
or, if A represents methyl,
R also represents acetyl, propionyl, methoxycarbonyl, ethoxycarbonyl, i-butoxycarbonyl, methoxycarbonylcarbonyl, 2-bromoethoxycarbonyl, methylaminocarbonyl, dimethylaminocarbonyl, methylsulphonyl, ethylsulphonyl, phenylsulphonyl or tolylsulphonyl,
G represents oxygen or represents in each case optionally fluorine-, chlorine- or bromine-substituted dimethylene (ethane-1,2-diyl), ethene-1,2-diyl or one of the groupings below, in which in each case the left side is attached to Z:
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁸)=N-O-, -C(R⁸)=N-O-CH₂-, -N(R⁹)-, -CQ-N(R⁹)-, -N(R⁹)-CQ-, -Q-CQ-N(R⁹)-, -N=C(R⁸)-Q-CH₂-, -CH(R⁸)-O-N=CH-, -C(R⁸)=N-N=CH-, -N(R⁹)-CQ-Q-, -CQ-N(R⁹)-CQ-Q-, -N(R⁹)-CQ-Q-CH₂-, -Q-C(R⁸)=N-O-CH₂-, -N(R⁹)-C(R⁸)=N-O-CH₂-, -O-CH₂-C(R⁸)=N-O-CH₂-, -N=N-C(R⁸)=N-O-CH₂-, -C(=N-O-R¹⁰)-C(R⁸)=N-O-CH₂-, -C(=N-O-R¹⁰)-C(R⁸)-O-N=CH-, -C(=N-O-R¹⁰)-C(R⁸)-N-N=CH-, -C(=N-O-R¹⁰)-C(R⁸)-O-N=C(CH₃)-, -T-Ar¹- or -T-Ar¹-Q-, where
n represents the number 0, 1 or 2,
Q represents oxygen or sulphur,
R⁸ represents hydrogen, cyano, methyl, ethyl or cyclopropyl and
R⁹ represents hydrogen, methyl, ethyl or cyclopropyl.
R¹⁰ represents hydrogen, methyl, ethyl, n- or i-propyl, n-, i-, s- or t-butyl or represents optionally fluorine-, chlorine-, bromine-, cyano-, methyl-, ethyl-, n- or i-propyl, n-, i-, s- or t-butyl-, methoxy-, ethoxy-, n- or i-propoxy-, trifluoromethyl-, trifluoroethyl-, difluoromethoxy-, trifluoromethoxy-, difluorochloromethoxy- or trifluoroethoxy-substituted benzyl,
T represents a single bond, represents oxygen, sulphur, -CH₂-O-, -CH₂-S- or represents optionally substituted alkanediyl,
Ar¹ represents phenylene or pyridinediyl, each of which is optionally mono- to trisubstituted by identical or different substituents, represents in each case optionally monosubstituted pyrimidinediyl, pyridazinediyl, pyrazinediyl, 1,2,3-triazinediyl, 1,2,4-triazinediyl or 1,3,5-triazinediyl or represents 1,2,4-thiadiazolediyl, 1,3,4-thiadiazolediyl, 1,2,4-oxadiazolediyl, 1,3,4-oxadiazolediyl, where the possible substituents are preferably selected from the list below:
fluorine, chlorine, bromine, cyano, methyl, ethyl, n- or i-propyl, cyclopropyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl and
L¹, L², L³ and L⁴ are identical or different and independently of one another each represent hydrogen, fluorine, chlorine, bromine, methyl or ethyl,
W represents hydrogen, fluorine, cyano or thiocyanato and
Z represents methyl, ethyl, n- or i-propyl, n-, i-, s- or t-butyl, allyl, 2-buten-1-yl, 2-(1-methyl)-propyl, 2-methyl-2-buten-1-yI, propargyl, tetrahydropyranyl, t-butyl substituted tetrazole or represents phenyl, 1,2,4-thiadiazolyl, 1,3,4-thiadiazolyl, 1,2,4-oxadiazolyl, 1,3,4-oxadiazolyl, pyridinyl, pyrimidyl, tetrahydrobenzofuranyl, pyridazinyl, pyrazinyl, 1,2,3-triazinyl, 1,2,4-triazinyl or 1,3,5-triazinyl, each of which is optionally mono- to trisubstituted by identical or different substituents, where the possible substituents are preferably selected from the list below:
amino, fluorine, chlorine, bromine, iodine, cyano, nitro, methyl, ethyl, formyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl, methoxycarbonyl, ethoxycarbonyl, phenyl;
in each case doubly attached methylenedioxy, ethylenedioxy, each of which is optionally mono- to tetrasubstituted by identical or different substituents from the group consisting of fluorine, chlorine, methyl, trifluoromethyl and ethyl,
or a grouping 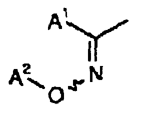 in which
A¹ represents methyl, ethyl, n- or i-propyl, n-, i-, s- or t-butyl, cyclopropyl or cyclobutyl and
A² represents methyl, ethyl, n- or i-propyl, n-, i-, s- or t-butyl, allyl, propargyl, but-2-en-1-yl, 2-methyl-prop-1-en-3-yl, cyanomethyl, methoxymethyl, ethoxymethyl, methoxyethyl, ethoxyethyl, methylthiomethyl, ethylthiomethyl, methylthioethyl, ethylthioethyl, dimethylaminomethyl, dimethylaminoethyl, methylaminomethyl, methylaminoethyl or benzyl.

4. Compounds of the formula (I) according to Claim 1
in which
A represents hydrogen, ethoxycarbonyl, methyl, ethyl or cyclopropyl, or has the same meaning as R,
R represents methylthio, ethylthio, n- or i-propylthio, n-, i-, s- or t-butylthio, difluoromethylthio, trifluoromethylthio, difluorochloromethylthio, dichlorofluoromethylthio, trichloromethylthio, 2,2,2-trifluoroethylthio, 1,1-difluoroethylthio, methoxycarbonylthio, ethoxycarbonylthio, represents phenylthio which is optionally mono- to trisubstituted by fluorine, chlorine, bromine, cyano, methyl, ethyl, n- or i-propyl, n-, i-, s- or t-butyl, methoxy, ethoxy, n- or i-propoxy, trifluoromethyl, trifluoroethyl, difluoromethoxy, trifluoromethoxy, difluorochloromethoxy, trifluoroethoxy, methoxycarbonyl or ethoxycarbonyl, represents -S-Y, -S-N(R¹R²),
in which
R¹ represents methyl, ethyl, n- or i-propyl, n-, i-, s- or t-butyl,
R² represents methyl, ethyl, n- or i-propyl, n-, i-, s- or t-butyl, methoxycarbonyl or ethoxycarbonyl, or together with R¹ and the nitrogen atom to which they are attached represents optionally methyl- or ethyl-substituted pyrrolidine, piperidine or morpholine,
Y represents the same grouping which is already attached to the sulphur atom,
or, if A represents methyl,
R also represents acetyl, propionyl, methoxycarbonyl, ethoxycarbonyl, i-butoxycarbonyl, methoxycarbonylcarbonyl, 2-bromoethoxycarbonyl, methylaminocarbonyl, dimethylaminocarbonyl, methylsulphonyl, ethylsulphonyl, phenylsulphonyl or tolylsulphonyl,
G represents oxygen or, in particular, -O-CH₂-,
L¹, L², L³ and L⁴ each represent hydrogen.
W represents hydrogen, fluorine, cyano or thiocyanato and
Z represents methyl, ethyl, n- or i-propyl, n-, i-, s- or t-butyl, allyl, 2-buten-1-yl, 2-methyl-2-buten-1-yl, propargyl, tetrahydropyranyl or represents phenyl which is in each case optionally mono- to trisubstituted by identical or different substituents, where the possible substituents are preferably selected from the list below:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl, methoxycarbonyl, ethoxycarbonyl, phenyl,
in each case doubly attached methylenedioxy or ethylenedioxy, each of which is optionally mono- to tetrasubstituted by identical or different substituents from the group consisting of fluorine, chlorine, methyl, trifluoromethyl and ethyl,
or a grouping 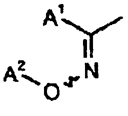 in which
A¹ represents methyl, ethyl, n- or i-propyl, n-, i-, s- or t-butyl, cyclopropyl or cyclobutyl and
A² represents methyl, ethyl, n- or i-propyl, n-, i-, s- or t-butyl, allyl, propargyl, but-2-en-1-yl, 2-methyl-prop-1-en-3-yl, cyanomethyl, methoxymethyl, ethoxymethyl, methoxyethyl, ethoxyethyl, methylthiomethyl, ethylthiomethyl, methylthioethyl, ethylthioethyl, dimethylaminomethyl, dimethylaminoethyl, methylaminomethyl, methylaminoethyl or benzyl.

5. Compounds of the formula (I) according to Claim 1
in which
A represents hydrogen, ethyl or methyl, or has the same meaning as R,
R represents methylthio, ethylthio, n- or i-propylthio, n-, i-, s- or t-butylthio, difluoromethylthio, trifluoromethylthio, difluorochloromethylthio, dichlorofluoromethylthio, trichloromethylthio, 2,2,2-trifluoroethylthio, methoxycarbonylthio, ethoxycarbonylthio, represents phenylthio which is optionally mono- to trisubstituted by fluorine, chlorine, bromine, cyano, methyl, ethyl, n- or i-propyl, n-, i-, s- or t-butyl, methoxy, ethoxy, n- or i-propoxy, trifluoromethyl, trifluoroethyl, difluoromethoxy, trifluoromethoxy, difluorochloromethoxy, trifluoroethoxy, methoxycarbonyl or ethoxycarbonyl, represents -S-Y, -S-N(R¹R²),
in which
R¹ represents methyl, ethyl, n- or i-propyl, n-, i-, s- or t-butyl,
R² represents methyl, ethyl, n- or i-propyl, n-, i-, s- or t-butyl, methoxycarbonyl or ethoxycarbonyl, or together with R¹ and the nitrogen atom to which they are attached represents optionally methyl- or ethyl-substituted pyrrolidine, piperidine or morpholine,
Y represents the same grouping which is already attached to the sulphur atom,
or, if A represents methyl,
R also represents acetyl, propionyl, methoxycarbonyl, ethoxycarbonyl, methoxycarbonylcarbonyl, 2-bromoethoxycarbonyl, methylaminocarbonyl, dimethylaminocarbonyl, methylsulphonyl, ethylsulphonyl, phenylsulphonyl or tolylsulphonyl,
G represents -C(R⁸)=N-O-CH₂-,
R⁸ represents cyclopropyl or, in particular, methyl,
L¹, L², L³ and L⁴ each represent hydrogen,
W represents hydrogen, fluorine, cyano or thiocyanato and
Z represents methyl, ethyl, n- or i-propyl, n-, i-, s- or t-butyl, allyl, 2-buten-1-yl, 2-methyl-2-buten-1-yl, propargyl or represents pyridyl, pyrimidyl or, in particular, phenyl, each of which is optionally mono- to trisubstituted by identical or different substituents where the possible substituents are preferably selected from the list below:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl, methoxycarbonyl, ethoxycarbonyl, methoximinomethyl, ethoximinomethyl, methoximinoethyl, ethoximinoethyl, in each case doubly attached methylenedioxy or ethylenedioxy, each of which is optionally mono- to tetrasubstituted by identical or different substituents from the group consisting of fluorine, chlorine, methyl, trifluoromethyl and ethyl.

6. Compounds of the formula (I) according to Claim 1
in which
A represents hydrogen, methyl or ethyl, or has the same meaning as R,
R represents methylthio, ethylthio, n- or i-propylthio, n-, i-, s- or t-butylthio, difluoromethylthio, trifluoromethylthio, difluorochloromethylthio, dichlorofluoromethylthio, trichloromethylthio, 2,2,2-trifluoroethylthio, methoxycarbonylthio, ethoxycarbonylthio, represents phenylthio which is optionally mono- to trisubstituted by fluorine, chlorine, bromine, cyano, methyl, ethyl, n- or i-propyl, n-, i-, s- or t-butyl, methoxy, ethoxy, n- or i-propoxy, trifluoromethyl, trifluoroethyl, difluoromethoxy, trifluoromethoxy, difluorochloromethoxy, trifluoroethoxy, methoxycarbonyl or ethoxycarbonyl, represents -S-Y, -S-N(R¹R²),
in which
R¹ represents methyl, ethyl, n- or i-propyl, n-, i-, s- or t-butyl,
R² represents methyl, ethyl, n- or i-propyl, n-, i-, s- or t-butyl, methoxycarbonyl or ethoxycarbonyl, or together with R¹ and the nitrogen atom to which they are attached represents optionally methyl- or ethyl-substituted pyrrolidine, piperidine or morpholine,
Y represents the same grouping which is already attached to the sulphur atom,
or, if A represents methyl,
R also presents acetyl, propionyl, methoxycarbonyl, ethoxycarbonyl, methoxycarbonylcarbonyl, 2-bromoethoxycarbonyl, methylaminocarbonyl, dimethylaminocarbonyl, methylsulphonyl, ethylsulphonyl, phenylsulphonyl or tolylsulphonyl,
G represents -T-Ar¹-O-,
Ar¹ represents 1,2,4-thiadiazoldiyl, 1,3,4-thiadiazoldiyl, 1,2,4-oxadiazoldiyl, 1,3,4-oxadiazoldiyl or represents pyridinediyl, pyrimidinediyl or 1,3,5-triazinediyl, each of which is optionally mono- or disubstituted by identical or different substituents from the group consisting of fluorine, chlorine, cyano, methyl, cyclopropyl, methoxy, methylthio, trifluoromethyl, difluoromethoxy, trifluoromethoxy, difluorochloromethoxy,
T represents a single bond, represents oxygen, sulphur, -CH₂-O-, CH₂-S-, methylene, ethylene or propylene and
L¹, L², L³ and L⁴ each represent hydrogen,
W represents hydrogen, fluorine, cyano or thiocyanato and
Z represents methyl, ethyl, n- or i-propyl, n-, i-, s- or t-butyl, allyl, 2-buten-1-yl, 2-(1-methyl)-propyl, 2-methyl-2-buten-1-yl, propargyl or represents pyridyl, pyrimidyl, thienyl, tetrahydrobenzofuranyl or, in particular, phenyl, each of which is optionally mono- to trisubstituted by identical or different substituents from the group consisting of amino, fluorine, chlorine, bromine, iodine, cyano, nitro, methyl, ethyl, formyl, n- or i-propyl, n-, i-, s- or t-butyl, trifluoromethyl, methoxy, t-butyl-substituted tetrazole, ethoxy, n- or i-propoxy, difluoromethoxy, difluorochloromethoxy, trifluoroethoxy, trifluoromethoxy, methoximinoethyl, ethoximinomethyl, methoximinoethyl, ethoximinoethyl and in each case doubly attached methylenedioxy or ethyleoedioxy, each of which is optionally mono- to tetrasubstituted by identical or different substituents from the group consisting of fluorine, chlorine, methyl, trifluoromethyl and ethyl.

7. Compounds of the formula (I) according to Claim 1
in which
A represents hydrogen or methyl, or has the same meaning as R,
R represents methylthio, ethylthio, n- or i-propylthio, n-, i-, s- or t-butylthio, difluoromethylthio, trifluoromethylthio, difluorochloromethylthio, dichlorofluoromethylthio, trichloromethylthio, 2,2,2-trifluoroethylthio, methoxycarbonylthio, ethoxycarbonylthio, represents phenylthio which is optionally mono- to trisubstituted by fluorine, chlorine, bromine, cyano, methyl, ethyl, n- or i-propyl, n-, i-, s- or t-butyl, methoxy, ethoxy, n- or i-propoxy, trifluoromethyl, trifluoroethyl, difluoromethoxy, trifluoromethoxy, difluorochloromethoxy, trifluoroethoxy, methoxycarbonyl or ethoxycarbonyl, represents -S-Y, -S-N(R¹R²),
in which
R¹ represents methyl, ethyl, n- or i-propyl, n-, i-, s- or t-butyl,
R² represents methyl, ethyl, n- or i-propyl, n-, i-, s- or t-butyl, methoxycarbonyl or ethoxycarbonyl, or together with R¹ and the nitrogen atom to which they are attached represents optionally methyl- or ethyl-substituted pyrrolidine, piperidine or morpholine,
Y represents the same grouping which is already attached to the sulphur atom,
or, if A represents methyl,
R also represents acetyl, propionyl, methoxycarbonyl, ethoxycarbonyl, methoxycarbonylcarbonyl, 2-bromoethoxycarbonyl, methylaminocarbonyl, dimethylaminocarbonyl, methylsulphonyl, ethylsulphonyl, phenylsulphonyl or tolylsulphonyl,
G represents -C(=N-O-R¹⁰)-C(R⁸)=N-O-CH₂- in which
R⁸ represents methyl or cyclopropyl and
R¹⁰ represents hydrogen, methyl, ethyl, n- or i-propyl, n-, i-, s- or t-butyl or represents optionally fluorine-, chlorine-, bromine-, cyano-, methyl-, ethyl-, n- or i-propyl-, n-, i-, s- or t-butyl-, methoxy-, ethoxy-, n- or i-propoxy-, trifluoromethyl-, trifluoroethyl-, difluoromethoxy-, trifluoromethoxy-, difluorochloromethoxy- or trifluoroethoxy-substituted benzyl,
L¹, L², L³ and L⁴ each represent hydrogen,
W represents hydrogen, fluorine, cyano or thiocyanato and
Z represents methyl, ethyl, n- or i-propyl, n-, i-, s- or t-butyl, allyl, 2-buten-1-yl, 2-methyl-2-buten-1-yl or represents pyridyl, pyrimidyl or, in particular, phenyl, each of which is optionally mono- to trisubstituted by identical or different substituents, where the possible substituents are preferably selected from the list below:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl, methoxycarbonyl, ethoxycarbonyl, methoximinomethyl, ethoximinomethyl, methoximinoethyl, ethoximinoethyl, in each case doubly attached methylenedioxy, ethylenedioxy, each of which is optionally mono- to tetrasubstituted by identical or different substituents from the group consisting of fluorine, chlorine, methyl, trifluoromethyl and ethyl, or t-butyl-substituted tetrazole.

8. Compounds of the formula (I) according to Claim 1 in which
G represents oxygen or one of the groupings below in which in each case the left side is attached to Z: -Q-CH₂-, -C(R⁸)=N-O-CH₂-, -N=C(R⁸)-Q-CH₂-, -CH(R⁸)-O-N=CH-, -C(R⁸)=N-N=CH-, -C(=N-O-R¹⁰)-C(R⁸)=N-O-CH₂-, -C(=N-O-R¹⁰)-C(R⁸)-O-N=CH-, -C(=N-O-R¹⁰)-C(R⁸)-N-N=CH- or -T-Ar¹-Q- where R⁸, R⁹, R¹⁰, T, Ar¹ and Q are as defined in Claim 1.

9. Compounds of the formula (I) according to Claim 1 in which
A represents methyl.

10. Compounds of the formula (I) according to Claim 1 in which
G represents oxygen, -O-CH₂-, -C(CH₂)=N-O-CH₂- or optionally fluorine-substituted pyrimidinyloxy,

11. Compounds of the formula (I) according to Claim 1 in which
Z represents unsubstituted or mono- or disubstituted phenyl.

12. Compositions comprising extenders and/or carriers and also, if appropriate, surfactants, **characterized in that** they contain at least one compound as defined in Claim 1.

13. Method for controlling pests, **characterized in that** compounds as defined in Claim 1 or compositions as defined in Claim 12 are allowed to act on pests and/or their habitat.

14. Use of compounds as defined in Claims 1 to 11 or compositions as defined in Claim 12 for controlling pests.

15. Process for preparing compositions, **characterized in that** compounds as defined in Claims 1 to 11 are mixed with extenders and/or surfactants.

16. Process for preparing compounds of the formula (I) as defined in Claim 1, **characterized in that** amides of the formula (II) 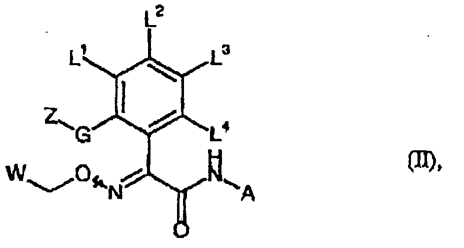 in which
A, G, L¹, L², L³, L⁴ and Z are each as defined in Claim 1,
are reacted with an electrophilic reagent of the formula (III)
R-X (III)
in which
R is as defined in Claim 1 and
X represents a leaving group,
if appropriate in the presence of a diluent and if appropriate in the presence of a base.

## Revendications

1. Composés de formule (I) 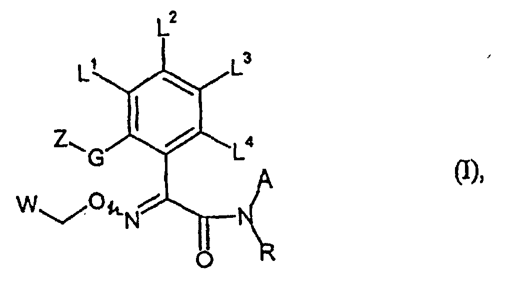 dans laquelle
A représente l'hydrogène, un reste alkoxycarbonyle, méthyle, éthyle ou cyclopropyle, ou a la même définition que R,
R est un reste alkylthio, halogénalkylthio, arylthio éventuellement substitué, alkoxycarbonylthio, -S-Y ou -S-N(R¹R²), où
R¹ est un reste alkyle,
R² est un reste alkyle, alkoxycarbonyle ou forme conjointement avec R¹ ainsi qu'avec l'atome d'azote auquel ils sont liés, un noyau hétérocyclique éventuellement substitué,
Y représente un groupement qui est le même que celui qui est déjà lié à l'atome S,
ou bien, lorsque A est le reste méthyle,
R représente aussi un reste alkylcarbonyle, alkoxycarbonyle, halogénalkoxycarbonyle, alkoxycarbonylcarbonyle, alkylaminocarbonyle, dialkyaminocarbonyle, alkylsulfonyle ou un reste arylsulfonyle éventuellement substitué,
G est une liaison simple, l'oxygène, le soufre ou un reste alcanediyle, alcènediyle, alcynediyle dont chacun porte éventuellement un substituant halogéno, hydroxy, alkyle, halogénalkyle ou cycloalkyle, ou l'un des groupements suivants, dont chacun est lié par le côté gauche à Z :
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁸)=N-O-, -C(R⁸)=N-O-CH₂-, -N(R⁹)-, -CQ-N(R⁹)-, -N(R⁹)-CQ-, -Q-CQ-N(R⁹)-, -N=C(R⁸)-Q-CH₂-, -CH(R⁸)-O-N=CH-, -C(R⁸)=N-N=CH-, -N(R⁹)-CQ-Q-, -CQ-N(R⁹)-CQ-Q-, -N(R⁹)-CQ-Q-CH₂-, -Q-C(R⁸)=N-O-CH₂-, -N(R⁹)-C(R⁸)=N-O-CH₂-, -O-CH₂-C(R⁸)=N-O-CH₂-, -N=N-C(R⁸)=N-O-CH₂-, -C(=N-O-R¹⁰)-C(R⁸)=N-O-CH₂-, -C(=N-O-R¹⁰)-C(R⁸)-O-N=CH-, -C(=N-O-R¹⁰)-C(R⁸)=N-N=CH-, -C(=N-O-R¹⁰)-C(R⁸)-O-N=C(CH₃)-, -T-Ar¹- ou -T-Ar¹-Q-
où
Ar¹ est un reste éventuellement substitué arylène, hétéroarylène, cycloalkylène ou hétérocycloalkylène (c'est-à-dire un noyau aliphatique à deux liaisons, dans lequel un ou plusieurs atomes de carbone sont remplacés par des hétéroatomes, c'est-à-dire des atomes différents de l'atome de carbone),
n représente les nombres 0, 1 ou 2,
Q est l'oxygène ou le soufre,
R⁸ représente l'hydrogène, un groupe cyano ou un reste alkyle, alkoxy, aklylthio, alkylamino, dialkylamino ou cycloalkyle dont chacun est éventuellement substitué,
R⁹ représente l'hydrogène, un groupe hydroxy, cyano ou un reste aryle, alkoxy ou cycloalkyle dont chacun est éventuellement substitué,
R¹⁰ représente l'hydrogène, un reste aryle ou un reste benzyle éventuellement substitué et
T représente une liaison simple, l'oxygène, le soufre, un reste -CH₂-O-, -CH₂-S- ou un reste alcanediyle éventuellement substitué,
L¹, L², L³ et L⁴ sont identiques ou différents et représentent chacun, indépendamment l'un de l'autre, l'hydrogène, un halogène ou un reste alkyle,
W est l'oxygène, le fluor, un groupe cyano ou thiocyanato et
Z représente un reste alkyle, alcényle, alcynyle, cycloalkyle, aryle ou hétérocyclyle dont chacun est éventuellement substitué.

2. Composés suivant la revendication 1,
dans lesquels
A représente l'hydrogène, un reste méthyle, éthoxycarbonyle, éthyle ou cyclopropyle, ou a la même définition que R,
R est un reste alkylthio, halogénalkylthio, alkoxycarbonylthio, ayant chacun 1 à 4 atomes de carbone dans les chaînes alkyle, un reste phénylthio portant éventuellement un substituant halogéno, cyano, alkyle, alkoxy ou alkoxycarbonyle ayant chacun 1 à 4 atomes de carbone, halogénalkyle ou halogénalkoxy ayant chacun 1 à 4 atomes de carbone et 1 à 8 atomes d'halogènes identiques ou différents, un reste -S-Y, -S-N(R¹R²), où
R¹ est un reste alkyle ayant 1 à 4 atomes de carbone,
R² est un reste alkyle ayant 1 à 4 atomes de carbone, alkoxycarbonyle ayant 1 à 4 atomes de carbone dans la partie alkyle, ou forme conjointement avec R¹ ainsi qu'avec l'atome d'azote auquel ils sont liés, un noyau hétérocyclique pentagonal ou hexagonal portant éventuellement un substituant méthyle ou éthyle,
Y représente un groupement qui est le même que celui qui est déjà lié à l'atome de soufre,
ou bien, lorsque A est un reste méthyle,
R représente aussi un reste alkylcarbonyle, alkoxycarbonyle, alkoxycarbonylcarbonyle, halogénalkoxycarbonyle, alkylaminocarbonyle, dialkylaminocarbonyle, alkylsulfonyle ayant chacun 1 à 4 atomes de carbone dans les chaînes alkyle ou un reste phénylsulfonyle portant éventuellement un substituant alkyle ayant 1 à 4 atomes de carbone,
G représente une liaison simple, l'oxygène, le soufre ou un reste alcanediyle, alcènediyle, alcynediyle ayant chacun jusqu'à 4 atomes de carbone et portant chacun, le cas échéant, un substituant halogéno, hydroxy, alkyle en C₁ à C₄, halogénalkyle en C₁ à C₄ ou cycloalkyle en C₃ à C₆ ou l'un des groupements suivants, liés chacun par le côté gauche à Z :
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁸)=N-O-, -C(R⁸)=N-O-CH₂-, -N(R⁹)-, -CQ-N(R⁹)-, -N(R⁹)-CQ-, -Q-CQ-N(R⁹)-, -N=C(R⁸)-Q-CH₂-, -CH(R⁸)-O-N=CH-, -C(R⁸)=N-N=CH-, -N(R⁹)-CQ-Q-, -CQ-N(R⁹)-CQ-Q-, -N(R⁹)-CQ-Q-CH₂-, -Q-C(R⁸)=N-O-CH₂-, -N(R⁹)-C(R⁸)=N-O-CH₂-, -O-CH₂-C(R⁸)=N-O-CH₂-, -N=N-C(R⁸)=N-O-CH₂-, -C(=N-O-R¹⁰)-C(R⁸)=N-O-CH₂-, -C(=N-O-R¹⁰)-C(R⁸)-O-N=CH-, -C(=N-O-R¹⁰)-C(R⁸)-N-N=CH-, -C(=N-O-R¹⁰)-C(R⁸)-O-N=C(CH₃)-, -T-Ar¹- ou -T-Ar¹-Q-
où
n représente les nombres 0, 1 ou 2,
Q est l'oxygène ou le soufre,
R⁸ représente l'hydrogène, un groupe cyano, un reste alkyle, alkoxy, alkylthio, alkylamino ou dialkylamino ayant chacun 1 à 6 atomes de carbone dans les groupes alkyle et portant chacun, le cas échéant, un substituant halogéno, cyano ou alkoxy en C₁ à C₄, ou un reste cycloalkyle ayant 3 à 6 atomes de carbone portant éventuellement dans chaque cas un substituant halogéno, cyano, carboxy, alkyle en C₁ à C₄ ou (alkoxy en C₁ à C₄)-carbonyle, et
R⁹ représente l'hydrogène, un groupe hydroxy, cyano, ou un reste alkyle ayant 1 à 6 atomes de carbone portant éventuellement un substituant halogéno, cyano ou alkoxy en C₁ à C₄, ou un reste cycloalkyle ayant 3 à 6 atomes de carbone portant éventuellement un substituant halogéno, cyano, carboxy, alkyle en C₁ à C₄ ou (alkoxy en C₁ à C₄)-carbonyle,
R¹⁰ représente l'hydrogène, un reste alkyle en C₁ à C₄ ou un reste benzyle portant éventuellement un substituant halogéno, alkyle ou alkoxy ayant chacun 1 à 6 atomes de carbone, halogénalkyle ou halogénalkoxy ayant chacun 1 à 6 atomes de carbone et 1 à 13 atomes d'halogènes identiques ou différents,
T représente un liaison simple, l'oxygène, le soufre, un groupe -CH₂-O-, -CH₂-S- ou un reste alcanediyle ayant 1 à 3 atomes de carbone,
Ar¹ représente un reste phénylène, naphtylène, cycloalkylène ou un reste hétéroarylène ou hétérocycloalkylène à noyau de 3 à 7 atomes dont l'un au moins un atome d'oxygène, de soufre ou d'azote et, le cas échéant, un ou deux autres représentent l'azote, chacun de ces restes portant éventuellement un ou plusieurs substituants identiques ou différents, les substituants possibles étant avantageusement choisis parmi ceux qui sont énumérés ci-après :
halogéno, cyano, nitro, amino, hydroxy, formyle, carboxy, carbamoyle, thiocarbamoyle ;
alkyle, alkoxy, alkylthio, alkylsulfinyle ou alkylsulfonyle chacun linéaire ou ramifié ayant chacun 1 à 6 atomes de carbone ;
alcényle ou alcényloxy chacun linéaire ou ramifié ayant chacun 2 à 6 atomes de carbone ;
halogénalkyle, halogénalkoxy, halogénalkylthio, halogénalkylsulfinyle ou halogénalkylsulfonyle chacun linéaire ou ramifié ayant chacun 1 à 6 atomes de carbone et 1 à 13 atomes d'halogènes identiques ou différents ;
halogénalcényle ou halogénalcényloxy chacun linéiare ou ramifié ayant chacun 2 à 6 atomes de carbone et 1 à 11 atomes d'halogènes identiques ou différents ;
alkylamino, dialkylamino, alkylcarbonyle, alkylcarbonyloxy, alkoxycarbonyle, alkylsulfonyloxy, hydroximino-alkyle ou alkoximino-alkyle chacun linéaire ou ramifié ayant chacun 1 à 6 atomes de carbone dans les parties alkyle individuelles, ainsi que :
cycloalkyle ayant 3 à 6 atomes de carbone et
L¹, L², L³ et L⁴ sont identiques ou différents et représentent chacun, indépendamment l'un de l'autre, l'hydrogène, le fluor, le chlore, le brome, un reste méthyle, éthyle, n-propyle, isopropyle, n-butyle, isobutyle, sec.-butyle ou tertio-butyle,
W représente l'hydrogène, le fluor, un groupe cyano ou thiocyanato et
Z est un reste alkyle ayant 1 à 8 atomes de carbone portant éventuellement un ou plusieurs substituants, identiques ou différents, halogéno, cyano, hydroxy, amino, alkoxy en C₁ à C₄, alkylthio en C₁ à C₄, alkylsulfinyle en C₁ à C₄ ou alkylsulfonyle en C₁ à C₄ (dont chacun peut éventuellement être substitué par un halogène) ;
un reste alcényle ou alcynyle ayant chacun jusqu'à 8 atomes de carbone et dont chacun est éventuellement substitué par un halogène ; tétrahydropyrannyle ;
cycloalkyle ayant 3 à 6 atomes de carbone portant éventuellement un ou plusieurs substituants, identiques ou différents, halogéno, cyano, carboxy, phényle (qui est éventuellement porteur d'un substituant halogéno, cyano, alkyle en C₁ à C₄, halogénalkyle en C₁ à C₄, alkoxy en C₁ à C₄ ou halogénalkoxy en C₁ à C₄), alkyle en C₁ à C₄ ou (alkoxy en C₁ à C₄) carbonyle ;
phényle, naphtyle, tétrahydrobenzofurannyle ou hétérocyclyle à noyau de 3 à 7 atomes dont l'un au moins est un atome d'oxygène, de soufre ou d'azote et, le cas échéant, un ou deux autres représentent l'azote, chacun de ces restes portant éventuellement un ou plusieurs substituants identiques ou différents, les substituants possibles étant avantageusement choisis parmi ceux qui sont énumérés ci-après :
halogéno, cyano, nitro, amino, hydroxy, formyle, carboxy, carbamoyle, thiocarbamoyle, formyle ;
alkyle, alkoxy, alkylthio, alkylsulfinyle ou alkylsulfonyle chacun linéaire ou ramifié ayant chacun 1 à 6 atomes de carbone ;
alcényle ou alcényloxy chacun linéaire ou ramifié ayant chacun 2 à 6 atomes de carbone ;
halogénalkyle, halogénalkoxy, halogénalkylthio, halogénalkylsulfinyle ou halogénalkylsulfonyle chacun linéaire ou ramifié ayant chacun 1 à 6 atomes de carbone et 1 à 13 atomes d'halogènes identiques ou différents ;
tétrazole à substituant tertio-butyle ;
halogénalcényle ou halogénalcényloxy chacun linéaire ou ramifié ayant chacun 2 à 6 atomes de carbone et 1 à 11 atomes d'halogènes identiques ou différents ;
alkylamino, dialkylamino, alkylcarbonyle, alkylcarbonyloxy, alkoxycarbonyle ou alkylsulfonyloxy chacun linéaire ou ramifié, ayant chacun 1 à 6 atomes de carbone dans les parties alkyle individuelles ; phényle ;
alkylène ou dioxyalkylène ayant chacun deux liaisons et chacun 1 à 6 atomes de carbone, portant chacun, le cas échéant, un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
cycloalkyle ayant 3 à 6 atomes de carbone ;
hétérocyclyle ou hétérocyclylméthyle ayant chacun un noyau de 3 à 7 atomes dont, chaque cas, 1 à 3 atomes sont des hétéroatomes identiques ou différents - en particulier azote, oxygène et/ou soufre - ou un groupement 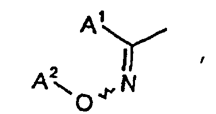 dans lequel
A¹ est un reste alkyle ayant 1 à 4 atomes de carbone ou cycloalkyle ayant 1 à 6 atomes de carbone et
A² est un reste alkyle ayant 1 à 4 atomes de carbone portant éventuellement un substituant cyano, alkoxy, alkylthio, alkylamino, dialkylamino ou phényle, un reste alcényle ou un reste alcynyle ayant chacun 2 à 4 atomes de carbone.

3. Composés de formule (I),
dans laquelle
A représente l'hydrogène, un reste méthyle, éthoxycarbonyle, éthyle ou cyclopropyle ou a la même définition que R,
R est un reste méthylthio, éthylthio, n-propylthio, isopropylthio, n-butylthio, isobutylthio, sec.-butylthio, tertio-butylthio, difluorométhylthio, trifluorométhylthio, difluorochlorométhylthio, dichlorofluorométhylthio, trichlorométhylthio, 2,2,2-trifluoréthylthio, 1,1-difluoréthylthio, méthoxycarbonylthio, éthoxycarbonylthio, un reste phénylthio portant éventuellement un à trois substituants, identiques ou différents, fluoro, chloro, bromo, cyano, méthyle, éthyle, n-propyle, isopropyle, n-butyle, isobutyle, sec.-butyle, tertio-butyle, méthoxy, éthoxy, n-propoxy, isopropoxy, trifluorométhyle, trifluoréthyle, difluorométhoxy, trifluorométhoxy, difluorochlorométhoxy, trifluoréthoxy, méthoxycarbonyle ou éthoxycarbonyle, un groupe -S-Y, -S-N(R¹R²), où
R¹ est un reste méthyle, éthyle, n-propyle, isopropyle, n-butyle, isobutyle, sec.-butyle ou tertio-butyle,
R² est un reste méthyle, éthyle, n-propyle, isopropyle, n-butyle, isobutyle, sec.-butyle, tertio-butyle, méthoxycarbonyle ou éthoxycarbonyle ou forme conjointement avec R¹ de même qu'avec l'atome d'azote auquel ils sont liés, un noyau pyrrolidine, pipéridine ou morpholine portant éventuellement un substituant méthyle ou éthyle,
Y représente le même groupement que celui qui est déjà lié à l'atome de soufre,
ou bien, lorsque A est un reste méthyle,
R est aussi un reste acétyle, propionyle, méthoxycarbonyle, éthoxycarbonyle, isobutoxycarbonyle, méthoxycarbonylcarbonyle, 2-brométhoxycarbonyle, méthylaminocarbonyle, diméthylaminocarbonyle, méthylsulfonyle, éthylsulfonyle, phénylsulfonyle ou tolylsulfonyle,
G représente l'oxygène ou un reste diméthylène (éthane-1,2-diyle), éthène-1,2-diyle dont chacun est éventuellement substitué par du fluor, du chlore ou du brome, ou bien l'un des groupements suivants dont chacun est lié par le côté gauche à Z :
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁸)=N-O-, -C(R⁸)=N-O-CH₂-, -N(R⁹)-, -CQ-N(R⁹)-, -N(R⁹)-CQ-, -Q-CQ-N(R⁹)-, -N=C(R⁸)-Q-CH₂-, -CH(R⁸)-O-N=CH-, -C(R⁸)=N-N=CH-, -N(R⁹)-CQ-Q-, -CQ-N(R⁹)-CQ-Q-, -N(R⁹)-CQ-Q-CH₂-, -Q-C(R⁸)=N-O-CH₂-, -N(R⁹)-C(R⁸)=N-O-CH₂-, -O-CH₂-C(R⁸)=N-O-CH₂-, -N=N-C(R⁸)=N-O-CH₂-, -C(=N-O-R¹⁰)-C(R⁸)=N-O-CH₂-, -C(=N-O-R¹⁰)-C(R⁸)-O-N=CH-, -C(=N-O-R¹⁰)-C(R⁸)-N-N=CH-, -C(=N-O-R¹⁰)-C(R⁸)-O-N=C(CH₃)-, -T-Ar¹- ou -T-Ar¹-Q-
où
n représente les nombres 0, 1 ou 2,
Q est l'oxygène ou le soufre,
R⁸ est l'hydrogène, un reste cyano, méthyle, éthyle ou cyclopropyle et
R⁹ est l'hydrogène, un reste méthyle, éthyle ou cyclopropyle,
R¹⁰ est l'hydrogène, un reste méthyle, éthyle, n-propyle, isopropyle, n-butyle, isobutyle, sec.-butyle, tertio-butyle ou un reste benzyle portant éventuellement un substituant fluoro, chloro, bromo, cyano, méthyle, éthyle, n-propyle, isopropyle, n-butyle, isobutyle, sec.-butyle, tertio-butyle, méthoxy, éthoxy, n-propoxy, isopropoxy, trifluorométhyle, trifluoréthyle, difluorométhoxy, trifluorométhoxy, difluorochlorométhoxy ou trifluoréthoxy,
T est une liaison simple, l'oxygène, le soufre, un groupe -CH₂-O-, -CH₂-S- ou un reste alcanediyle éventuellement substitué,
Ar¹ est un reste phénylène ou pyridinediyle portant chacun, le cas échéant, un à trois substituants identiques ou différents, un reste pyrimidinediyle, pyridazinediyle, pyrazinediyle, 1,2,3-triazinediyle, 1,2,4-triazinediyle ou 1,3,5-triazinediyle portant chacun, le cas échéant, un substituant, ou un reste 1,2,4-thiadiazolediyle, 1,3,4-thiadiazolediyle, 1,2,4-oxadiazolediyle, 1,3,4-oxadiazolediyle, les substituants possibles étant avantageusement choisis parmi ceux qui sont énumérés ci-après :
fluor, chlore, brome, cyano, méthyle, éthyle, n-propyle, isopropyle, cyclopropyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, trifluorométhylthio, difluorochlorométhyl thio, trifluorométhylsulfinyle ou trifluorométhylsulfonyle et
L¹, L², L³ et L⁴ sont identiques ou différents et représentent, indépendamment l'un de l'autre, l'hydrogène, le fluor, le chlore, le brome, un reste méthyle ou éthyle,
W est l'hydrogène, le fluor, un reste cyano ou thiocyanato et
Z est un reste méthyle, éthyle, n-propyle, isopropyle, n-butyle, isobutyle, sec.-butyle, tertio-butyle, allyle, 2-butène-1-yle, 2-(1-méthyl)propyle, 2-méthyl-2-butène-1-yle, propargyle, tétrahydropyrannyle, tétrazole à substituant tertio-butyle, ou un reste phényle, 1,2,4-thiadiazolyle, 1,3,4-thiadiazolyle, 1,2,3-oxadiazolyle, 1,3,4-oxadiazolyle, pyridinyle, pyrimidyle, tétrahydrobenzofurannyle, pyridazinyle, pyrazinyle, 1,2,3-triazinyle, 1,2,4-triazinyle ou 1,3,5-triazinyle portant chacun, le cas échéant, un à trois substituants identiques ou différents, les substituants possibles étant avantageusement choisis parmi ceux qui sont énumérés ci-après :
amino, fluor, chlore, brome, iode, cyano, nitro, méthyle, éthyle, formyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, trifluorométhylthio, difluorochlorométhylthio, trifluorométhylsulfinyle, trifluorométhylsulfonyle, méthoxycarbonyle, éthoxycarbonyle, phényle ;
méthylènedioxy, éthylènedioxy ayant chacun deux liaisons et portant chacun, le cas échéant, un à quatre substituants, identiques ou différents, fluoro, chloro, méthyle, trifluorométhyle ou éthyle,
ou un groupement 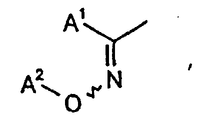 dans lequel
A¹ est un reste méthyle, éthyle, n-propyle, isopropyle, n-butyle, isobutyle, sec.-butyle, tertio-butyle, cyclopropyle ou cyclobutyle et
A² est un reste méthyle, éthyle, n-propyle, isopropyle, n-butyle, isobutyle, sec.-butyle, tertio-butyle, allyle, propargyle, but-2-ène-1-yle, 2-méthylprop-1-ène-3-yle, cyanométhyle, méthoxyméthyle, éthoxyméthyle, méthoxyéthyle, éthoxyéthyle, méthylthiométhyle, éthylthiométhyle, méthylthio-éthyle, éthylthio-éthyle, diméthylaminométhyle, diméthylamino-éthyle, méthylaminométhyle, méthylamino-éthyle ou benzyle.

4. Composés de formule (I) suivant la revendication 1, formule dans laquelle
A représente l'hydrogène, un reste éthoxycarbonyle, méthyle, éthyle ou cyclopropyle ou a la même définition que R,
R est un reste méthylthio, éthylthio, n-propylthio, isopropylthio, n-butylthio, isobutylthio, sec.-butylthio, tertio-butylthio, difluorométhylthio, trifluorométhylthio, difluorochlorométhylthio, dichlorofluorométhylthio, trichlorométhylthio, 2,2,2-trifluoréthylthio, 1,1-difluoréthylthio, méthoxycarbonylthio, éthoxycarbonylthio, un reste phénylthio portant éventuellement un à trois substituants fluoro, chloro, bromo, cyano, méthyle, éthyle, n-propyle, isopropyle, n-butyle, isobutyle, sec.-butyle, tertio-butyle, méthoxy, éthoxy, n-propoxy, isopropoxy, trifluorométhyle, trifluoréthyle, difluorométhoxy, trifluorométhoxy, difluorochlorométhoxy, trifluoréthoxy, méthoxycarbonyle ou éthoxycarbonyle, un groupe -S-Y, -S-N(R¹R²), où
R¹ est un reste méthyle, éthyle, n-propyle, isopropyle, n-butyle, isobutyle, sec.-butyle ou tertio-butyle,
R² est un reste méthyle, éthyle, n-propyle, isopropyle, n-butyle, isobutyle, sec.-butyle, tertio-butyle, méthoxycarbonyle, éthoxycarbonyle, ou forme conjointement avec R¹ de même qu'avec l'atome d'azote auquel ils sont liés un noyau pyrrolidinyle, pipéridine ou morpholine éventuellement substitué par un radical méthyle ou éthyle,
Y est le même groupement que celui qui est déjà lié à l'atome de soufre,
ou bien, lorsque A est un reste méthyle,
R est aussi un reste acétyle, propionyle, méthoxycarbonyle, éthoxycarbonyle, isobutoxycarbonyle, méthoxycarbonylcarbonyle, 2-brométhoxycarbonyle, méthylaminocarbonyle, diméthylaminocarbonyle, méthylsulfonyle, éthylsulfonyle, phénylsulfonyle ou tolylsulfonyle,
G représente l'oxygène ou en particulier -O-CH₂-,
L¹, L², L³ et L⁴ représentent l'hydrogène,
W est l'hydrogène, le fluor, un groupe cyano ou thocyanato et
Z est un reste méthyle, éthyle, n-propyle, isopropyle, n-butyle, isobutyle, sec.-butyle, tertio-butyle, allyle, 2-butène-1-yle, 2-méthyl-2-butène-1-yle, propargyle, tétrahydropyrannyle ou un reste phényle portant dans chaque cas un à trois substituants identiques ou différents, les substituants possibles étant avantageusement choisis parmi ceux qui sont énumérés ci-après :
fluor, chlore, brome, cyano, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluorochlorométhoxy, trifluoréthoxy, difluorométhylthio, trifluorométhylthio, difluorochlorométhylthio, trifluorométhylsulfinyle ou trifluorométhylsulfonyle, méthoxycarbonyle, éthoxycarbonyle, phényle,
un reste méthylènedioxy ou éthylènedioxy ayant chacun deux liaisons et portant chacun, le cas échéant, un à quatre substituants, identiques ou différents, fluoro, chloro, méthyle, trifluorométhyle ou éthyle,
ou un groupement 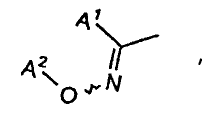 dans lequel
A¹ est un reste méthyle, éthyle, n-propyle, isopropyle, n-butyle, isobutyle, sec.-butyle, tertio-butyle, cyclopropyle ou cyclobutyle et
A² est un reste méthyle, éthyle, n-propyle, isopropyle, n-butyle, isobutyle, sec.-butyle, tertio-butyle, allyle, propargyle, but-2-ène-1-yle, 2-méthylprop-1-ène-3-yle, cyanométhyle, méthoxyméthyle, éthoxyméthyle, méthoxyéthyle, éthoxyéthyle, méthylthiométhyle, éthylthiométhyle, méthylthio-éthyle, éthylthio-éthyle, diméthylaminométhyle, diméthylamino-éthyle, méthylaminométhyle, méthylamino-éthyle ou benzyle.

5. Composés de formule (I) suivant la revendication 1, formule dans laquelle
A représente l'hydrogène, un reste éthyle ou méthyle, ou a la même définition que R,
R est un reste méthylthio, éthylthio, n-propylthio, isopropylthio, n-butylthio, isobutylthio, sec.-butylthio, tertio-butylthio, difluorométhylthio, trifluorométhylthio, difluorochlorométhylthio, dichlorofluorométhylthio, trichlorométhylthio, 2,2,2-trifluoréthylthio, méthoxycarbonylthio, éthoxycarbonylthio, un reste phénylthio portant éventuellement un à trois substituants fluoro, chloro, bromo, cyano, méthyle, éthyle, n-propyle, isopropyle, n-butyle, isobutyle, sec.-butyle, tertio-butyle, méthoxy, éthoxy, n-propoxy, isopropoxy, trifluorométhyle, trifluoréthyle, difluorométhoxy, trifluorométhoxy, difluorochlorométhoxy, trifluoréthoxy, méthoxycarbonyle ou éthoxycarbonyle, un groupe -S-Y, -S-N(R¹R²), où
R¹ est un reste méthyle, éthyle, n-propyle, isopropyle, n-butyle, isobutyle, sec.-butyle ou tertio-butyle,
R² est un reste méthyle, éthyle, n-propyle, isopropyle, n-butyle, isobutyle, sec.-butyle, tertio-butyle, méthoxycarbonyle ou éthoxycarbonyle, ou forme conjointement avec R¹ ainsi qu'avec l'atome d'azote auquel ils sont liés un noyau pyrrolidine, pipéridine ou morpholine éventuellement substitué par un radical méthyle ou éthyle,
Y est le même groupement que celui qui est déjà lié à l'atome de soufre,
ou bien, lorsque A est un reste méthyle,
R est aussi un reste acétyle, propionyle, méthoxycarbonyle, éthoxycarbonyle, méthoxycarbonylcarbonyle, 2-brométhoxycarbonyle, méthylaminocarbonyle, diméthylaminocarbonyle, méthylsulfonyle, éthylsulfonyle, phénylsulfonyle ou tolylsulfonyle,
G est un groupe -C (R⁸)=N-O-CH₂-,
R⁸ est un reste cyclopropyle ou en particulier méthyle,
L¹, L², L³ et L⁴ représentent l'hydrogène,
W est l'hydrogène, le fluor, un groupe cyano ou thiocyanato et
Z est un reste méthyle, éthyle, n-propyle, isopropyle, n-butyle, isobutyle, sec.-butyle, tertio-butyle, allyle, 2-butène-1-yle, 2-méthyl-2-butène-1-yle, propargyle ou un reste pyridyle, pyrimidyle ou en particulier phényle portant chacun, le cas échéant, un à trois substituants identiques ou différents, les substituants possibles étant avantageusement choisis parmi ceux qui sont énumérés ci-après :
fluor, chlore, brome, cyano, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, trifluorométhylthio, difluorochlorométhylthio, trifluorométhylsulfinyle ou trifluorométhylsulfonyle, méthoxycarbonyle, éthoxycarbonyle, méthoximinométhyle, éthoximinométhyle, méthoximino-éthyle, éthoximinoéthyle, un reste méthylènedioxy ou éthylènedioxy ayant chacun deux liaisons et portant chacun, le cas échéant, un à quatre substituants, identiques ou différents, fluoro, chloro, méthyle, trifluorométhyle ou éthyle.

6. Composés de formule (I) suivant la revendication 1, formule dans laquelle
A représente l'hydrogène, un reste méthyle ou éthyle, ou a la même définition que R,
R est un reste méthylthio, éthylthio, n-propylthio, isopropylthio, n-butylthio, isobutylthio, sec.-butylthio, tertio-butylthio, difluorométhylthio, trifluorométhylthio, dichlorofluorométhylthio, dichlorofluorométhylthio, trichlorométhylthio, 2,2,2-trifluoréthylthio, méthoxycarbonylthio, éthoxycarbonylthio, un reste phénylthio portant éventuellement un à trois substituants fluoro, chloro, bromo, cyano, méthyle, éthyle, n-propyle, isopropyle, n-butyle, isobutyle, sec.-butyle, tertio-butyle, méthoxy, éthoxy, n-propoxy, isopropoxy, trifluorométhyle, trifluoréthyle, difluorométhoxy, trifluorométhoxy, difluorochlorométhoxy, trifluoréthoxy, méthoxycarbonyle ou éthoxycarbonyle, un groupe -S-Y, -S-N(R¹R²), où
R¹ est un reste méthyle, éthyle, n-propyle, isopropyle, n-butyle, isobutyle, sec.-butyle ou tertio-butyle,
R² est un reste méthyle, éthyle, n-propyle, isopropyle, n-butyle, isobutyle, sec.-butyle, tertio-butyle, méthoxycarbonyle, éthoxycarbonyle, ou forme conjointement avec R¹ de même qu'avec l'atome d'azote auquel ils sont liés un noyau pyrrolidine, pipéridine ou morpholine portant éventuellement un substituant méthyle ou éthyle,
Y représente un même groupement que celui qui est déjà lié à l'atome de soufre,
ou bien, lorsque A est un reste méthyle,
R est aussi un reste acétyle, propionyle, méthoxycarbonyle, éthoxycarbonyle, méthoxycarbonylcarbonyle, 2-brométhoxycarbonyle, méthylaminocarbonyle, diméthylaminocarbonyle, méthylsulfonyle, éthylsulfonyle, phénylsulfonyle ou tolylsulfonyle,
G est un groupe -T-Ar¹-O-,
Ar¹ est un reste 1,2,4-thiadiazolediyle, 1,3,4-thiadiazolediyle, 1,2,4-oxadiazolediyle, 1,3,4-oxadiazolediyle ou un reste pyridinedyle, pyrimidinediyle ou 1,3,5-triazinediyle dont chacun porte éventuellement un ou deux substituants, identiques ou différents, fluoro, chloro, cyano, méthyle, cyclopropyle, méthoxy, méthylthio, trifluorométhyle, difluorométhoxy, trifluorométhoxy, difluorochlorométhoxy,
T est une liaison simple, l'oxygène, le soufre, un groupe -CH₂-O-, -CH₂-S-, méthylène, éthylène ou propylène et
L¹, L², L³ et L⁴ représentent l'hydrogène,
W est l'hydrogène, le fluor, un groupe cyano ou thiocyanato et
Z est un reste méthyle, éthyle, n-propyle, isopropyle, n-butyle, isobutyle, sec.-butyle, tertio-butyle, allyle, 2-butène-1-yle, 2-(1-méthyl)propyle, 2-méthyl2-butène-1-yle, propargyle ou un reste tétrazole, éthoxy, n-propoxy, isopropoxy, difluorométhoxy, difluorochlorométhoxy, trifluoréthoxy, trifluorométhoxy, méthoximinométhyle, éthoximino-méthyle, méthoximino-éthyle, éthoximino-éthyle portant chacun, le cas échéant, un à trois substituants, identiques ou différents, amino, fluoro, chloro, bromo, iodo, cyano, nitro, méthyle, éthyle, formyle, n-propyle, isopropyle, n-butyle, isobutyle, sec.-butyle ou tertio-butyle, trifluorométhyle, méthoxy ou tertiobutyle, ou bien un reste pyridyle, pyrimidyle, thiényle, tétrahydrobenzofurannyle ou en particulier phényle à substituant méthylènedioxy ou éthylènedioxy ayant chacun deux liaisons, portant chacun, le cas échéant, un à quatre substituants, identiques ou différents, fluoro, chloro, méthyle, trifluorométhyle ou éthyle.

7. Composés de formule (I) suivant la revendication 1, formule dans laquelle
A est l'hydrogène ou un reste méthyle ou a la même définition que R,
R est un reste méthylthio, éthylthio, n-propylthio, isopropylthio, n-butylthio, isobutylthio, sec.-butylthio, tertio-butylthio, difluorométhylthio, trifluorométhylthio, dichlorofluorométhylthio, dichlorofluorométhylthio, trichlorométhylthio, 2,2,2-trifluoréthylthio, méthoxycarbonylthio, éthoxycarbonylthio, un reste phénylthio portant éventuellement un à trois substituants fluoro, chloro, bromo, cyano, méthyle, éthyle, n-propyle, isopropyle, n-butyle, isobutyle, sec.-butyle, tertio-butyle, méthoxy, éthoxy, n-propoxy, isopropoxy, trifluorométhyle, trifluoréthyle, difluorométhoxy, trifluorométhoxy, difluorochlorométhoxy, trifluoréthoxy, méthoxycarbonyle ou éthoxycarbonyle, un groupe -S-Y, -S-N(R¹R²), où
R¹ est un reste méthyle, éthyle, n-propyle, isopropyle, n-butyle, isobutyle, sec.-butyle ou tertio-butyle,
R² est un reste méthyle, éthyle, n-propyle, isopropyle, n-butyle, isobutyle, sec.-butyle, tertio-butyle, méthoxycarbonyle ou éthoxycarbonyle, ou forme conjointement avec R¹ ainsi qu'avec l'atome d'azote auquel ils sont liés un reste pyrrolidine, pipéridine ou morpholine portant éventuellement un radical méthyle ou éthyle,
Y représente un même groupement que celui qui est déjà lié à l'atome d'azote,
ou bien, lorsque A est un reste méthyle,
R est aussi un reste acétyle, propionyle, méthoxycarbonyle, éthoxycarbonyle, méthoxycarbonylcarbonyle, 2-brométhoxycarbonyle, méthylaminocarbonyle, diméthylaminocarbonyle, méthylsulfonyle, éthylsulfonyle, phénylsulfonyle ou tolylsulfonyle,
G est un groupe -C(=N-O-R¹⁰)-C(R⁸)=N-O-CH₂-, dans lequel
R⁸ est un reste méthyle ou cyclopropyle et
R¹⁰ est l'hydrogène, un reste méthyle, éthyle, n-propyle, isopropyle, n-butyle, isobutyle, sec.-butyle, tertio-butyle ou un reste benzyle portant éventuellement un substituant fluoro, chloro, bromo, cyano, méthyle, éthyle, n-propyle, isopropyle, n-butyle, isobutyle, sec.-butyle, tertio-butyle, méthoxy, éthoxy, n-propoxy, isopropoxy, trifluorométhyle, trifluoréthyle, difluorométhoxy, trifluorométhoxy, difluorochlorométhoxy ou trifluoréthoxy,
L¹, L², L³ et L⁴ représentent l'hydrogène,
W est l'hydrogène, le fluor, un groupe cyano ou thiocyanato et
Z est un reste méthyle, éthyle, n-propyle, isopropyle, n-butyle, isobutyle, sec.-butyle, tertio-butyle, allyle, 2-butène-1-yle, 2-méthyl2-butène-1-yle ou un reste pyridyle, pyrimidyle ou en particulier phényle portant éventuellement un à trois substituants identiques ou différents, les substituants possibles étant avantageusement choisis parmi ceux qui sont énumérés ci-après :
fluor, chlore, brome, cyano, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, trifluorométhylthio, difluorochlorométhylthio, trifluorométhylsulfinyle, trifluorométhylsulfonyle, méthoxycarbonyle, éthoxycarbonyle, méthoximinométhyle, éthoximinométhyle, méthoximino-éthyle, éthoximino-éthyle, méthylènedioxy, éthylènedioxy ayant chacun deux liaisons et portant chacun, le cas échéant, un à quatre substituants, identiques ou différents, fluoro, chloro, méthyle, trifluorométhyle ou éthyle, ou tétrazole portant un substituant tertio-butyle.

8. Composés suivant la revendication 1, dans lesquels
G représente l'oxygène ou l'un des groupements suivants dont chacun est lié par le côté gauche à Z : -Q-CH₂-, -C(R⁸)=N-O-CH₂-, -N=C(R⁸)-Q-CH₂-, -CH (R⁸)-O-N=CH-, -C(R⁸)=N-N=CH-, -C(=N-O-R¹⁰)-C(R⁸)=N-O-CH₂-, -C(=N-O-R¹⁰)-C(R⁸)-O-N=CH-, -C (=N-O-R¹⁰)-C(R⁸)-N-N-=CH- ou -T-Ar¹, R⁸, R⁹, R¹⁰, T, Ar¹ et Q ayant les définitions indiquées dans la revendication 1.

9. Composés de formule (I) suivant la revendication 1, formule dans laquelle
A est le reste méthyle.

10. Composés de formule (I) suivant la revendication 1, formule dans laquelle
G représente l'oxygène, un groupe -O-CH₂-, - C (CH₂) =N-O-CH₂- ou un reste pyrimidinyloxy éventuellement substitué par du fluor.

11. Composés de formule (I) suivant la revendication 1, formule dans laquelle
Z est un reste phényle non substitué ou portant un ou deux substituants.

12. Composition contenant un diluant et/ou des supports ainsi que, le cas échéant, des substances tensioactives, **caractérisée par** une teneur en au moins un composé tel que défini dans la revendication 1.

13. Procédé pour combattre des parasites, **caractérisé en ce qu'**on fait agir des composés suivant la revendication 1 ou une composition telle que définie dans la revendication 12, sur les parasites et/ou sur leur milieu

14. Utilisation de composés suivant les revendications 1 à 11 ou d'une composition telle que définie dans la revendication 12 pour combattre des parasites.

15. Procédé de préparation de compositions, **caractérisé en ce qu'**on mélange des composés tels que définis dans les revendications 1 à 11 avec des diluants et/ou des agents tensio-actifs.

16. Procédé de production de composés de formule (I) suivant la revendication 1, **caractérisé en ce qu'**on fait réagir des amides de formule (II) 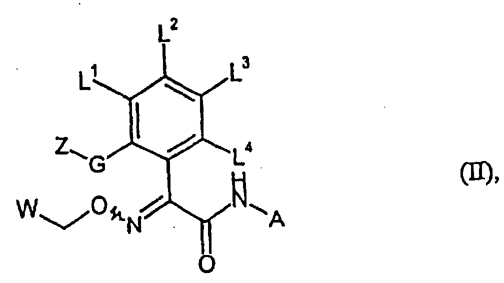 dans laquelle
A, G, L¹, L², L³, L⁴ et Z ont les définitions indiquées dans la revendication 1,
avec un réactif électrophile de formule (III)
R-X (III)
dans laquelle
R a la définition indiquée dans la revendication 1, et
X est un groupe partant,
le cas échéant en présence d'un diluant et en présence éventuelle d'une base.
